# EUROPEAN PATENT SPECIFICATION

(11) **EP 2 852 607 B1**
(45) Date of publication and mention of the grant of the patent: **24.10.2018**
(21) Application number: 13794398.1
(22) Date of filing: 24.04.2013
(51) Int. Cl.: C07J 75/00, C07J 17/00, C07J 71/00, A61K 31/704, A61K 31/7048, C07H 1/00, C07H 15/256, C07H 17/08

(54) **SYNTHESIS OF STEROID SAPONINS**
SYNTHESE VON STEROIDSAPONINEN
SYNTHESE DE SAPONINES STEROIDES

(30) Priority: 23.05.2012 AU 2012902119; 11.04.2013 AU 2013204005
(43) Date of publication of application: 01.04.2015
(73) Proprietor: Oncology Research International Limited, Perth, WA 6000 (AU)
(72) Inventor: MUSCROFT-TAYLOR, Andrew Clive, Christchurch, 7604 (NZ); MARSHALL, Philip Andrew, Tennyson, South Australia 5022 (AU); MASON, Jennifer Mary, Lower Hutt, 5012 (NZ); WALKER, David Millar, Wellington, 6011 (NZ)
(74) Representative: Frommberger, Moritz
(86) International application number: PCT/AU2013/000416
(87) International publication number: WO 2013/173862

(56) References cited:
- DENG S ET AL: "Synthesis of three diosgenyl saponins: dioscin, polyphyllin D, and balanitin 7", CARBOHYDRATE RESEARCH, PERGAMON, GB, vol. 317, no. 1-4, 30 April 1999 (1999-04-30), pages 53-62, XP004179994, ISSN: 0008-6215, DOI: 10.1016/S0008-6215(99)00066-X
- ZOU C-C ET AL: "The synthesis of gracillin and dioscin: two typical representatives of spirostanol glycosides", CARBOHYDRATE RESEARCH, PERGAMON, GB, vol. 338, no. 8, 4 April 2003 (2003-04-04) , pages 721-727, XP004417407, ISSN: 0008-6215, DOI: 10.1016/S0008-6215(03)00004-1
- SHUJIE HOU ET AL: "Facile Sythesis of Dioscin and Its Analogues", CHEMISTRY LETTERS, vol. 34, no. 9, 1 January 2005 (2005-01-01), pages 1220-1221, XP055222514, JAPAN ISSN: 0366-7022, DOI: 10.1246/cl.2005.1220
- HOU S ET AL: "Synthesis and antitumor activity of icogenin and its analogue", BIOORGANIC & MEDICINAL CHEMISTRY LETTERS, PERGAMON, AMSTERDAM, NL, vol. 16, no. 9, 1 May 2006 (2006-05-01), pages 2454-2458, XP025106882, ISSN: 0960-894X, DOI: 10.1016/J.BMCL.2006.01.074 [retrieved on 2006-05-01]
- KARELL PÉREZ-LABRADA ET AL: "New insights into the structure-cytotoxicity relationship of spirostan saponins and related glycosides", BIOORGANIC & MEDICINAL CHEMISTRY, vol. 20, no. 8, 1 April 2012 (2012-04-01), pages 2690-2700, XP055222522, GB ISSN: 0968-0896, DOI: 10.1016/j.bmc.2012.02.026
- HERNANDEZ J. C. ET AL.: 'Synthesis of novel spirostanic saponins and their cytotoxic activity' BIOORGANIC & MEDICINAL CHEMISTRY vol. 16, 2008, pages 2063 - 2076, XP022482502

## Description

### Field of the Invention

The present invention provides an improved synthesis of a class of steroid saponins. Furthermore, the present invention provides a method of selectively discriminating between the C2 and C3 hydroxyl groups of a mono-glycosylated steroid saponin - a key step in the preparation of this class of compounds. Additionally, the present invention provides a range of steroid saponin derivatives, and methods of making them. The scope of the present invention is defined in claim 1.

### Cross Reference to Related Application(s)

The present invention is related to the following two international patent applications assigned to the present applicant: PCT/AU2007/001091 and PCT/AU2007/001092. The aforementioned applications describe advantageous therapeutic applications, compositions and uses of some steroid saponins that can be synthesised using the methods of the present invention.

### Background

There remains a great need for new compounds and therapies to treat all manner of pathogenic, deficiency, hereditary and physiological diseases. In particular, with rising life expectancies, there has been a significant rise in the incidence of non-infectious, age-related diseases, such as cancer.

Furthermore, it is paramount that new, efficient methods for accessing these active agents are developed to provide access to them in sufficient quantities to provide broad availability of their novel therapies.

Steroid saponins, a class of secondary metabolites derived from various plant and marine species, are of particular interest as novel active agents due to their remarkable bioactivity. Some saponins have been shown to bind and cross cell membranes, others have been utilised as surfactants, still others have been used as adjuvants in vaccines. Saponins have also been utilised in Chinese medicine and as such, have been promoted as dietary supplements. Furthermore, some steroid saponins are known to enhance the activity of a number of chemotherapeutic and anti-cancer agents, ultimately inhibiting growth of cancerous cells. Other steroid saponins have demonstrated an ability to inhibit angiogenesis in a number of *in vivo* and *ex vivo* model systems. Accordingly, steroid saponins provide a class of interesting molecules with diverse biological activity.

Some naturally occurring steroid saponins have been extracted from plant sources. However, the relatively small and seasonally variable amounts of the compound available from plant sources via extraction limits the potential commercial development of this class of compounds and its physiologically active analogues and derivatives as therapeutic agents. Obtaining access to the steroid saponins via synthetic routes is thus paramount to their further development as pharmaceutical agents.

Nevertheless, the synthesis of these complex molecules remains challenging. Broadly speaking, some problems associated with achieving an efficient synthesis include the large number of stereocentres; the contrasting physico-chemical properties of the lipophilic sapogenin/aglycone moiety and the more hydrophilic glycoside moiety; and their comparatively high molecular weights.

More specifically, the construction of the di- and/or poly-glycoside moiety has proved particular difficult to achieve. In particular, discrimination of the C2 / C3 hydroxyl groups of the mono-glycoside saponin has proved difficult, but is nevertheless a key step in order to selectively and efficiently functionalise the derived saponin with further glycosidic or other moieties at either of the C2 or C3 positions.

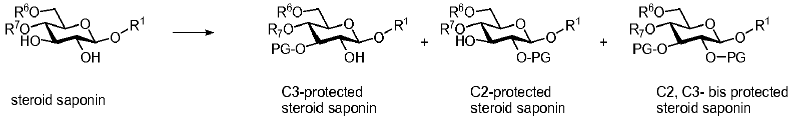

The C2 and C3 hydroxyl groups of the mono-glycosylated steroid saponin have similar physico-chemical properties, and thus substantially similar reactivities (Journal of Organic Chemistry (1997), 62, pp. 8406-8418). Achieving regio-selectivity at one or other of these positions is thus challenging. In view of this, previous syntheses towards steroid saponin derivatives have utilised elaborate and inefficient protecting group strategies comprising many steps in order to discriminate between these two groups. Alternative syntheses have utilised non- or semi-selective conditions to obtain mixtures of regio-isomers which subsequently require complicated separation and purification techniques in order to access one or other preferred regioisomer. However, separation of C2-mono protected and C3-mono protected compounds, is often difficult due to their similar physico-chemical properties. All of these strategies are inefficient, require separation of complex mixtures and/or necessitate removal of contaminating by-products and thus ultimately lead to reduced yields of the desired steroid saponin. This is clearly undesirable when access to commercial scale quantities of the active agent is required.

Previously described attempts provide low yields and/or poor selectivity for one or other regioisomer. For example, previous attempts to selectively protect the C3-hydroxyl of a steroid saponin with a silyl protecting group, namely tertbutyldimethylsilyl chloride, provided 35% of the undesired C2-protected steroid saponin, and 52% of the desired C3-steriod saponin. This mixture of products ultimately requires extensive separation and purification in order to access one or other preferred regioisomer (Carbohydrate Research, (1998) 306, pp. 189-195).

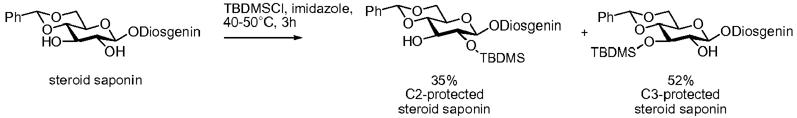

Similarly poor selectivity has been reported with other protecting groups, such as pivaloyl chloride, wherein 9.5% of the undesired C2-protected steroid saponin, 45% of the desired C3-steriod saponin and 5% of the C2,C3-bis protected steroid saponin were obtained (Bioorganic & Medicinal Chemistry (2008), 16, pp. 2063-2076).

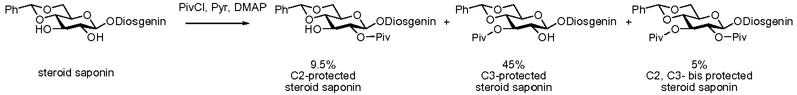

Furthermore, attempts to selectively prepare a C3-protected steroid saponin using known coupling reagents also reportedly result in mixtures of products. For example, use of levulinic acid with N,N'-dicyclohexylcarbodiimide and DMAP in DCM provided the C3- and C2-protected material in 75% yield and a ratio of 2:1 (Bioorganic & Medicinal Chemistry Letters (2006), 16, pp. 2454-2458).

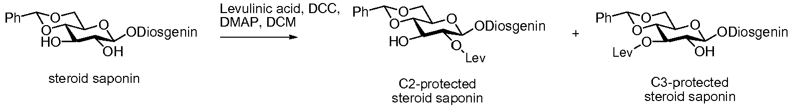

Still other attempts to subsequently functionalise similar steroid saponins, without protection at either of C2 or C3 have resulted in, for example, no selectivity and complex mixtures comprising the starting steroid saponin (43%), C2-mono functionalised (8%), C3-mono functionalised (32%) and C2,C3-bis functionalised compounds (17%) (Journal of Carbohydrate Chemistry (1999), 18, pp. 1107-1120).

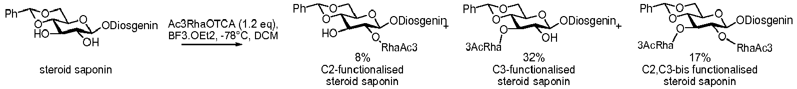

In view of the inefficient synthesis described in the art, development of a new method for efficient and selective discrimination of the C2 / C3 hydroxyl groups, especially of mono-glycosylated saponins is paramount.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application.

Deng, S. et al., Carbohydr. Res. 317 (1999) 53-62 report on the synthesis of Dioscin, polyphyllin D, and balanitin 7, which belong to a group of structurally similar diosgenyl saponins, by stepwise glycosylation. Three representative spirostanol saponins that have typical structure of the sugar moiety, diosgenyl 2,3-di-O-α-L-rharnnopyranosyl-β-D-glucopyranoside, diosgenyl 2,4-di-O-α-L-rhamnopyranosyl-β-D-glucopyranoside (dioscin), and diosgenyl 2,6-di-O-α-L-rhamnopyranosyl-β-D-glucopyranoside, were synthesized in a facile way according to Hou et al., Chem. Lett. 34 (2005) 1220-1221. A variety of spirostan saponins and related glycosides were synthesized and evaluated for their cytotoxicity against the human myeloid leukemia cell line (HL-60) by Perez-Labrada, K. et al., Bioorg. Med. Chem. 20 (2012) 2690-2700.

Where the terms "comprise", "comprises", "comprised" or "comprising" are used in this specification (including the claims) they are to be interpreted as specifying the presence of the stated features, integers, steps or components, but not precluding the presence of one or more other features, integers, steps or components, or group thereof.

### Summary of the Invention

In seeking new efficient synthesis of a range of active agents, specifically steroid saponins, the Applicant has surprisingly found a method of discriminating between the C2 / C3 hydroxyl groups of the mono-glycoside saponin. The new method advantageously provides efficient, regio- and stereo-selective access to this class of compounds. Furthermore, the method also provides access to a range of new saponin derivatives, not previously known or characterised. As such, the method described by the Applicant provides the desired C3-protected mono-glycosylated saponin in excellent selectivity. In some embodiments, none of the undesired C2-protected mono-glycoside is observed. In view of this surprising and unexpected result, the applicant's method advantageously provides a more efficient route to access this class of active agents, and thus provides access to a range of novel physiologically active analogues and derivatives thereof. The present invention thus extends to such novel steroid saponin analogues and derivatives.

Accordingly, in one aspect, the present invention provides a method for the preparation of a compound of Formula X as defined in claim 1. Preferred embodiments are the subject of the dependent claims.

### Detailed Description of the Invention

Various terms that will be used throughout the specification have meanings that will be well understood by a skilled addressee. However, for ease of reference, some of these terms will now be defined, their scope being limited by that of the claims.

The term "protected" as used throughout the specification in the context of any compound, residue or functional group will, unless the context otherwise requires, be taken to refer to the use of a removable protecting group to prevent an undesirable reaction of the respective compound, residue or functional group. The expression "protecting group" and like expressions will be understood correspondingly. Protecting groups, if present, may if desired be simultaneously or subsequently removed in conventional manner. The use and removal of appropriate protecting groups will be well within the capacity of those skilled in this art.

The term "oxygen protecting group" as used throughout the specification means a group that can prevent the oxygen moiety reacting during further derivatisation of the protected compound and which can be readily removed when desired. Examples of oxygen protecting groups include but are not limited to acyl groups (such as acetyl and benzoyl), optionally substituted alkoxy (such as methoxy methyl ether (MOM), p-methoxy ethoxy methyl ether (MEM), p-methoxy benzyl ether (PMB), methylthio methyl ether, Pivaloyl (Piv), Tetrahydropyran (THP)), and silyl ethers (such as Trimethylsilyl (TMS) tert-butyl dimethyl silyl (TBDMS) and triisopropylsilyl (TIPS).

The terms "alcohol", "hydroxyl" or "hydroxy" are all understood to refer to a functional group of the formula -OH, and may be used interchangeably throughout the specification. Where a molecule or compound comprises more than one functional group of the formula -OH, these may be differentiated based on the order of attachment to, or systematic numbering of, the molecule or compound. Where possible, the order of attachment is determined by standard International Union of Pure and Applied Chemistry (IUPAC) nomenclature. For example reference to C2-hydroxyl of a monoglycoside saponin, refers to the secondary alcohol at the 2-position of the glycoside ring. Similarly, reference to C3-hydroxyl of a monoglycoside saponin refers to the secondary alcohol at the 3-position of the glycoside ring.

"Acyl" and "acyl group" as used throughout the specification, will be understood to refer to a group of the following formula: R'-C(=O)-; wherein R' is independently selected from the group consisting of H, halogen, optionally substituted C₁-C₁₂ alkyl, optionally substituted C₂-C₁₂ alkenyl, optionally substituted C₂-C₁₂ alkynyl, optionally substituted C₂-C₁₀heteroalkyl, optionally substituted C₃-C₁₂ cycloalkyl, optionally substituted bicycloalkyl, optionally substituted C₃-C₁₂ cycloalkenyl, optionally substituted C₂-C₁₂ heterocycloalkyl, optionally substituted C₂-C₁₂ heterocycloalkenyl, optionally substituted C₆-C₁₈ aryl, optionally substituted C₁-C₁₈ heteroaryl, optionally substituted aroyl, optionally substituted phenyl, optionally substituted C₁-C₁₀ alkoxyphenyl, optionally substituted C₁-C₁₀ dialkoxyphenyl, optionally substituted C₁-C₁₀ alkylphenyl, or optionally substituted C₁-C₁₀ dialkylphenyl. Examples of suitable acyl groups include acetoyl, propionyl, trichloroacetyl, benzoyl, 2-chlorobenzoyl, 4-chlorobenzoyl, 4-nitrobenzoyl and 4-methoxybenzoyl.

The term "acylating agent" as used throughout the specification, will be understood to mean a reagent or compound that provides an acyl group upon reaction with a nucleophile. In some embodiments, the nucleophile is oxygen as part of an alcohol functional group.

The term "leaving group" is any atom, group of atoms or molecular fragment, that is displaced during heterolytic bond cleavage, and takes with it the pair of bonding electrons. The leaving group may be charged or uncharged, but is generally stable. Accordingly in any situation the choice of leaving group will depend upon the ability of the particular group to be displaced by the incoming chemical moiety. Suitable leaving groups are well known in the art, see for example "Advanced Organic Chemistry" Jerry March 4th Edn. pp 351-357, Oak Wick and Sons NY (1997). Examples of suitable leaving groups include, but are not limited to, halogen, alkoxy (such as ethoxy, methoxy), sulphonyloxy, optionally substituted arylsulfonyl, optionally substituted silyl, and optionally substituted acyl (such as acetyl and trichloroacetyl), optionally substituted akylthio, and optionally substituted aryl thio.

The terms "coupling reaction", "coupling conditions" and the like, as used throughout the specification are understood to mean reactions and/or conditions that may be used to couple a saccharide molecule to another saccharide molecule, which may be the same or a different saccharide to assemble larger polysaccharides; or to couple a saccharide to a sapogenin to form a mono-glycoside steroid saponin, or similarly to couple additional saccharides to form di- or poly-saccharide saponins; or to couple a saccharide to a compound of any one of Formulas A, B, C X, Y, Y^{A}, Y^{B}, Y^{C}, E, F and G.

"Alkenyl" as a group or part of a group denotes an aliphatic hydrocarbon group containing at least one carbon-carbon double bond and which may be straight or branched having 2-12 carbon atoms, typically 2-10 carbon atoms, typically 2-6 carbon atoms, in the normal chain. The group may contain a plurality of double bonds in the normal chain and the orientation about each is independently E or Z. The alkenyl group is typically a 1-alkenyl group. Exemplary alkenyl groups include, but are not limited to, ethenyl, propenyl, butenyl, pentenyl, hexenyl, heptenyl, octenyl and nonenyl.

"Alkyl" as used throughout the specification as a group or part of a group refers to a straight or branched aliphatic hydrocarbon group, of for example one to twenty carbon atoms, typically a C₁-C₁₀ alkyl, typically C₁-C₆ alkyl unless otherwise noted. Examples of suitable straight and branched C₁-C₆ alkyl substituents include methyl, ethyl, n-propyl, 2-propyl, n-butyl, sec-butyl, t- butyl, hexyl, and the like.

"Alkyloxy" and "alkoxy" as used throughout the specification refer to an alkyl-O- group in which alkyl is as defined herein. Examples include, but are not limited to, methoxy and ethoxy.

"Aryl" as a group or part of a group denotes (i) an optionally substituted monocyclic, or fused polycyclic, aromatic carbocycle (ring structure having ring atoms that are all carbon) preferably having from 5 to 12 atoms per ring. Examples of aryl groups include phenyl, naphthyl, and the like; (ii) an optionally substituted partially saturated bicyclic aromatic carbocyclic moiety in which a phenyl and a C₅-C₇ cycloalkyl or C₅-C₇ cycloalkenyl group are fused together to form a cyclic structure, such as tetrahydronaphthyl, indenyl or indanyl. Typically an aryl group is a C₆-C₈ aryl group and most preferably is phenyl.

"Arylalkyl" means an aryl-alkyl- group in which the aryl and alkyl moieties are as defined herein. Preferred arylalkyl groups contain a C₁-C₅ alkyl moiety. Exemplary arylalkyl groups include benzyl, phenethyl, 1-naphthalenemethyl and 2-naphthalenemethyl.

"Arylamino" includes both mono-arylamino and di-arylamino unless specified. Mono-arylamino means a group of formula arylNH-, in which aryl is as defined herein, di-arylamino means a group of formula (aryl)₂N- where each aryl may be the same or different and are each as defined herein for aryl.

"Aryloxy" refers to an aryl-O- group in which the aryl is as defined herein. Preferably the aryloxy is a C₆-C₁₈aryloxy, more preferably a C₆-C₁₀aryloxy.

A "bond" is a covalent linkage between atoms in a compound or molecule. The bond may be a single bond, a double bond, or a triple bond.

"Cycloalkenyl" means a non-aromatic monocyclic or multicyclic ring system containing at least one carbon-carbon double bond and preferably having from 5-10 carbon atoms per ring. Exemplary monocyclic cycloalkenyl rings include cyclopentenyl, cyclohexenyl or cycloheptenyl. The cycloalkenyl group may be substituted by one or more substituent groups. A cycloalkenyl group typically is a C₃-C₁₂ alkenyl group.

"Cycloalkyl" refers to a saturated monocyclic or fused or spiro polycyclic, carbocycle preferably containing from 3 to 9 carbons per ring, such as cyclopropyl, cyclobutyl, cyclopentyl, cyclohexyl and the like, unless otherwise specified. It includes monocyclic systems such as cyclopropyl and cyclohexyl, bicyclic systems such as decalin, and polycyclic systems such as adamantane. A cycloalkyl group typically is a C₃-C₁₂ alkyl group.

"Haloalkyl" refers to an alkyl group as defined herein in which one or more of the hydrogen atoms has been replaced with a halogen atom selected from the group consisting of fluorine, chlorine, bromine and iodine. A haloalkyl group typically has the formula CₙH₍₂ₙ₊₁₋ₘ₎Xₘ wherein each X is independently selected from the group consisting of F, CI, Br and I. In groups of this type n is typically from 1 to 10, more preferably from 1 to 6, most preferably 1 to 3. m is typically 1 to 6, more preferably 1 to 3. Examples of haloalkyl include fluoromethyl, difluoromethyl and trifluoromethyl.

"Haloalkenyl" refers to an alkenyl group as defined herein in which one or more of the hydrogen atoms has been replaced with a halogen atom independently selected from the group consisting of F, CI, Br and I.

"Halogen" or "halo" represents chlorine, fluorine, bromine or iodine.

"Heteroalkyl" refers to a straight- or branched-chain alkyl group preferably having from 2 to 12 carbons, typically 2 to 6 carbons in the chain, in which one or more of the carbon atoms (and any associated hydrogen atoms) are each independently replaced by a heteroatomic group selected from S, O, P and NR' where R' is selected from the group consisting of H, optionally substituted C₁-C₁₂ alkyl, optionally substituted C₃-C₁₂ cycloalkyl, optionally substituted C₆-C₁₈ aryl, and optionally substituted C₁-C₁₈ heteroaryl. Exemplary heteroalkyls include alkyl ethers, secondary and tertiary alkyl amines, amides, alkyl sulfides, and the like. Examples of heteroalkyl also include hydroxyl C₁-C₆ alkyl, C₁-C₆ alkyloxy C₁-C₆ alkyl, amino C₁-C₆ alkyl, C₁-C₆ alkylamino C₁-C₆ alkyl, and di(C₁-C₆ alkyl)amino C₁-C₆ alkyl.

"Heteroaryl" either alone or part of a group refers to groups containing an aromatic ring (preferably a 5 or 6 membered aromatic ring) having one or more heteroatoms as ring atoms in the aromatic ring with the remainder of the ring atoms being carbon atoms. Suitable heteroatoms include nitrogen, oxygen and sulphur. Examples of heteroaryl include thiophene, benzothiophene, benzofuran, benzimidazole, benzoxazole, benzothiazole, benzisothiazole, naphtho[2,3-bi]thiophene, furan, isoindolizine, xantholene, phenoxatine, pyrrole, imidazole, pyrazole, pyridine, pyrazine, pyrimidine, pyridazine, tetrazole, indole, isoindole, 1H-indazole, purine, quinoline, isoquinoline, phthalazine, naphthyridine, quinoxaline, cinnoline, carbazole, phenanthridine, acridine, phenazine, thiazole, isothiazole, phenothiazine, oxazole, isooxazole, furazane, phenoxazine, 2-, 3- or 4- pyridyl, 2-, 3-, 4-, 5-, or 8- quinolyl, 1-, 3-, 4-, or 5-isoquinolinyl 1-, Z-, or 3- indolyl, and 2-, or 3- thienyl. A heteroaryl group is typically a C₁-C₁₈ heteroaryl group.

"Heterocyclic" refers to saturated, partially unsaturated or fully unsaturated monocyclic, bicyclic or polycyclic ring system containing at least one heteroatom selected from the group consisting of nitrogen, sulfur and oxygen as a ring atom. Examples of heterocyclic moieties include heterocycloalkyl, heterocycloalkenyl and heteroaryl.

"Heterocycloalkyl" refers to a saturated monocyclic, bicyclic, or polycyclic ring containing at least one heteroatom selected from nitrogen, sulfur or oxygen, preferably from 1 to 3 heteroatoms in at least one ring. Each ring is preferably from 3 to 10 membered, more preferably 4 to 7 membered. Examples of suitable heterocycloalkyl substituents include pyrrolidyl, tetrahydrofuryl, tetrahydrothiofuranyl, piperidyl, piperazyl, tetrahydropyranyl, morphilino, 1,3-diazapane, 1,4-diazapane, 1,4-oxazepane, and 1,4-oxathiapane. A heterocycloalkyl group typically is a C₂-C₁₂ heterocycloalkyl group.

The term "glycoside" as used throughout the specification is to be understood to mean a compound that contains a saccharide (sugar) moiety (monosaccharide, disaccharide or polysaccharide), linked to a triterpene or steroid or steroid alkaloid aglycone (non-saccharide) component. In most circumstances, the saccharide (sugar) moiety is linked to the C-3 position of the aglycone, although other linkages are contemplated within the scope of the present invention. For example the furostanol glycosides, which contain a saccharide attached to the C-26 position, and spirostanol glycosides are both sub-classes of the steroid saponins.

"Lower alkyl" as a group means unless otherwise specified, an aliphatic hydrocarbon group which may be straight or branched having 1 to 6 carbon atoms in the chain, more preferably 1 to 4 carbons such as methyl, ethyl, propyl (n-propyl or isopropyl) or butyl (n-butyl, isobutyl or tertiary-butyl).

The term "saponin" as used throughout the specification is to be understood to mean a glycoside including a saccharide (sugar) attached to the aglycone, generally through the C-3 position of the aglycone.

The term "steroid saponin" as used throughout the specification is to be understood to mean a glycoside including one or more saccharide units (including one or more monosaccharide, disaccharide or polysaccharide units) attached to an aglycone which does not contain a nitrogen atom.

In this regard, it will be understood that the term "steroid saponin" includes within its scope any salts or any other derivatives of the compounds that are functionally equivalent, in particular with respect of therapeutic active agents. As such, they may be pharmaceutically acceptable salts. Furthermore, they may be naturally-occurring or synthetic steroid saponins.

The term "pharmaceutically acceptable salts" used throughout the specification is understood to mean salts that retain the desired biological activity of the above-identified compounds, and include pharmaceutically acceptable acid addition salts and base addition salts. Suitable pharmaceutically acceptable acid addition salts of compounds of the present invention may be prepared from an inorganic acid or from an organic acid. Examples of such inorganic acids are hydrochloric, sulfuric, and phosphoric acid. Appropriate organic acids may be selected from aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic arylsulfonic. Suitable pharmaceutically acceptable base addition salts include metallic salts made from lithium, sodium, potassium, magnesium, calcium, aluminium, and zinc, and organic salts made from organic bases such as benzathine, chloroprocaine, choline, diethanolamine, ethanolamine, ethyldiamine, meglumine and procaine. Other examples of organic salts are: ammonium salts, quaternary salts such as tetramethylammonium salt; amino acid addition salts such as salts with histidine, glycine, lysine and arginine. Additional information on pharmaceutically acceptable salts can be found in Stahl and Wermuth's Handbook of Pharmaceutically Acceptable Salts, 2nd Edition, Wiley-VCH, 2002. In the case of agents that are solids, it is understood by those skilled in the art that the inventive compounds, agents and salts may exist in different amorphous, crystalline or polymorphicforms, all of which are intended to be within the scope of the present invention and specified formulae.

Additionally, compounds referred to herein are intended to cover, where applicable, solvated as well as unsolvated forms of the compounds. Thus, each formula includes compounds having the indicated structure, including the solvated as well as the non-solvated forms. Thus, where the solvent is water each formula includes compounds having the indicated structure, including the hydrated as well as the non-hydrated forms.

A steroid "aglycone" is also called a "genin" or "sapogenin" and the terms may be used interchangeably throughout the specification and all are to be understood to mean the non-saccharide portion of a saponin molecule.

The term "unsubstituted" as used throughout the specification means that there is no substituent or that the only substituents are hydrogen.

The term "optionally substituted" as used throughout the specification denotes that the group may or may not be further substituted or fused (so as to form a condensed polycyclic system), with one or more non-hydrogen substituent groups. In certain embodiments the substituent groups are one or more groups independently selected from the group consisting of halogen, =O, =S, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, heteroarylalkyl, arylalkyl, cycloalkylalkenyl, heterocycloalkylalkenyl, arylalkenyl, heteroarylalkenyl, cycloalkylheteroalkyl, heterocycloalkylheteroalkyl, arylheteroalkyl, heteroarylheteroalkyl, hydroxy, hydroxyalkyl, alkyloxy, alkyloxyalkyl, alkyloxycycloalkyl, alkyloxyheterocycloalkyl, alkyloxyaryl, alkyloxyheteroaryl, alkyloxycarbonyl, alkylaminocarbonyl, alkenyloxy, alkynyloxy cycloalkyloxy, cycloalkenyloxy, heterocycloalkyloxy, heterocycloalkenyloxy, aryloxy, phenoxy, benzyloxy, heteroaryloxy, arylalkyloxy, amino, alkylamino, acylamino, aminoalkyl, arylamino, sulfonylamino, sulfinylamino, sulfonyl, alkylsulfonyl, arylsulfonyl, aminosulfonyl, sulfinyl, alkylsulfinyl, arylsulfinyl, aminosulfinylaminoalkyl, -C(=O)OH, -C(=O)R^{a}, -C(=O)OR^{a}, C(=O)NR^{a}R^{b}, C(=NOH)R^{a}, C(=NR^{a})NR^{b}R^{c}, NR^{a}R^{b}, NR^{a}C(=O)R^{b}, NR^{a}C(=O)OR^{b}, NR^{a}C(=O)NR^{b}R^{c}, NR^{a}C(=NR^{b})NR^{c}R^{d}, NR^{a}SO₂R^{b},-SR^{a}, SO₂NR^{a}R^{b}, -OR^{a}, OC(=O)NR^{a}R^{b}, OC(=O)R^{a} and acyl,
wherein R^{a}, R^{b}, R^{c} and R^{d} are each independently selected from the group consisting of H, optionally substituted C₁-C₁₂ alkyl, optionally substituted C₁-C₁₂ haloalkyl, optionally substituted C₂-C₁₂ alkenyl, optionally substituted C₂-C₁₂ alkynyl, optionally substituted C₂-C₁₀ heteroalkyl, optionally substituted C₃-C₁₂ cycloalkyl, optionally substituted C₃-C₁₂ cycloalkenyl, optionally substituted C₂-C₁₂ heterocycloalkyl, C₂-C₁₂ heterocycloalkenyl, optionally substituted C₆-C₁₈ aryl, optionally substituted C₁-C₁₈ heteroaryl, and acyl, or any two or more of R^{a}, R^{b}, R^{c} and R^{d}, when taken together with the atoms to which they are attached form a heterocyclic ring system with 3 to 12 ring atoms.

It is recognized that the steroid saponins of the present invention are advantageous therapeutic agents for the treatment of disease. In using the compounds of the invention they can be administered in any form or mode which makes the compound available to provide a biological effect. One skilled in the art of preparing formulations can readily select the proper form and mode of administration depending upon the particular characteristics of the compound selected, the condition to be treated, the stage of the condition to be treated and other relevant circumstances. As previously described, and in view of the advantageous therapeutic effect(s), compounds referred to herein are intended to cover, where applicable, pharmaceutically acceptable salts, hydrates, solvates thereof that may advantageously provide or improve the bioavailability and/or administration of the compound of the present invention to obtain a desired therapeutic effect.

Saponins are conventionally divided into three major classes: (i) triterpene glycosides; (ii) steroidal glycosides; and (iii) steroidal alkaloid glycosides. They all have in common the attachment of one or more saccharide units to the aglycone, generally at the C-3 position. Steroid saponins are generally as described in Hostettmann K and Marston A (2005). Chemistry & pharmacology of natural products: Saponins. Cambridge University Press.

As discussed previously herein, steroid saponins do not contain a nitrogen atom in the aglycone moiety.

It will be appreciated that the steroid saponin in the various embodiments of the present invention include naturally occurring steroid saponins and non-naturally occurring steroid saponins (ie chemically synthesized steroid saponins). In addition, it will also be appreciated that the steroid saponin in the various embodiments of the present invention also includes pro-drugs of the steroid saponin, derivatives of steroid saponins, including for example, any esters, ketones, carboxylic acids, salts, substituted forms, halogenated forms or other heteroatom containing forms, unsaturated forms, or any other functional derivative, as far as falling within the scope of the claims.

The saccharide portion of steroid saponins may generally include one or more saccharide units, such as a monosaccharide, a disaccharide unit or a polysaccharide unit.

It will also be appreciated that a steroid saponin may generally also include an aglycone with a saccharide attached at one or more positions of the aglycone moiety.

As discussed above, a saccharide unit may generally be a monosaccharide, a disaccharide or a polysaccharide. The saccharide may be composed of a suitable monosaccharide, such as D-glucose (Glc), L-rhamnose (Rha), D-galactose (Gal), D-glucuronic acid (GlcA), D-xylose (Xyl), L-arabinose (Ara), D-fucose (Fuc), D-galacturonic acid (GalA). The saccharide unit may also be a substituted saccharide, such as an amino saccharide, a sulfated saccharide, an acylated saccharide, an *N*-acylated amino saccharide, and functional derivatives of any of the aforementioned monosaccharides.

Similarly, a disaccharide may generally be any combination of two monosaccharides, as described above.

Polysaccharides may generally be linear or branched, and include any combination of two or more monosaccharide, including the monosaccharide described previously herein.

In this regard, and as described previously herein, polysaccharides are generally described in the context of the arrangement of the component monosaccharides.

The method of the invention can be performed on any scale from laboratory through pilot plant to commercial (kilogram) scale.

As previously described, in some aspects, the present invention provides a new method for accessing a range of steroid saponins.

With regard to the new method described herein, the present invention provides a method for the preparation of a compound of Formula X 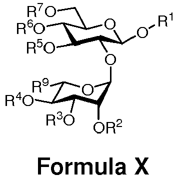
wherein R¹ is a sapogenin of Formula E, F or G as specified below;
R², R³, and R⁴ are each independently an oxygen protecting group;
R⁵ is an acyl group selected from the group consisting of benzoyl, 2-chlorobenzoyl, 4-chlorobenzoyl, 4-nitrobenzoyl and 4-methoxybenzoyl;
R⁶ and R⁷ are H;
R⁹ is selected from the group consisting of H, CH₃, and an oxygen protected by an oxygen protecting group;
the method comprising
(i) reacting a compound of Formula A with an acylating agent selected from the group consisting of benzoyl chloride, 2-chlorobenzoyl chloride, 4-chlorobenzoyl chloride, 4-nitrobenzoyl chloride and 4-methoxybenzoyl chloride in an acylation reaction in the presence of a base; 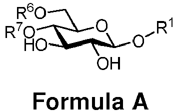 wherein
   R¹ is a sapogenin of Formula E, F or G as specified below;
   R⁶ and R⁷ are each independently an oxygen protecting group, or when taken together form a cyclic di-oxygen protecting group;
   to provide a compound of Formula B 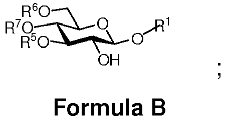
   R¹ is sapogenin of Formula E, F or G as specified below;
   R⁵ is an acyl group selected from the group consisting of benzoyl, 2-chlorobenzoyl, 4-chlorobenzoyl, 4-nitrobenzoyl and 4-methoxybenzoyl;
   R⁶ and R⁷ are each independently an oxygen protecting group or when taken together form a cyclic di-oxygen protecting group;
(i) reacting a compound of Formula B with a compound of Formula C under coupling conditions 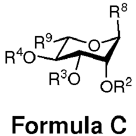
   wherein R², R³, and R⁴ are each independently an oxygen protecting group;
   R⁸ is a leaving group; and
   R⁹ is selected from the group consisting of H, CH₃, and an oxygen protected by an oxygen protecting group;
(iii) selectively removing the oxygen protecting groups at R⁶ and R⁷ to provide a compound of Formula X.
   In an embodiment, the present invention provides a method further comprising
(iv) converting a compound of formula X into a compound of formula Y, 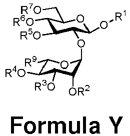
   wherein R¹ is a sapogenin of Formula E, F or G as specified below;
   R², R³, R⁴, R⁵ and R⁷ are each H;
   R⁶ is H or a saccharide;
   R⁹ is selected from the group consisting of H, OH and CH₃;
   pharmaceutically acceptable salts, hydrates and solvates thereof.

In some embodiments due to the nature of the protecting groups at R², R³, R⁴, R⁶ and R⁷, the oxygen protecting group that can be a component of R⁹ and the acyl group at R⁵ steps (III) and (IV) can be carried out concurrently as all protecting groups can be removed in a single operation. In these embodiments a compound of formula X is not isolated as a single operation removes all protecting groups.

Where R², R³, and R⁴ are each oxygen protecting groups, they may be any suitable oxygen protecting group. In some embodiments of Formula C, R², R³, and R⁴ are each independently an acyl group. In other embodiments, R², R³, and R⁴ of Formula C are each independently selected from an optionally substituted benzoyl and acetyl; in other embodiments R², R³, and R⁴ are each independently an optionally substituted benzoyl.

In some embodiments, the choice of leaving group at R⁸ will depend upon the ability of the particular group to be displaced by the incoming chemical moiety. In some embodiments the leaving group is chosen such as to make Formula C an activated donor. Examples of suitable leaving groups are halogen, optionally substituted acyl (such as acetate), optionally substituted alkoxy (such as ethoxy, methoxy), optionally substituted acetimidate (such as trichloroacetimidate or *N*-(phenyl)trifluoroacetimidate), sulphonyloxy, optionally substituted arylsulfonyl, optionally substituted silyl, optionally substituted akylthio, optionally substituted arylthio. In some embodiments R⁸ of Formula C is selected from the group consisting chloro, iodo, bromo, fluoro, ethoxy, methoxy, mesylate, tosylate, triflate, trimethyl silyl, *t*-butyldimethyl silyl, methanethio, ethanethio, *t*-butylthio, trichloroacetyl, trichloroacetimidate and *N-*(phenyl)trifluoroacetimidate. In other embodiments R⁸ is trichloroacetimidate or *N-*(phenyl)trifluoroacetimidate.

R⁶ and R⁷ of Formula A may each independently be any oxygen protecting group. Suitable oxygen protecting groups have been described as discussed for R², R³ and R⁴ above and are equally applicable to R⁶ and R⁷. R⁶ and R⁷ of Formula A may each independently be an acyl or alkoxyl protecting group. R⁶ and R⁷ may be an acetal, or when taken together, form a cyclic acetal. In an embodiment, R⁶ and R⁷ when taken together, form a cyclic group selected from the group consisting of:

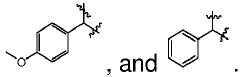

The sapogenin at R¹ of Formula A, Formula X, and Formula Y is a compound of Formula E, F or G as follows: 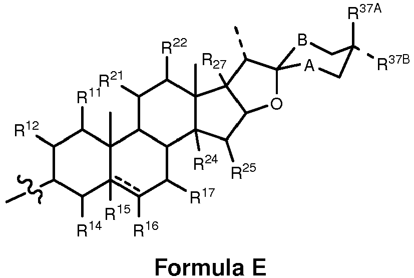 wherein
R¹¹, R¹², R¹⁴, R¹⁶, R¹⁷, R²¹, R²², R²⁴, R²⁵ and R²⁷ are independently H, OH, =O, pharmacologically acceptable ester groups or pharmacologically acceptable ether groups;
R¹⁵ is H when C-5,C-6 is a single bond, and nothing when C-5,C-6 is a double bond; A is either O concurrently with B being CH₂, or B is O concurrently with A being CH₂;
R^{37A} is H concurrently with R^{37B} being CH₃, or R^{37A} is CH₃ concurrently with R^{37B} being H;
or a pharmaceutically acceptable salt, or derivative thereof. 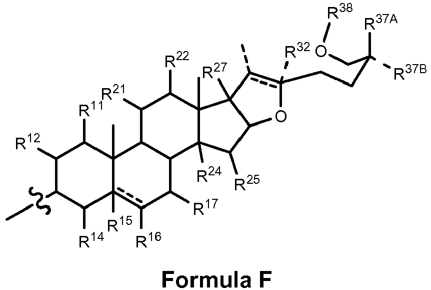 wherein
R¹¹, R¹², R¹⁴, R¹⁶, R¹⁷, R²¹, R²², R²⁴, R²⁵ and R²⁷ are independently H, OH, =O, pharmacologically acceptable ester groups or pharmacologically acceptable ether groups;
R¹⁵ is H when C-5,C-6 is a single bond, and nothing when C-5,C-6 is a double bond;
R³² is either a hydroxyl or an alkoxyl group when C-20, C-22 is a single bond, or nothing when C-20, C-22 is a double bond;
R^{37A} is H concurrently with R^{37B} being CH₃, or R^{37A} is CH₃ concurrently with R^{37B} being H;
R³⁸ is H or a saccharide; or a pharmaceutically acceptable salt, or derivative thereof;
or a pharmaceutically acceptable salt, or derivative thereof. 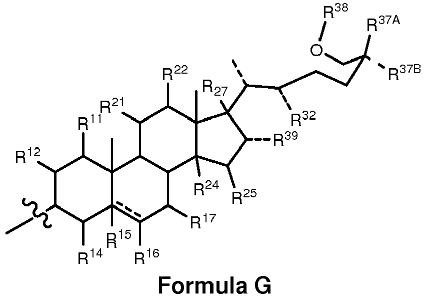 wherein
R¹¹, R¹², R¹⁴, R¹⁶, R¹⁷, R²¹, R²², R²⁴, R²⁵ and R²⁷ are each independently H, OH, =O, pharmacologically acceptable ester groups or pharmacologically acceptable ether groups;
R¹⁵ is H when C-5,C-6 is a single bond, and nothing when C-5,C-6 is a double bond;
R³² and R³⁹ are each independently H, OH, =O, pharmacologically acceptable ester groups or pharmacologically acceptable ether groups;
R^{37A} is H concurrently with R^{37B} being CH₃, or R^{37A} is CH₃ concurrently with R^{37B} being H;
R³⁸ is H or a saccharide; or a pharmaceutically acceptable salt, or derivative thereof;
or a pharmaceutically acceptable salt, or derivative thereof.

The sapogenin at R¹ is selected from the group consisting of spirostanol aglycones and furostanol aglycones selected from the group consisting of diosgenin, yamogenin (neodiosgenin), yuccagenin, sarsasapogenin, tigogemn, smilagenin, hecogenin, gitogemn, convallamarogenin, neoruscogenin, solagenin, protodiosgenin, pseudoprotodiosgenin, methyl protodiosgenin, protoyamogenin, methyl protoyamogenin, and pharmaceutically acceptable salts, hydrates thererof.

It is understood that compounds referred to herein are intended to include pharmaceutically acceptable salts, hydrates and solvates thereof. As such, in some aspects the compound of the present invention may be any pharmaceutically acceptable salt that retains the desired biological activity of the above-identified compounds. In some aspects pharmaceutically acceptable salts include acid addition salts and base addition salts. Where the salt is an acid addition salt, the salt may be prepared from either organic or inorganic acids. Similarly, where the salt is a base addition salt, it may be prepared by addition of organic or inorganic bases. In some aspects, compounds of the present invention may be prepared from inorganic acids such as hydrochloric, sulfuric, and phosphoric acid. In other aspects, the pharmaceutically acceptable salts of the present invention may be prepared from organic acids such as aliphatic, cycloaliphatic, aromatic, heterocyclic carboxylic and sulfonic classes of organic acids, examples of which are formic, acetic, propionic, succinic, glycolic, gluconic, lactic, malic, tartaric, citric, fumaric, maleic, alkyl sulfonic and arylsulfonic. Furthermore, in other aspects the compounds of the present invention may be prepared by addition of metallic salts made including lithium, sodium, potassium, magnesium, calcium, aluminium, and zinc, or by addition of organic bases such as benzathine, chloroprocaine, choline, diethanolamine, ethanolamine, ethyldiamine, meglumine, and procaine. In further aspects compounds of the present invention may be organic salts including ammonium salts, quaternary salts such as tetramethylammonium salt; amino acid addition salts such as salts with histidine, glycine, lysine and arginine. In some other aspects compounds of the present invention may be a salt selected from the group consisting of sodium, potassium, ammonium, tetraalkyl ammonium, ethanolamine, diethanolamine, phosphate, and choline.

The base in step (i) may be selected from any nucleophilic or non-nucleophilic base, including organic and inorganic bases. Examples of suitable inorganic bases include alkali earth metal carbonates, alkali earth metal acetates, alkali earth metal hydroxides and alkali earth metal alkyloxides. Specific examples of suitable inorganic bases include sodium carbonate, sodium bicarbonate, potassium carbonate, cesium carbonate, potassium bicarbonate, sodium acetate, potassium acetate, sodium hydroxide, potassium hydroxide, sodium methoxide, and sodium ethoxide. Suitable organic bases include alkyl and aromatic bases, especially nitrogen-containing alkyl and aromatic bases. In some embodiments, the base is a tertiary amine or an aromatic amine, especially a hindered tertiary amine. Examples of suitable organic bases include trialkyl amines, such as trimethylamine, triethylamine, and diisopropylethylamine; heteroaromatic bases, typically nitrogen-containing heteroaromatic bases, such as optionally substituted imidazoles, optionally substituted pyridines such as pyridine, 4-dimethylaminopyridine and 2,6-lutidine; and cyclic- and poly-cyclic nitrogen-containing bases such as optionally substituted piperidine, including N-formyl piperidine, 2,2,6,6-tetramethylpiperidine, and 1,8-diazabicycloundec-7-ene. In some embodiments the base is pyridine or an optionally substituted pyridine derivative such as 4-dimethylaminopyridine.

The amount of base chosen will depend upon the steroid saponin, the acylating agent, the solvent (if any), the temperature, and the desired speed of reaction; but is chosen to ensure the method provides the desired regioisomer. Typically an excess of base on a molar equivalent is used. In some embodiments the amount of base 1 to 3 molar equivalents. In other embodiments the amount is from 1 to 2 molar equivalents. In still other embodiments the amount of base is 1 to 1.5 molar equivalents. In other embodiments the amount of base is 1.2 molar equivalents.

Furthermore, method step (i) may be carried out in the presence of a suitable solvent or may be conducted solvent-less. In some cases the base in step (i) may also be the solvent, for example, where the base is an optionally substituted pyridine or optionally substituted piperidine. In some embodiments a suitable solvent is a solvent capable of solvating a glycosylated steroid saponin. In other embodiments, the suitable solvent is a solvent capable of solvating a compound of Formula A. In other embodiments, a suitable solvent is selected from hydrocarbon solvents, halogenated solvents or mixtures thereof. Examples of suitable solvents include, but are not limited to, acetonitrile, acetone, benzene, benzonitrile, 1-butanol, 2-butanol, tert-butyl alcohol, butyl acetate, carbon tetrachloride, chlorobenzene, chloroform, cyclohexane, cyclopentane, 1,2-dichlorobenzene, dichloromethane, 1,2-dichloroethane, diethyl amine, diethyl ether, diethyl ketone, dimethoxyethane diethylene glycol, diethylene glycol dimethyl ether, N,N-dimethylacetamide, N,N-dimethylformamide, dimethyl sulfoxide, 1,4-dioxane, ethylene glycol, ethyl acetate, hexane, heptanes, 2-methoxyethanol, 2-methoxyethyl acetate, methyl acetate, 1-octanol, isopropanol, 1-propanol, ethanol, methanol, tetrachloroethylene, 1,1,2-trichlorotrifluoroethane, 2,2,2-trifluoroethanol, tetrahydrofuran, toluene, triethyl amine, pentane, petroleum ether, pyridine, water, o-xylene, p-xylene, m-xylene, and mixtures thereof. In other embodiments a suitable solvent is selected from the group consisting of dichloromethane, tetrahydrafuran, 1,4-dioxane, dichloroethane, chloroform, carbon tetrachloride and pyridine and mixture thereof. In some embodiments, the solvent is a halogenated solvent. In some embodiments, the solvent is dichloromethane. In other embodiments, the solvent is a dry solvent, or is substantially free from water.

Method step (i) may be carried out at any suitable temperature although it is typically conducted at from -100 to 80°C. In other embodiments, a suitable temperature to conduct method step (i) may be at from -85 to 40°C. In other embodiments, a suitable temperature to carry out method step (i) at from -80 to 25°C. In some embodiments, the method is conducted over a range of temperatures, especially where the temperature is increased over the course of the reaction. In some preferred embodiments the temperature of the reaction in method step (i) is initially in the range of -85 to -70°C, and the temperature is subsequently increased over the course of the reaction to a temperature in the range of 10 to 25°C. In some embodiments the temperature of the reaction in method step (i) is initially in the range of -10 to 20°C and then subsequently increased over the course of the reaction to a temperature in the range of 10 to 25°C. In some embodiments the temperature of the reaction in method step (i) is in the range of from 0 to 20°C. In some embodiments the temperature of the reaction in method step (i) is in the range of from 5 to 15°C. In some embodiments the temperature of the reaction in method step (i) is in the range of from 8 to 13°C.

The method of step (i) may be conducted at atmospheric pressures or may be conducted at pressures greater than or less than atmospheric pressures.

In some embodiments, method of step (i) may be conducted under an inert atmosphere. Where the method step (i) is conducted under an inert atmosphere, the atmosphere may be Nitrogen (N₂) or Argon (Ar).

The method of step (i) of the present invention typically takes from less than 1 minute to 6 hours, more typically from 10 minutes to 3 hours, most typically from 30 minutes to 2.5 hours. In some aspects, the method of the present invention is conducted over 1 to 2 hours. As will be appreciated, however, it is quite easy for a skilled addressee to monitor the reaction.

The acylating agent and the compound of Formula A may be reacted in any of a number of ratios although the ratio is typically from 5:1 to 0.8:1. In other aspects, the ratio of acylating agent to a compound of Formula A is from 3:1 to 1:1. In other aspects, the ratio of acylating agent to a compound of Formula A is from 2:1 to 1:1. In other aspects, the ratio of acylating agent to a compound of Formula A is from 1.3:1 to 1:1. In other aspects, the ratio of acylating agent to a compound of Formula A is from 1.2:1 to 1:1. In still other aspects, the ratio of acylating agent to a compound of Formula A is from 1.1:1 to 1:1.

As previously described, in some aspects the present invention provides, in part, methods of coupling as indicated in and limited by the claims.

Saccharide coupling reactions generally fall into two categories, according to how the saccharide moiety to be coupled is activated.

The first general category of reaction uses a saccharide trihaloacetimidate (e.g. trichloroacetimidate) as the activated saccharide moiety for coupling. The activated saccharide is coupled, via the activated carbon centre, to a glycoside acceptor, namely another saccharide molecule (via an unprotected OH group thereof) or a diketone (via an unprotected OH group thereof, including an unprotected OH group of a saccharide moiety of the diketone). In some embodiments, a suitable catalyst may also be used.

The second general category of reaction uses a thioglycoside (e.g. thioethyl saccharide or thio-2-propyl saccharide) as the activated saccharide moiety for coupling, in the presence of a suitable catalyst such as a catalytic amount of N-iodosuccimide (NIS) and an iodine, silyl or silver cocatalyst.

The methods of the present invention encompass each general category of coupling reaction, as far as falling within the scope of the claims.

It is possible to first assemble a di- or higher polysaccharide and then subsequently couple the di- or higher polysaccharide to a steroid or sapogenin molecule. Alternately, it is possible to first assemble a mono-glycoside saponin and then couple additional saccharides to form di- or poly-glycoside saponins of the present invention. Either of these methods is applicable to the present invention. Typically, the present invention provides initial assembly of a mono-glycoside saponin and then subsequent coupling of additional saccharides to form the desired di- or poly-glycoside saponins.

Examples of suitable saccharides include a mono aldose or ketose having 5 or 6 carbon atoms, preferably in the cyclised furanose or pyranose form, either as α- or β-anomer and having D or L optical isomerism. Examples of suitable saccharides include glucose, mannose, fructose, galactose, maltose, cellobiose, sucrose, rhamnose, xylose, arabinose, fucose, quinovose, apiose, lactose, galactose-glucose, glucose-arabinose, fucose-glucose, rhamnose-glucose, rhamnose-galactose, glucose-glucose-glucose, glucose-glucose-galactose, gluctose-rhamnose, mannose-glucose, rhamnose-(glucose)-glucose, rhamnose-(rhamnose)-glucose, glucose-(rhamnose)-glucose, glucose-(rhamnose)-galaclose, giucose-(rhamnose)-rhamnose, galactose-(rhamnose)-galactose, and protected, typically acylated, derivatives thereof, as far as covered by the claims.

As such, the coupling conditions of method of step (ii) may be any suitable coupling conditions. As previously described, in some embodiments, the compound of Formula C in method step (ii) is an activated saccharide. The choice of activated saccharide will depend upon the desired steroid saponin. Suitable leaving groups for activation of the saccharide of Formula C have been discussed with respect to R⁸.

In some embodiments, method of step (ii) uses a saccharide trihaloacetimidate (e.g. trichloroacetimidate or *N*-(phenyl)trifluoroacetimidate) as the activated saccharide of Formula C. In other embodiments, method of step (ii) uses a thioglycoside as the activated saccharide of Formula C. In still other embodiments, method step C uses a haloglycoside as the the activated saccharide of Formula C. In still other embodiments, the activated saccharide of Formula C may be any of suitable saccharide previously described, or derivatives thereof.

Furthermore, method step (ii) may be carried out in the presence of a suitable solvent or may be conducted solvent-less. In some embodiments a suitable solvent is a solvent capable of solvating a glycosylated steroid saponin. In other embodiments, the suitable solvent is a solvent capable of solvating a compound of Formula A or a compound of Formula C. In other embodiments, a suitable solvent is selected from hydrocarbon solvents, halogenated solvents or mixtures thereof. Examples of suitable solvents include, but are not limited to, acetonitrile, acetone, benzene, benzonitrile, 1-butanol, 2-butanol, tert-butyl alcohol, butyl acetate, carbon tetrachloride, chlorobenzene, chloroform, cyclohexane, cyclopentane, 1,2-dichlorobenzene, dichloromethane, 1,2-dichloroethane, diethyl amine, diethyl ether, diethyl ketone, dimethoxyethane diethylene glycol, diethylene glycol dimethyl ether, N,N-dimethylacetamide, N,N-dimethylformamide, dimethyl sulfoxide, 1,4-dioxane, ethylene glycol, ethyl acetate, hexane, heptanes, 2-methoxyethanol, 2-methoxyethyl acetate, methyl acetate, trichlorotrifluoroethane, 2,2,2-trifluoroethanol, tetrahydrofuran, toluene, triethyl amine, pentane, petroleum ether, pyridine, water, o-xylene, p-xylene, m-xylene, and mixtures thereof. In other embodiments a suitable solvent is selected from the group consisting of dichloromethane, tetrahydrafuran, 1,4-dioxane, dichloroethane, chloroform, carbon tetrachloride and pyridine and mixture thereof. In some embodiments, the solvent is a halogenated solvent. In some embodiments, the solvent is dichloromethane. In other embodiments, the solvent is a dry solvent, or is substantially free from water.

Method step (ii) may be carried out at any suitable temperature although it is typically conducted at from -100 to 80°C. In other embodiments, a suitable temperature to conduct method step (ii) may be at from -85 to 40°C. In other embodiments, a suitable temperature to carry out method step (i) at from -80 to 25°C. In some embodiments, the method is conducted over a range of temperatures, especially where the temperature is decreased and/or increased over the course of the reaction. In some preferred embodiments the temperature of the reaction in method step (ii) is initially in the range of 0 to 40°C, the temperature is then decreased to a temperature in the range of -80 to -25°C, and subsequently increased to a temperature in the range of -25 to 10°C. In other embodiments the temperature of the reaction in method step (ii) is initially in the range of -50 to 0°C, and the temperature is subsequently increased to a temperature in the range of 0 to 40°C.

The method of step (i) may be conducted at atmospheric pressures or may be conducted at pressures greater than or less than atmospheric pressures.

The method of step (i) of the present invention typically takes from less than 1 minute to 48 hours, more typically from 5 minutes to 3 hours, most typically from 30 minutes to 2.5 hours. In some aspects, the method of the present invention is conducted over 1 to 2 hours. As will be appreciated, however, it is quite easy for a skilled addressee to monitor the reaction.

In some embodiments, method of step (ii) may be conducted under an inert atmosphere. Where the method step (ii) is conducted under an inert atmosphere, the atmosphere may be Nitrogen (N₂) or Argon (Ar).

As previously described, the method of step (ii) may be conducted in the presence of a catalyst. The catalyst may be any suitable catalyst. In some embodiments, the catalyst may be a Lewis acid, a Lewis base, a Brønsted acid or a Bronsted base. In still other embodiments, the catalyst may be a reagent that can activate a saccharide, for example, the catalyst may be a reagent that forms a leaving group *in situ.* In some embodiments, the catalyst may be a silylating agent. In still other embodiments, the catalyst may be both a Lewis acid and a silylating agent. In other embodiments, the catalyst is trimethylsilyl trifluoromethanesulfonate.

As previously described, removal of protecting groups can be achieved in conventional manner according to the nature of the protecting group. In some embodiments, one protecting group may be selectively removed, whilst other protecting groups remain intact. The use and removal of appropriate protecting groups will be well within the capacity of those skilled in this art.

For example, treatment of a compound which comprises a saccharide moiety with residues protected by acetyl or benzoyl protecting groups, with NaOMe in methanol at around pH 10 with stirring, for a period of time, typically 1-8 hours, at room temperature results in complete de-*O*-acetylation or de-*O*-benzoylation, that is, removal of the acetyl and/or benzoyl protecting groups. Such a deprotection provides, in this instance, free hydroxyl groups.

As discussed previously therefore the de-protection regime chosen in each instance will depend upon the exact nature of the oxygen protecting groups located at R², R³, R⁴, R⁶ and R⁷, and as a possible component of R⁹ and the acyl group at R⁵. In some instances all oxygen protecting groups can be removed in a single operation thus leading to manufacturing efficiencies. In other instances due to the nature and identity of these groups it is necessary to manipulate the protecting groups in a multi-step operation to arrive at the desired product. It is noted that a skilled addressee equipped with a competent working knowledge of protecting group chemistry would be readily able to analyse the compounds to be deprotected and from the identity of the protecting groups devise a de-protection protocol to arrive at the desired end product.

In other embodiments, for example, treatment of a compound which comprises a saccharide moiety protected, in part, by an acetal, such as para-methoxy phenyl (PMP), with anionic ion exchange resin in methanol and tetrahydrafuran results in selective removal of the para-methoxy phenyl protecting group only. As such, where the compound comprising a saccharide moiety also comprises other protected residues, such as benzoyl and acetyl protected hydroxyl groups, these other protecting groups remain intact. Suitable anionic ion exchange resins include those sold under the names Amberjet™, Amberlite™ and Ambersep™.

In some aspects, the compound of formula X is Compound X-1:

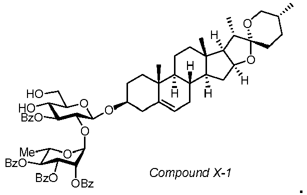

Where the compound of Formula X is Compound X-1, it may be accessed by the method of the present invention, as follows:

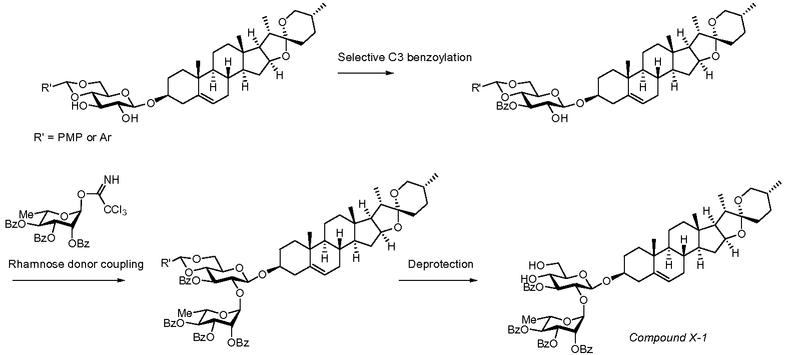

That is the acetal protected compound is subjected to selective C3 benzoylation to achieve the desired mono-benzoylated derivative. This is then subjected to Rhamnose coupling with a suitably activated saccharide to produce the desired coupled product which is then reacted to remove the acetal protecting group. This product can be further elaborated by a number of different reactions to provide entry to a large number of structurally related compounds.

Advantageously, the present method provides access to a range of steroid saponins. For example, wherein the compound of Formula X is Compound X-1, the present method provides access to the following desired steroid saponins:

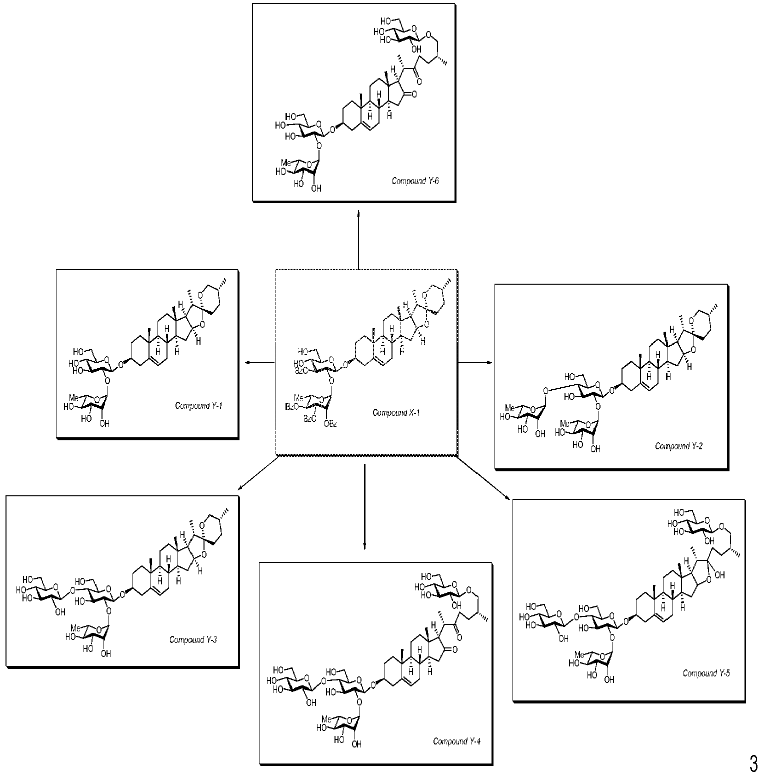

In an embodiment, the present invention provides a method further including converting a compound of Formula X into a compound of Formula Y. A compound of Formula X may be converted to a compound of Formula Y by methods recognised in the art, including but not limited to functional group interconversions, oxidations, reductions, alkylation, protections, deprotections and the coupling of additional saccharide units.

For example, in some aspects, the compound of Formula Y may be prepared, according to the present methods, wherein steps (i) to (iv) and R¹, R², R³, R⁴, R⁵, R⁶ R⁷ and R⁹ are as previously described for Formula A, B, C, X and Y.

In some aspects, a compound of Formula X may be converted to a compound of Formula Y by removing any protecting groups from a compound of Formula X to provide a compound of Formula Y. In some aspect, removal of protecting groups can be achieved in conventional manner according to the nature of the protecting group. In some aspects, treating a compound of Formula X with a base will provide a compound of Formula Y. The base may be selected from any suitable base. In some embodiments the base may be selected from any nucleophilic or non-nucleophilic base, including organic and inorganic bases. In still other embodiments the base may be selected from those previously described in respect of in step (i) of the present invention. In still other embodiments, the base may be used in combination with a solvent, or may be used in the absence of a solvent. Where a solvent is used, the solvent may be selected from any suitable solvent. For example, in some embodiments, treating a compound of Formula X with K₂CO₃ in methanol will provide a compound of Formula Y. In other embodiments treating a compound of Formula X with NH₃ in methanol will provide a compound of Formula Y. In still other embodiments, treating a compound of Formula X with NaOH in dichloromethane and/or methanol will provide a compound of Formula Y.

In an embodiment, a compound of Formula X may be converted to a Formula Y by
(a) selectively introducing an oxygen protecting group at R⁷;
(b) coupling a suitable saccharide under coupling conditions such that R⁶ is a saccharide;
   to provide a compound of Formula X' 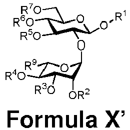
   wherein R¹ is a sapogenin;
   R², R³, R⁴ and R⁷ are each independently an oxygen protecting group;
   R⁵ is an acyl group selected from the group consisting of benzoyl, 2-chlorobenzoyl, 4-chlorobenzoyl, 4-nitrobenzoyl and 4-methoxybenzoyl;
   R⁶ is a saccharide;
   R⁹ is selected from the group consisting of H, CH₃, and an oxygen protected by an oxygen protecting group; and
(c) converting a compound of Formula X' into a compound of Formula Y, a pharmaceutically acceptable salt, hydrate or solvate thereof.

In other aspects, a compound of Formula X may be converted to a compound of Formula Y by:
(a) selectively introducing an oxygen protecting group at R⁷;
(b) coupling a suitable saccharide under coupling conditions such that R⁶ is a saccharide to provide a compound of Formula X' as previously defined; and
(c) removing any protecting groups to provide a compound of Formula Y.

In other aspects, a compound of Formula X may be converted to a compound of Formula Y by:
(a) selectively introducing an oxygen protecting group at R⁷;
(b) coupling a suitable saccharide under coupling conditions such that R⁶ is a saccharide to provide a compound of Formula X' as previously defined;
(c) converting the sapogenin at R¹ into a sapogenin of Formula G as previously defined; and
(d) removing any protecting groups to provide a compound of Formula Y.

In still other embodiments, a compound of Formula X may be converted to a compound of Formula Y by:
(a) selectively introducing an oxygen protecting group at R⁷;
(b) coupling a suitable saccharide under coupling conditions such that R⁶ is a saccharide to provide a compound of Formula X' as previously defined;
(c) converting the sapogenin at R¹ into a sapogenin of Formula F as previously defined; and
(d) removing any protecting groups to provide a compound of Formula Y.

In a comparative example, a compound of Formula Y wherein R¹ is a sapogenin of Formula G, may be prepared from a compound of Formula X by oxidising a compound of the Formula Y^{A} 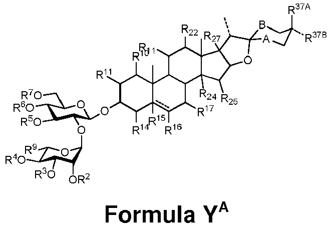
R², R³, R⁴, R⁵, and R⁷ are each independently an oxygen protecting group;
R⁶ is an oxygen protecting group or a saccharide;
R⁹ is selected from the group consisting of H, Me, and an oxygen protected by an oxygen protecting group;
R¹¹, R¹², R¹⁴, R¹⁶, R¹⁷, R²¹, R²², R²⁴, R²⁵ and R²⁷ are independently H, OH, =O, pharmacologically acceptable ester groups or pharmacologically acceptable ether groups;
R¹⁵ is H when C-5,C-6 is a single bond, and nothing when C-5,C-6 is a double bond;
A is either O concurrently with B being CH₂, or B is O concurrently with A being CH₂;
R^{37A} is H concurrently with R^{37B} being CH₃, or R^{37A} is CH₃ concurrently with R^{37B} being H;
to provide a compound of Formula Y^{B} 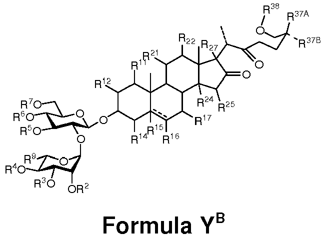
wherein R², R³, R⁴, R⁵, R⁶, R⁷, R⁹, R¹1, R¹², R¹⁴, R¹⁵, R¹⁶, R¹⁷, R²¹, R²², R²⁴, R²⁵, R²⁷, R^{37A} and R^{37B} are as defined for Formula Y^{A}; and
R³⁸ is H or a saccharide; or a pharmaceutically acceptable salt, or derivative thereof;
followed by removable of any protecting groups to provide a compound of Formula Y.

In other comparative examples, a compound of Formula Y wherein R¹ is a sapogenin of Formula F, may be prepared by selectively reducing a compound of Formula Y^{B} 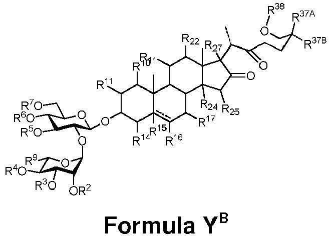
R², R³, R⁴, R⁵, R⁶, R⁷, R⁹, R¹¹, R¹², R¹⁴, R¹⁵, R¹⁶, R¹⁷, R²¹, R²², R²⁴, R²⁵, R²⁷, R^{37A} and R^{37B} are as defined for Formula Y^{A};
R³⁸ is H or a saccharide; or a pharmaceutically acceptable salt, or derivative thereof; to provide a compound of Formula Y^{C}

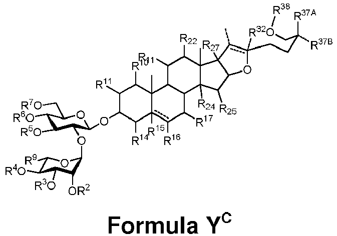

R², R³, R⁴, R⁵, R⁶, R⁷, R⁹, R¹¹, R¹², R¹⁴, R¹⁵, R¹⁶, R¹⁷, R²¹, R²², R²⁴, R²⁵, R²⁷, R^{37A} and R^{37B} and R³⁸ are as defined for Formula Y^{B};
followed by removable of any protecting groups to provide a compound of Formula Y.

In some embodiments, the compound of Formula Y is selected from the group consisting of 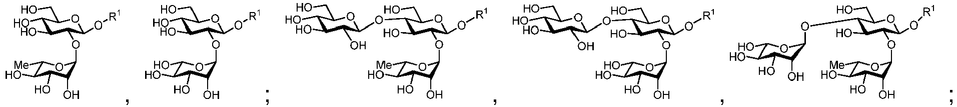 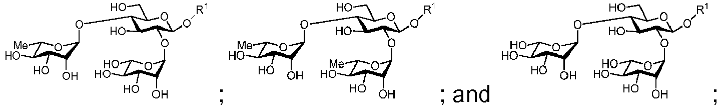 where R¹ is a sapogenin.

In other embodiments, the sapogenin at R¹ is selected from the group consisting of: 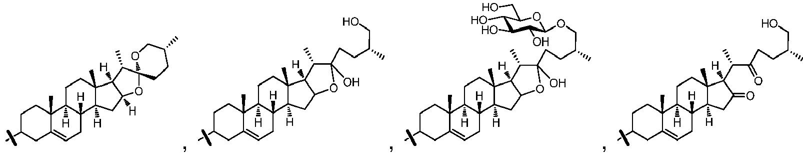 and

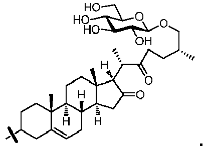

Accordingly, in some embodiments the compound of Formula Y may be selected from the group consisting of: 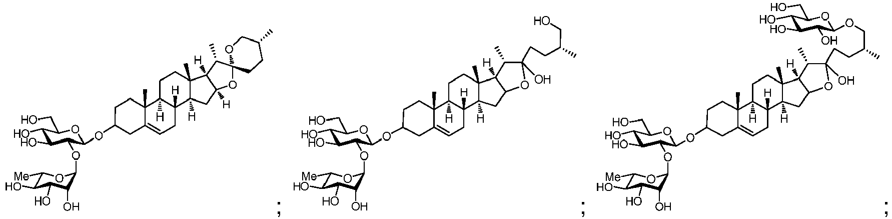 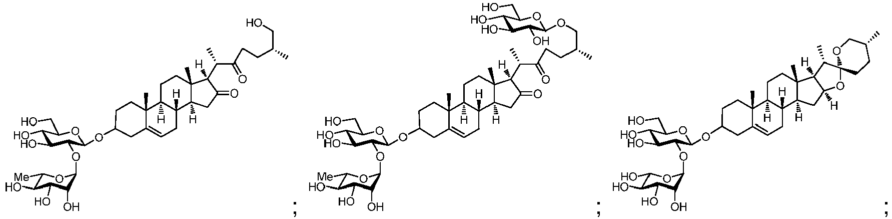 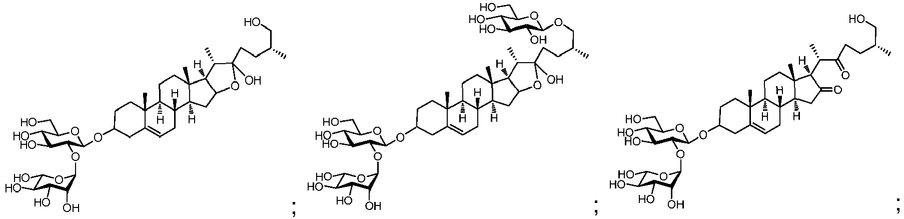 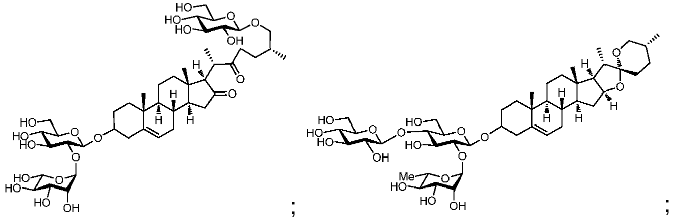 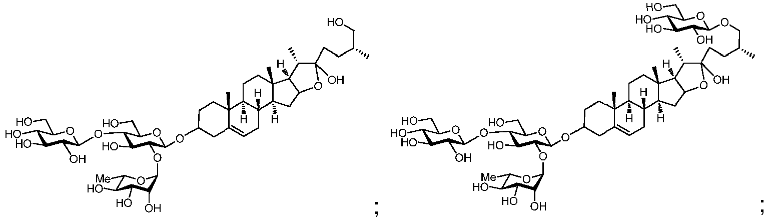 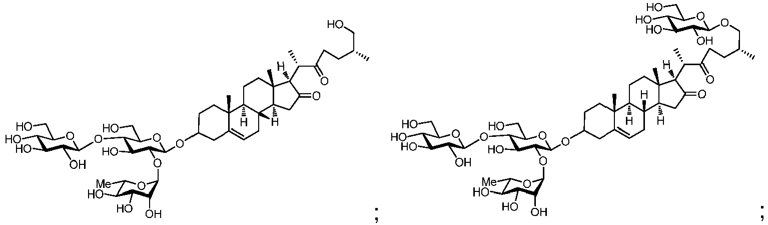 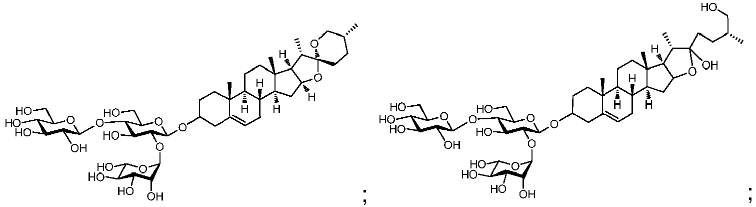 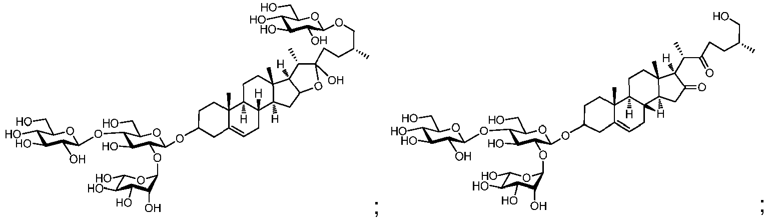 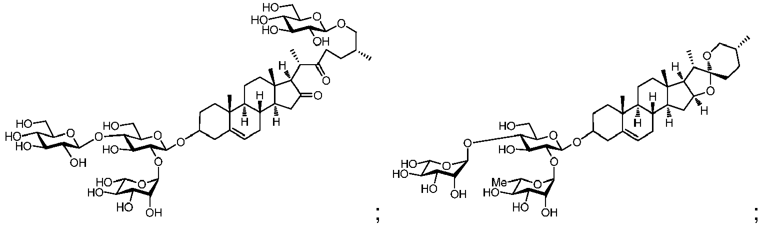 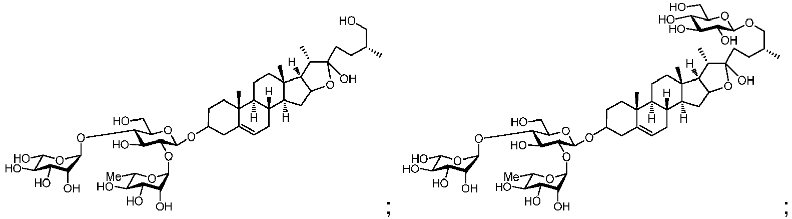 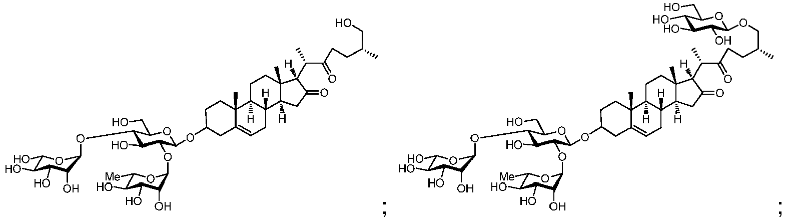 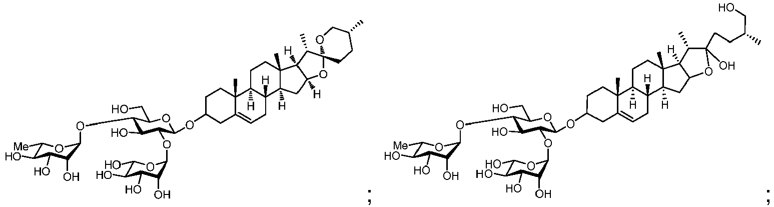 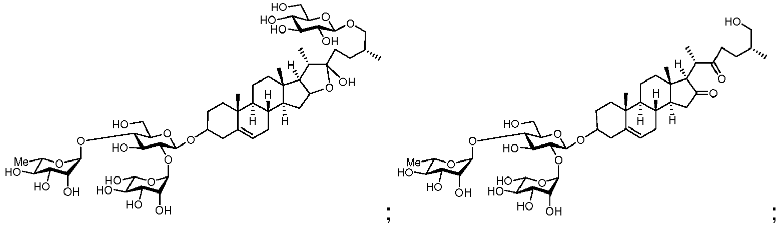 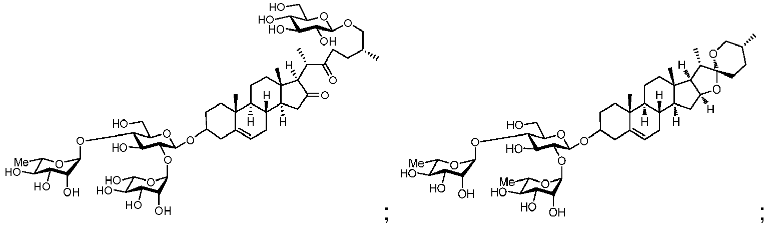 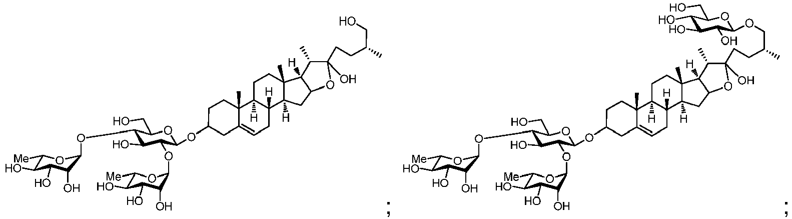 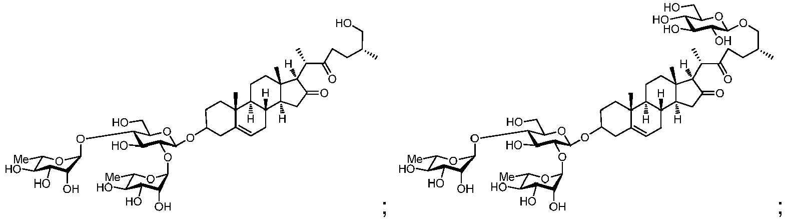 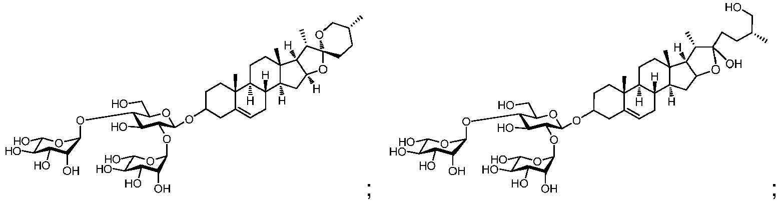 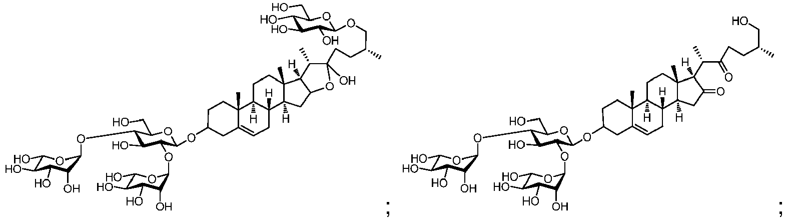 and

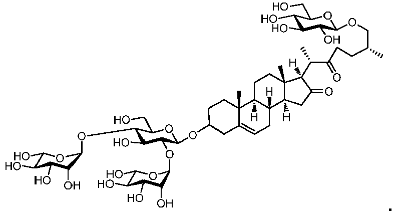

In still other embodiments, the compound of Formula Y is selected from

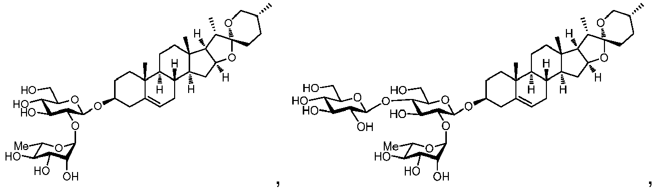

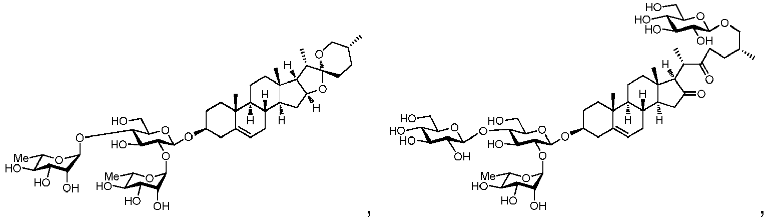

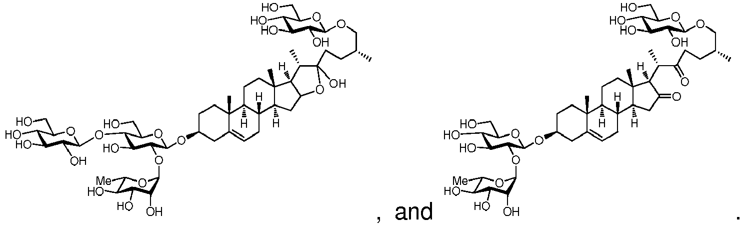

The invention will now be illustrated by way of examples according to the present invention and comparative examples; however, the examples are not to be construed as being limitations thereto. Additional compounds, other than those described below, may be prepared using methods and synthetic protocols or appropriate variations or modifications thereof, as described herein. The invention is limited by the claims.

### Examples

In the examples described below, unless otherwise indicated, all temperatures in the following description are in degrees Celsius and all parts and percentages are by weight, unless indicated otherwise.

Various starting materials and other reagents were purchased from commercial suppliers, such as Aldrich Chemical Company or Lancaster Synthesis Ltd., and used without further purification, unless otherwise indicated. All solvents were purified by using standard methods in the art, unless otherwise indicated.

¹H NMR spectra were recorded on a Bruker Avance III-500 at 500 MHZ, and ¹³C-NMR spectra were recorded on a Bruker Avance III-500 at 126 MHZ. When peak multiplicities are reported, the following abbreviations are used: s = singlet, d = doublet, t = triplet, m = multiplet, br = broadened, dd = doublet of doublets, dt = doublet of triplets. Coupling constants, when given, are reported in Hertz.

Mass spectra were obtained using Waters Q-TOF Premier™ Tandem Mass Spectrometer with electro-spray ionisation.

### Example (1) (Comparative Example):

### Preparation of Diosgenyl-(4,6-O-(4-methoxybenzylidene)-3-benzoyl)-β-D-glucopyranoside (Precursor 1)

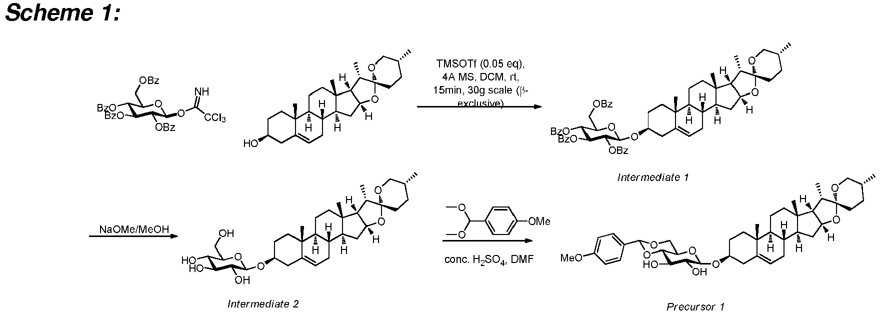

### Intermediate 1: Preparation of 2,3,4,6-tetra-O-benzoyl-β-D-glucopyranose (Intermediate 1)

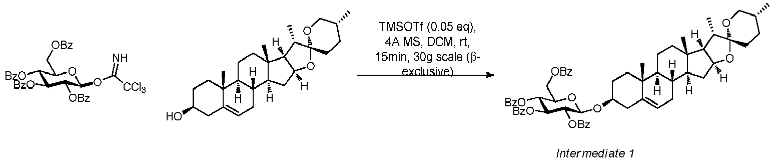

2,3,4,6-Tetra-O-benzoyl-D-glucopyranoside trichloroacetimidate (50.3 g, 67.9 mmol) and Diosgenin (26.0 g, 63 mmol) were dissolved in a mixture of dichloromethane (anhydrous, 11 mL) and toluene (anhydrous, 314 mL) and the solution dried by rotary evaporation at 40°C. The product was dissolved in dichloromethane (anhydrous, 222 mL) and cooled to 0°C under dry nitrogen. TMSOTf (0.250 mL, 1.38 mmol) was added and the solution warmed to ambient temperature and stirred for 1 h. The reaction was then quenched with N-methylmorpholine (0.343 mL, 3.1 mmol). Additional DCM was added (20 mL) and the product precipitated by the slow addition of methanol (450 mL) and the subsequent slow addition of a mixture of methanol and water (200 mL of 3:1 methanol:water). The product was collected by filtration, washed with a mixture of methanol and water (450 mL of 4:1 methanol:water) and dried under vacuum to give diosgenyl 2,3,4,6-tetra-O-benzoyl-β-D-glucopyranose (Intermediate 1).

¹H NMR 500 MHz (CDCl₃) δ 7.81-8.03 (m, 8H), 7.23-7.56 (m, 12 H), 5.89 (t, 1H, *J*=9.7 Hz), 5.62 (t, 1H, *J*=9.7 Hz), 5.49 (dd, 1H, *J*=7.9, 9.7 Hz), 5.22 (m, 1 H), 4.94 (d, 1H, *J*=7.9 Hz), 4.60 (dd, 1H, *J*=3.4, 12.0 Hz), 4.52 (dd, 1H, *J*=5.9, 12.0 Hz), 4.37-4.43 (m, 1H), 4.12-4.18 (m, 1H), 3.34-3.56 (3H, M), 0.74-2.20 (m, 36 H). ES-MS *m*/*z* C₆₁H₆₈O₁₂Na calcd 1015.4608, found 1015.4604.

### Intermediate 2: Preparation of Diosgenyl-β-D-glucopyranoside (Intermediate 2)

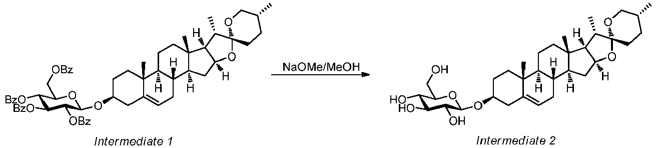

Under nitrogen, diosgenyl 2,3,4,6-tetra-O-benzoyl-β-D-glucopyranose (Intermediate 1) (59 g, 59.4 mmol) was dissolved in dichloromethane (dry, 400 mL) and methanol (dry, 400 mL). Sodium methoxide (30% in methanol, 1.9 mL, 10.1 mmol) was added and the solution stirred overnight. If during this time the pH fell below 9 then additional sodium methoxide was added. The product containing solution was neutralised with washed acidic ion-exchange resin (Amberjet 1200H). The resin was removed by filtration and any residual acidity quenched with *N*-methylmorpholine. The product was dried to a syrup by rotary evaporation, the syrup suspended in methanol (275 mL) to give a filterable solid which was then collected by filtration. The solid was washed with methanol (165 mL) and ethyl acetate (165 mL). The product was dried under vacuum at 30°C to give Diosgenyl-β-D-glucopyranoside (Intermediate 2) (23.4 g, 68%) as a sesqui hydrate.

The filtrates were combined, ethyl acetate added (330 mL) and the mixture concentrated to approximately 190 mL. A second crop of product was collected by filtration and washed with ethyl acetate (100 mL). The second crop of product is further purified by column chromatography (eluent 1:9 Methanol:dichloromethane) to provide more Diosgenyl-β-D-glucopyranoside (Intermediate 2) (5.21 g, 15.2%) (total yield = 28.6 g, 83.4%).

¹H NMR (500 MHz, 3:1 CDCl₃/CD₃OD): δ5.37 (dd, *J* = 2.1, 3.1 Hz, 1 H), 4.42 (q, *J* = 7.4 Hz, 1 H), 4.40 (d, *J* = 7.8 Hz, 1 H), 3.84 (dd, *J* = 2.9, 12.0 Hz, 1 H), 3.83 (dd, *J* = 4.7, 12.0 Hz, 1 H), 3.58 (m, 1 H), 3.47 (ddd, *J* = 2.1, 4.2, 11.6 Hz, 1 H), 3.45-3.20 (m, 5 H), 2.41 (ddd, *J* = 2.1, 4.7, 13.2 Hz, 1 H), 2.27 (m, 1 H), 2.05-0.92 (m, 23 H), 1.03 (s, 3 H), 0.97 (d, *J* = 6.9 Hz, 3 H), 0.80 (d, *J* = 6.3 Hz, 3 H), 0.80 (s, 3 H); ¹³C NMR (126 MHz, 3:1 CDCl₃/CD₃OD): δ141.78, 123.08, 110.95, 102.51, 82.36, 80.39, 77.87, 77.25, 74.94, 71.60, 68.22, 63.38, 63.18, 57.85, 51.49, 42.99, 41.63, 41.08, 40.00, 38.57, 38.19, 33.39, 33.02, 32.78, 32.64, 31.54, 30.89, 30.00, 22.18, 20.56, 18.25, 17.50, 15.60. ES-MS *m*/*z* C₃₃H₅₂O₈Na calcd 599.3560, found 599.3554.

### Precursor 1: Preparation of Diosgenyl-(4,6-0-(4-methoxybenzylidene)-3-benzoyl)-β-D-glucopyranoside (Precursor 1)

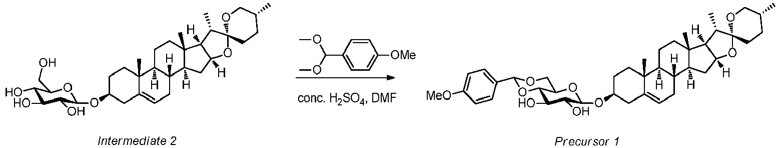

To a solution of Diosgenyl-β-D-glucopyranoside (35.95 g, 62.3 mmol) in DMF (270 mL) was added anisaldehyde dimethyl acetal (42.5 mL, 249 mmol) and 5 drops of concentrated H₂SO₄, pH ∼ 2.5. The solution was heated at 60°C under house vacuum for 8 h to remove the methanol. The reaction was cooled and transferred to a separating funnel with ethyl acetate (400 mL), where it was washed iteratively with H₂O (3 x 300 mL), 0.5 M aqueous HCl (2 x 200 mL) and then sat. aq. NaHCO₃ (200 mL) which caused the precipitation of a grey material at interface of organic and aqueous layers.

This grey material was identified as the desired product contaminated with a small amount of DMF, and was taken up in ethyl acetate and precipitated out with hexanes to give a grey powder (13.89 g, 32%).

The ethyl acetate layer was evaporated under reduced pressure to yield an orange oil which solidified on standing. This orange solid where dissolved in ethyl acetate and precipitated with hexanes to yield a grey powder (13.47 g, 31%).

The orange filtrate could not be induced to precipitate more product instead resulting in an oiling-out due to high 4-methoxybenzaldehyde content which acts as a solvent, so consequently was absorbed onto Celite and columned through a short silica plug eluting with a gradient of 3:1 - 2:1 PE/EA, then 3:1 - 1:1 toluene/EA to give an additional portion of yellow solid (12.62 g 29%; cumulative 39.98 g, 92%).

### Example 2:

### Preparation of a compound of Formula X; Diosgenyl-(2,3,4-tribenzoyl)-a-L-rhamnopyranosyl-(1→2)-3-benzoyl)-β-D-glucopyranoside (Compound X-1)

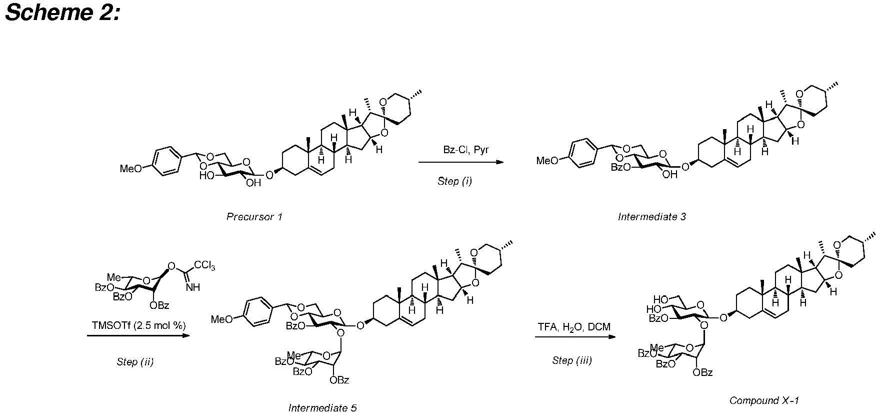

### Intermediate 3: Step (i)

### Selective benzoylation of Precursor 1 to provide Diosgenyl-(4,6-O-(4-methoxybenzylidene)-3-benzoyl)-β-D-glucopyranoside (Intermediate 3)

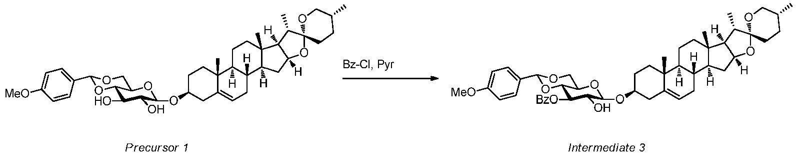

To a solution of Diosgenyl-(4,6-0-(4-methoxybenzylidene)-3-benzoyl)-β-D-glucopyranoside (18.37 g, 26.4 mmol) and DMAP (0.161 g, 1.322 mmol) dissolved in pyridine (42.8 mL) and Dichloromethane (68.0 mL) cooled to -78°C was added benzoyl chloride (3.38 mL, 29.1 mmol, 1.1 equiv) dropwise (transiently forming a chunky solid, which could be Pyr.HCl, before stirring into the reaction volume). The solution was allowed to warm to room temperature with stirring overnight.

The reaction was quenched with the addition of MeOH (10 mL), diluted with 200 mL of DCM and washed with 0.5N HCI (4 x 250 mL), NaHCO₃ (200mL), brine (200 mL), and dried with MgSO₄. The crude material was absorbed onto Celite evaporating with 50 mL of toluene to drive off residual DCM. This was loaded as a slurry in toluene (200 mL) to the top of a silica column and eluted with stepwise gradient of 2% EA/toluene (diBz elution), 5% EA/toluene (intermediate) and then 10% EA/toluene (monoBz).

Collected fractions were combined to yield Diosgenyl-(4,6-0-(4-methoxybenzylidene)-3-benzoyl)-β-D-glucopyranoside (Intermediate 3) (13.11, 62%). Diosgenyl-(4,6-O-(4-methoxybenzylidene)-2,3-dibenzoyl)-β-D-glucopyranoside was also isolated, 3.17 g, 13.3%.

### Intermediate 5: Step (ii)

### Coupling to rhamnose moiety at provide Diosgenyl-(2,3,4-tribenzoyl)-α-L-rhamnopyranosyl-(1→2)-(4,6-O-(4-methoxybenzylidene)-3-benzoyl)-β-D-glucopyranoside (Intermediate 5)

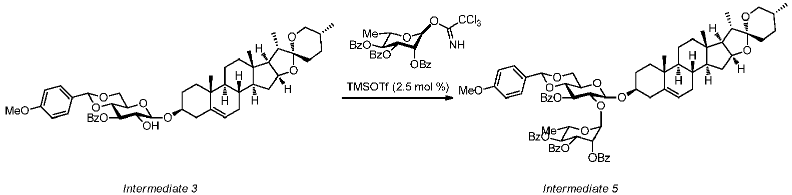

To a solution of Diosgenyl-(4,6-O-(4-methoxybenzylidene)-3-benzoyl)-β-D-glucopyranoside (Intermediate 3) (18.4g, 23.03 mmol), 2,3,4-tri-O-benzoyl-α/β-L-rhamnopyranoside trichloroacetimidate (17.87 g, 28.8 mmol, 1.25 equiv) and 4 Å MS sieves (2 g/g acceptor; 37 g) in DCM (450 mL) stirred at -78°C was added trimethylsilyl trifluoromethanesulfonate (0.104 mL, 0.576 mmol) dropwise immediately forming a bright yellow solution. The reaction was allowed to warm room temperature overnight in the cold bath (lagged with foil) with stirring.

A small aliquot, quenched with 1 drop NEt₃ (- yellow colour disappeared) and evaporated showed the complete consumption of starting materials by ¹H NMR.

The reaction was quenched with addition of NEt₃ (2 mL) and filtered through a bed of Celite to separate the sieves. The solid support was washed with DCM (2 x 50 mL). Evaporation of the organic solution gave a white foam. The foam was slurried with Et₂O (-200 mL), filtered, and washed with cold Et₂O (2 x 50 mL) to yield Diosgenyl-(2,3,4-tribenzoyl)-α-L-rhamnopyranosyl-(1→2)-(4,6-O-(*4-methoxy*benzylidene)-3-benzoyl)-β-D-glucopyranoside as white powder in excellent purity (22.75 g, 79%).

The yellow filtrate was determined to be comprised of decomposed rhamnose donor and a small amount of the desired product. Absorption onto Celite and elution through silica eluting with a gradient of EA/toluene (2%, 4% then 6%) gave an additional 3.07 g (10.6%) of product (cumulative yield 25.82 g, 90%).

### Compound X-1: Step (iii)

### Deprotection of Intermediate 5 to provide a compound of Formula X; Diosgenyl-(2,3,4-tribenzoyl)-α-L-rhamnopyranosyl-(1→2)-3-benzoyl)-β-D-glucopyranoside (Compound X-1)

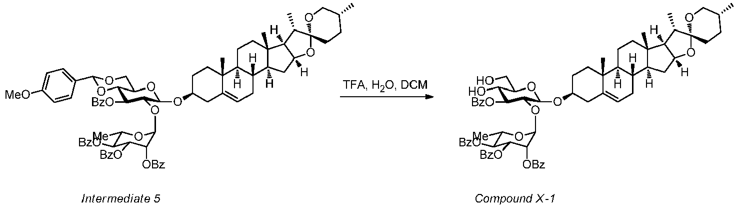

Diosgenyl-(2,3,4-tribenzoyl)-α-L--rhamnopyranosyl-(1→2)-(4,6-O-(4-methoxybenzylidene)-3-benzoyl)-β-D-glucopyranoside (20 g, 15.91 mmol) was dissolved in dichloromethane (125 mL, 1943 mmol) and water (45 mL). The biphasic mixture was stirred as trifluoroacetic acid (15.91 mL) was added at 0°C forming a bright yellow / green fluorescent coloured solution.

The reaction was allowed to stir for 3.5 hours. The reaction was quenched by washing with water (2 x 150 mL), NaHCO₃ (2 x 200 mL), brine (200 mL), dried with MgSO₄ and evaporated to give a white foam.

The crude material was dissolved in a minimum of hot EA (-30 mL) and added dropwise to a PE (500 mL) causing the precipitation of a white solid as a 'stringy' material. The solution was allowed to stir overnight resulting in the formation of a gel. Filtration gave a white solid which was washed with cold 10% EA/PE. (18.202 g, 100%).

Alternatively, Diosgenyl-(2,3,4-tribenzoyl)-α-L-rhamnopyranosyl-(1→2)-(4,6-O-(*4-*methoxybenzylidene)-3-benzoyl)-β-D-glucopyranoside (1.76 g, 1.43 mmol) and Amberjet® 1200 H (8.8 g) were slurried in methanol (24 mL) and tetrahydrofuran (12 mL) in a 100 mL round bottomed flask. The reaction was heated to reflux for 15 h. The reaction was then quenched with triethylamine (0.2 mL). The resin was removed by filtration and the solvent evaporated under reduced pressure. The crude product was dissolved in methanol (25 mL) and water added dropwise (15 mL) resulting in the crystallisation of a white solid. The solid product was isolated by filtration and the cake washed with 1:1 methanol/water (2 x 15 mL) followed by petroleum ether 60-80 (2 x 15 mL). The product was dried overnight under vacuum at 45°C, to give Diosgenyl-(2,3,4-tribenzoyl)- α-L-rhamnopyranosyl-(1-2)-3-benzoyl)-*β*-D-glucopyranoside (Compound X-1), 1.33 g, 81%.

¹H NMR 500 MHz (CDCl₃) δ8.03 (d, 2H), 7.90 (d, 2H), 7.77 (d, 2H), 7.74 (d, 2H), 7.56 (t, J = 7.5 Hz, 1H), 7.53 (t, J = 7.5 Hz, 1H), 7.41 (m, 3H), 7.33 (m, 3H), 7.28 (t, J = 7.5Hz, 2H), 7.23 (t, J=7.5 Hz, 2H), 5.74 (dd, J = 3.6, 10.0 Hz, 1H), 5.48-5.57 (m, 3H), 5.44 (dd, J = 1.6, 3.6 Hz, 1H), 5.16 (d, J = 1.3 Hz, 1H), 4.82 (d, J = 7.9 Hz, 1H), 4.77 (m, 1H), 4.45 (q, 1H), 3.73-3.95 (m, 6H), 3.47-3.54 (m, 2H), 3.38-3.41 (m, 2H), 2.64 (app ddd, 1H), 2.45 (t, 1H), 2.03 (m, 3H), 1.09-1.93 (m, 20H), 1.34 (d, J =6.4 Hz, 3H), 0.99 (d, J =6.4 Hz, 3H), 0.95 (s, 3H), 0.81 (d, J= 6.4 Hz, 3H), 0.80 (s, 3H). ¹³C NMR 126 MHz (CDCl₃,MeOD 3:1) δ 166.3, 165.8, 165.3, 164.7, 139.9, 133.24, 133.17, 133.0. 132.9, 129.61, 129.55, 129.5, 129.3, 129.0, 128.9, 128.23, 128.17, 128.1 122.0, 109.4, 99.4, 97.6, 80.8, 79.0, 78.4, 75.9, 75.5, 71.8, 70.2, 69.6, 68.7, 66.7, 66.6, 61.9, 61.4, 56.3, 49.9, 41.4, 40.1, 39.5, 38.6, 37.0, 36.7, 32.0, 31.6, 31.3, 31.1, 30.0, 29.7, 28.5, 20.6, 19.0, 17.1, 16.8, 16.0. ES-MS *m*/*z* C₆₇H₇₈O₁₆Na calcd: 1161.5188, found 1161.5186

### Example 3 (Comparative Example):

### Preparation of Diosgenyl-(4,6-0-(benzylidene)-3-benzoyl)-β-D-glucopyranoside (Precursor 2)

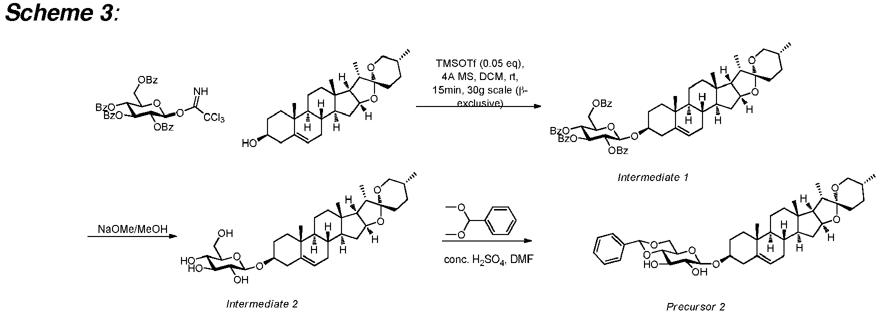

Preparation of 2,3,4,6-tetra-*O*-benzoyl-*β*-D-glucopyranose (Intermediate 1) and Diosgenyl-β-D-glucopyranoside (Intermediate 2) are are as described above.

### Precursor 2: Preparation of Diosgenyl-(4,6-0-(benzylidene)-3-benzoyl)-β-D-glucopyranoside (Precursor 2)

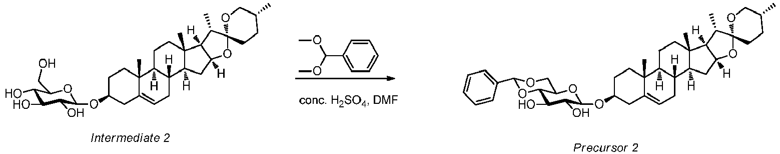

Diosgenyl-β-D-glucopyranoside (Intermediate 2) (18.97 g, 31.24 mmol) was slurried in DMF (142 mL). Benzaldehyde dimethyl acetal (9.48 mL, 62.5 mmol) was added and the reaction mixture heated to 60°C (bath temperature). Concentrated sulfuric acid (180 µL, 3.36 mmol) was added and the reaction mixture stirred at 60°C under vacuum for 6.5 hours. Benzaldehyde dimethyl acetal (0.95 mL, 6.3 mmol) was added and the reaction mixture stirred at 60°C under vacuum for 4 hours. Benzaldehyde dimethyl acetal (0.95 mL, 6.3 mmol) was added and the reaction mixture stirred at 60°C under vacuum for 5 hours. The reaction was quenched with *N*-methylmorpholine (1.8 mL, 16 mmol). The reaction mixture was cooled to ambient temperature and diluted with DCM (350 mL) and the organic layer washed with a 1:1 mixture of saturated sodium bicarbonate and water (300 mL) followed by a 2:1 mixture of saturated sodium bicarbonate and saturated brine (300 mL). The organic layer was dried (MgSO₄), filtered and concentrated *in vacuo.* The resulting crude product was slurried in acetonitrile (100 mL) for 80 minutes, filtered and washed with acetonitrile (2 x 40 mL) and dried under vacuum at 30°C for 16 h to afford Diosgenyl-(4,6-O-(benzylidene))-β-D-glucopyranoside (15.6 g, 75% yield) as a white solid;

¹H NMR (500 MHz, 3:1 CDCl₃/CD₃OD): δ7.50 (m, 2 H), 7.7 (m, 3 H), 5.54 (s, 1 H), 5.38 (dd, *J* = 2.3, 3.0 Hz, 1 H), 4.51 (d, *J* = 7.8 Hz, 1 H), 4.42 (dt, *J* = 6.7, 7.5 Hz, 1 H), 4.31 (dd, *J* = 4.8, 10.5 Hz, 1 H), 3.78 (t, *J* = 10.5 Hz, 1 H), 3.71 (t, *J* = 9.1 Hz, 1 H), 3.59 (m, 1 H), 3.52 (t, *J* = 9.1 Hz, 1 H), 3.46 (m, 2 H), 3.37 (m, 3 H), 2.43 (ddd, *J* = 2.3, 4.8, 13.3 Hz, 1 H), 2.29 (m, 1 H), 2.06-1.06 (m, 22 H), 1.04 (s, 3 H), 0.98 (d, *J* = 7.0 Hz, 3 H), 0.80 (s, 3 H), 0.79 (d, *J* = 7.9 Hz, 3 H);¹³C NMR (126 MHz, 3:1 CDCl₃/CD₃OD): δ137.70, 134.48, 126.45, 125.51, 123.59, 119.10, 106.90, 99.16, 78.31, 78.09, 76.66, 71.82, 70.55, 66.07, 64.19, 63.65, 59.34, 53.81, 47.44, 38.95, 37.59, 37.04, 35.95, 34.53, 34.16, 29.36, 28.99, 28.74, 28.60, 27.50, 26.90, 25.96, 18.15, 16.54, 14.23, 13.47, 11.58; HRMS (TOF ES+) *m*/*z* calcd for C₄₀H₅₆O₈Na 687.3873, found 687.3877.

### Example 4:

### Preparation of a compound of Formula X; Diosgenyl-(2,3,4-tribenzoyl)-α-L-rhamnopyranosyl-(1→2)-3-benzoyl)-β-D-glucopyranoside (Compound X-1)

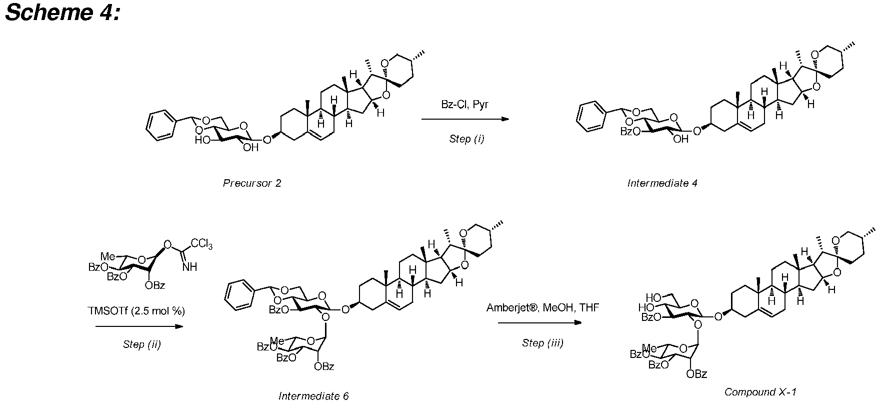

### Intermediate 4: Step (i)

### Selective benzoylation of Precursor 2 to provide Diosgenyl-(4,6-0-(benzylidene)-3-benzoyl)-β-D-glucopyranoside (Intermediate 4)

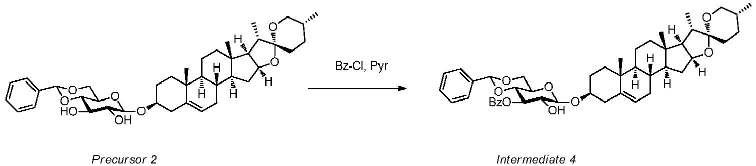

Diosgenyl-(4,6-*O*-(benzylidene))-β-D-glucopyranoside (Precursor 2) (16.0 g, 24.1 mmol) was dissolved in pyridine (60 mL) and DCM (60 mL) and the solvent removed under reduced pressure to azeotropically dry starting material. The resulting solid was dissolved in pyridine (39 mL) and DCM (62 mL) at ambient temperature under argon. Benzoyl chloride (3.35 mL, 28.8 mmol) was added dropwise maintaining a batch temperature below 22°C. The reaction mixture was stirred at ambient temperature for 35 minutes then quenched with methanol (5.0 mL, 120 mmol). The solvent was removed under reduced pressure and the resulting crude product dissolved in DCM (200 mL) and washed with 5% citric acid solution (3 x 70 mL), water (70 mL) and saturated sodium bicarbonate (70 mL). The organic layer was dried (MgSO₄), filtered and concentrated *in vacuo.* The resulting crude product was purified by flash chromatography (2 to 8% ethyl acetate in toluene) to afford diosgenyl-(4,6-O-(4-methoxybenzylidene)-3-benzoyl)-β-D-glucopyranoside (Intermediate 4) (10.12 g, 55% yield) as a white solid.

¹H NMR (500 MHz, 3:1 CDCl₃/CD₃OD): δ8.07 (dd, *J* = 1.3, 8.5 Hz, 2 H), 7.57 (tt, *J* = 1.3, 7.5 Hz, 1 H), 7.45 (t, *J* = 7.5 Hz, 2 H), 7.40 (m, 2 H), 7.29 (m, 3 H), 5.52 (s, 1 H), 5.46 (t, *J* = 9.4 Hz, 1 H), 5.39 (dd, *J* = 1.9, 3.4 Hz, 1 H), 4.66 (d, *J* = 7.7 Hz, 1 H), 4.42 (q, *J* = 7.7 Hz, 1 H), 4.37 (dd, *J* = 4.9, 10.5 Hz, 1 H), 3.83 (t, *J* = 10.3 Hz, 1 H), 3.78 (t, *J* = 9.4 Hz, 1 H), 3.63 (m, 3 H), 3.48 (dd, *J* = 2.0, 4.3, 10.7 Hz, 1 H), 3.37 (t, *J* = 11.0 Hz, 1 H), 2.46 (ddd, *J* = 2.0, 4.5, 13.2 Hz, 1 H), 2.30 (m, 1 H), 2.06-0.95 (m, 22 H), 1.04 (s, 3 H), 0.98 (d, *J* = 6.9 Hz, 3 H), 0.80 (s, 3 H), 0.79 (d, *J* = 7.9 Hz, 3 H); ¹³C NMR (126 MHz, 3:1 CDCl₃/CD₃OD): δ167.91, 141.65, 138.29, 138.29, 134.52, 131.25, 131.11, 130.32, 129.69, 129.47, 127.45, 123.25, 110.95, 103.51, 102.80, 82.35, 80.95, 80.21, 75.82, 74.33, 70.10, 68.23, 67.70, 63.39, 57.85, 51.48, 43.00, 41.64, 41.09, 39.97, 38.57, 38.20, 33.41, 33.03, 32.79, 32.65, 31.55, 30.94, 30.01, 22.20, 20.59, 18.28, 17.52; HRMS (TOF ES+) *m*/*z* calcd for C₄₇H₆₀O₉Na 791.4135, found 791.4127.

### Intermediate 6: Step (ii)

### Coupling to rhamnose moiety to provide Diosgenyl-(2,3,4-tribenzoyl)-α-L-rhamnopyranosyl-(1→2)-(4,6-O-(benzylidene)-3-benzoyl)-β-D-glucopyranoside (Intermediate 6)

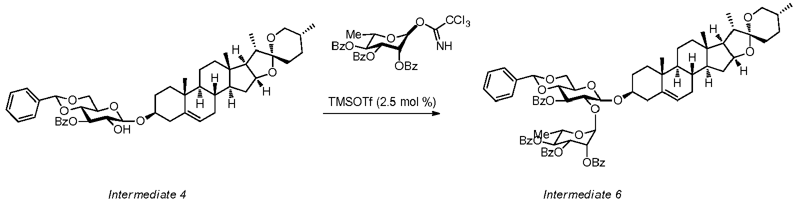

Diosgenyl-(4,6-O-(4-methoxybenzylidene)-3-benzoyl)-β-D-glucopyranoside (Intermediate 4) (10.0 g, 13.0 mmol), 2,3,4-tri-O-benzoyl-α/β-L-rhamnopyranoside trichloroacetimidate (11.0 g, 17.7 mmol) and 4Å molecular sieves were slurried in DCM (250 mL) and cooled to -40°C under argon. Trifluoromethane sulfonate (72 µL, 0.4 mmol) was added dropwise and the reaction mixture stirred at -40°C (batch temperature) for a further 90 minutes. Triethylamine (1.54 mL, 11.0 mmol) was added and the reaction mixture allowed to warm to ambient temperature. The molecular sieves were removed by filtration and the solvent evaporated under reduced pressure. The crude product, a pale yellow oil, was triturated with acetonitrile (200 mL) and slurried for one hour at ambient temperature. The solid product was isolated by filtration, washed with acetonitrile (2 x 30 mL) dried under vacuum at 30°C for 16 h to afford diosgenyl-(2,3,4-tribenzoyl)-α-L-rhamnopyranosyl-(1→2)-(4,6-O-(benzylidene)-3-benzoyl)-β-D-glucopyranoside (Intermediate 6) (13.7 g, 86% yield) as a white solid;

¹H NMR (500 MHz, 3:1 CDCl₃/CD₃OD): δ8.02 (dd, *J* = 1.3, 8.5 Hz, 2 H), 7.90 (dd, *J* = 1.2, 8.5 Hz, 2 H), 7.77 (dd, *J* = 1.3, 8.5 Hz, 2 H), 7.74 (dd, *J* = 1.2, 8.5 Hz, 2 H), 7.57 (tt, *J* = 1.3, 7.5 Hz, 1 H), 7.53 (tt, *J* = 1.3, 7.5 Hz, 1 H), 7.39-7.4- (m, 4 H), 7.22-7.37 (m, 11 H), 5.78 (t, *J* = 9.2 Hz, 1 H), 5.75 (dd, *J* = 3.5, 10.1 Hz, 1 H), 5.57 (t, *J* = 10.1 Hz, 1 H), 5.52 (s, 1 H), 5.51 (m, 1 H), 5.48 (dd, *J* = 1.7, 3.5 Hz, 1 H), 5.22 (d, *J* = 1.5 Hz, 1 H), 4.92 (d, *J* = 7.6 Hz, 1 H), 4.76 (m, 1 H), 4.42 (m, 2 H), 4.04 (dd, *J* = 7.7, 9.3 Hz, 1 H), 3.66-3.88 (m, 4 H), 3.49 (m, 1 H), 3.38 (t, *J* = 11.2 Hz, 1 H), 2.65 (ddd, *J* = 2.2, 4.5, 13.1 Hz, 1 H), 2.46 (m, 1 H), 2.04 (m, 3 H), 2.09-1.00 (m, 20 H), 1.36 (d, *J* = 6.6 Hz, 3 H), 0.99 (d, *J* = 7.2 Hz, 3 H), 0.95 (s, 3 H), 0.81 (d, *J* = 6.5 Hz, 3 H), 0.79 (s, 3 H); ¹³C NMR (126 MHz, 3:1 CDCl₃/CD₃OD): δ167.36; 167.04, 166.85, 166.29, 141.33, 138.16, 134.77, 134.70, 134.50, 134.47, 131.17, 131.09, 131.02, 130.91, 130.64, 130.50, 130.42, 130.33, 129.76, 129.70, 129.58, 129.47, 127.45, 123.67, 110.95, 102.83, 101.68, 99.27, 82.34, 80.88, 80.14, 77.56, 76.08, 73.29, 71.78, 71.02, 70.02, 68.27, 68.24, 67.66, 63.40, 57.79, 51.42, 43.01, 41.65, 41.03, 40.04, 38.50, 38.20, 33.48, 33.09, 32.87, 32.67, 31.55, 31.22, 30.02, 22.15, 20.56, 18.62, 18.28, 17.51, 15.63; HRMS (TOF ES+) *m*/*z* calcd for C₇₄H₈₂O₁₆Na 1249.5501, found 1249.5505.

### Compound X-1: Step (iii)

### Deprotection of Intermediate 6 to provide a compound of Formula X; Diosgenyl-(2,3,4-tribenzoyl)-α-L-rhamnopyranosyl-(1→2)-3-benzoyl)-β-D-glucopyranoside (Compound X-1)

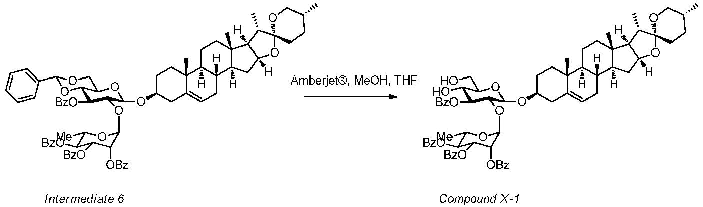

Diosgenyl-(2,3,4-tribenzoyl)-α-L-rhamnopyranosyl-(1→2)-(4,6-O-(benzylidene)-3-benzoyl)-β-D-glucopyranoside (Intermediate 6) (1.76 g, 1.43 mmol) and Amberjet® 1200 H (8.8 g) were slurried in methanol (24 mL) and tetrahydrofuran (12 mL) in a 100 mL round bottomed flask. The reaction was heated to reflux for 15 h with monitoring of the reaction by ¹H NMR. Upon completion the reaction was quenched with triethylamine (0.2 mL). The resin was removed by filtration and the solvent evaporated under reduced pressure. The crude product was dissolved in methanol (25 mL) and water added drop wise (15 mL) resulting in the crystallisation of a white solid. The solid product was isolated by filtration and the cake washed with 1:1 methanol/water (2 x 15 mL) followed by petroleum ether 60-80 (2 x 15 mL). The product was dried overnight under vacuum at 45°C, to give Diosgenyl-(2,3,4-tribenzoyl)-α-L-rhamnopyranosyl-(1→2)-3-benzoyl)-β-D-glucopyranoside (Compound X-1), 1.33 g, 81%.

¹H NMR 500 MHz (CDCl₃): δ 8.03 (d, 2H), 7.90 (d, 2H), 7.77 (d, 2H), 7.74 (d, 2H), 7.56 (t, J = 7.5 Hz, 1H), 7.53 (t, J = 7.5 Hz, 1H), 7.41 (m, 3H), 7.33 (m, 3H), 7.28 (t, J = 7.5Hz, 2H), 7.23 (t, J=7.5 Hz, 2H), 5.74 (dd, J = 3.6, 10.0 Hz, 1H), 5.48-5.57 (m, 3H), 5.44 (dd, J = 1.6, 3.6 Hz, 1H), 5.16 (d, J = 1.3 Hz, 1H), 4.82 (d, J = 7.9 Hz, 1H), 4.77 (m, 1H), 4.45 (q, 1H), 3.73-3.95 (m, 6H), 3.47-3.54 (m, 2H), 3.38-3.41 (m, 2H), 2.64 (app ddd, 1H), 2.45 (t, 1H), 2.03 (m, 3H), 1.09-1.93 (m, 20H), 1.34 (d, J =6.4 Hz, 3H), 0.99 (d, J =6.4 Hz, 3H), 0.95 (s, 3H), 0.81 (d, J= 6.4 Hz, 3H), 0.80 (s, 3H); ¹³C NMR (126 MHz, CDCl_{3/},MeOD 3:1): δ 166.3, 165.8, 165.3, 164.7, 139.9, 133.24, 133.17, 133.0. 132.9, 129.61, 129.55, 129.5, 129.3, 129.0, 128.9, 128.23, 128.17, 128.1 122.0, 109.4, 99.4, 97.6, 80.8, 79.0, 78.4, 75.9, 75.5, 71.8, 70.2, 69.6, 68.7, 66.7, 66.6, 61.9, 61.4, 56.3, 49.9, 41.4, 40.1, 39.5, 38.6, 37.0, 36.7, 32.0, 31.6, 31.3, 31.1, 30.0, 29.7, 28.5, 20.6, 19.0, 17.1, 16.8, 16.0. ES-MS *m*/*z* C₆₇H₇₈O₁₆Na calcd: 1161.5188, found 1161.5186

### Example 5

### Selective benzoylation of Precursor 2 to provide Diosgenyl-(4,6-O-(benzylidene)-3-benzoyl)-β-D-glucopyranoside (Intermediate 4) with crystallisation

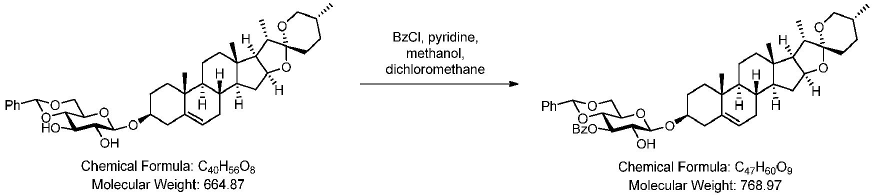

In a 50 L Buchi, precursor 2 (2.642 kg) was dissolved in dichloromethane (DCM) (16.5 kg) and pyridine (12.59 kg) by heating to 40-45°C. After dissolution, the solvent was evaporated (azeotropic drying). The precursor 2 was then dissolved in dry DCM (8.21 kg, ≤ 0.01% water) and dry pyridine (3.90 kg, ≤ 0.01% water) by stirring overnight at ambient temperature under nitrogen. The solution was then transferred into a 60 L reactor and stirred under nitrogen. The bowl was rinsed into the reactor with dry dichloromethane (5.26 kg) and dry pyridine (2.53 kg).

The jacket temperature of the reactor was then set to -5°C and Benzoyl chloride (BzCI) (694 g, 1.2 equivs) was added slowly to the solution at such a rate that the temperature of the reaction mixture was kept below 12°C. After the addition was complete the reaction mixture was stirred at 20°C for 35 min. The reaction mixture was sampled for completion and no additional BzCI was required (Additional benzoyl chloride can be added if required).

The reaction mixture was quenched by adding methanol (592 g) (over ∼ 5 min) and the mixture stirred for an additional 30 min at 20°C. The solution was then concentrated to dryness. The resulting crude material was dissolved in DCM (33.7 kg) and the resulting organic layer washed with cold 9% citric acid (5 x 16.5 kg) and then water (10.9 L). Additional DCM (10.6 kg) was added and the organic layer washed with 5% sodium bicarbonate (10.4 L). The bicarbonate wash was back extracted with DCM (10.64 kg). The organic phases were combined and the solvent removed on the rotary evaporator.

The crude intermediate 4 was slurried in DCM (20.8 kg). Toluene (13.0 kg) was added and the solvent removed by evaporation. The residue is slurried a second time with DCM (26.3 kg) while warming to 45°C for 30 min. Toluene (14.1 kg) was then added and the solution evaporated to give a mixture that had a net weight of 14.5 kg. Additional toluene was then added (39.4 kg) and the suspension stirred overnight at 20°C. Stirring was stopped and the suspension allowed to settle for 2 days at 20°C. The product was recovered by filtration and dried under vacuum (2.032 kg, 67% yield, 96% pure by NMR & HPLC).

### Example (6) (Comparative Example):

### Scope study of selective acylation (Step i) with Diosgenyl-(4,6-O-(4-methoxybenzylidene)-3-benzoyl)-β-D-glucopyranoside (Intermediate 3)

The scope of selective acylation at C3 was examined with Diosgenyl-(4,6-O-(4-methoxybenzylidene)-3-benzoyl)-β-D-glucopyranoside (Intermediate 3). Specifically, the influence of each of the acylating agent, solvent, base, temperature and time on the selectivity was examined. Results are summarised below in Table 1.

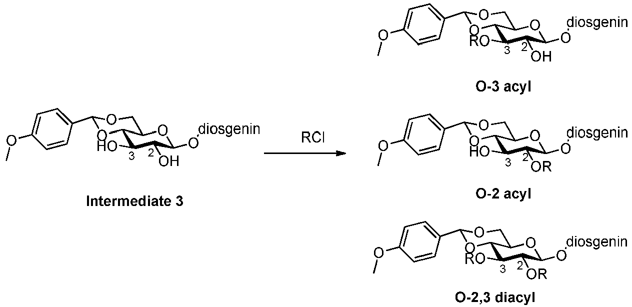

Diosgenyl-(4,6-O-(4-methoxybenzylidene)-3-benzoyl)-β-D-glucopyranoside (Intermediate 3) (50 mg) was dissolved in solvent (anhydrous, 2 mL) and base (10 eq) was added. The solution was brought to the temperature, unless otherwise stated acyl chloride (3 eq) was added and the reaction stirred for the required time. Water (0.5 mL) was added and volatiles were removed under reduced pressure. The residue was partitioned between ethyl acetate and saturated aqueous bicarbonate and then the organic phase was washed with water, dried (MgSO₄) and concentrated under reduced pressure.

**Table 1:**

| **Experiment No. and conditions** | | | | **Ratio of Products** | | |
|---|---|---|---|---|---|---|
| **Expt** | **Acyl group (R)** | **Base/solvent** | **Temperature/time** | **O-2 acyl** | **O-3 acyl** | **O-2,3 diacyl** |
| 1 | 1.0 eq benzoyl | pyridine, DCM | -78-18 °C then 18 °C, 1 h | 0.0 | 4.7 | 1.0 |
| 2 | 1.2 eq benzoyl | pyridine, DCM | up to 25 °C | 0.0 | 4.0 | 1.0 |
| 3 | 1.2 eq benzoyl | pyridine, DCM | -78-18 °C then 18 °C, 1 h | 0.0 | 9.0 | 1.0 |
| 4 | benzoyl | pyridine, DCM | -78-18 °C then 18 °C, 1 h | 1.0 | 27.8 | 1.0 |
| 5 | 4-chlorobenzoyl | pyridine, DCM | -78-18 °C then 18 °C, 2 h | 5.0 | 192.0 | 1.0 |
| 6 | 4-nitrobenzoyl | pyridine, DCM | -78-18 °C then 18 °C, 2 h | 1.0 | 72.5 | 7.5 |
| 7 | 4-methoxybenzoyl | pyridine, DCM | -78-18 °C then rt overnight | 21.0 | 276.0 | 1.0 |
| 8 | 2-chlorobenzoyl | pyridine, DCM | -78-18 °C then 18 °C, 2 h | 1.0 | 22.5 | 4.0 |
| 9 | acetoyl | pyridine, DCM | -78-18 °C then 18 °C, 2 h | 1.0 | 22.5 | 3.6 |
| 10 | acetoyl | pyridine, DCM | -78-18 °C then 18 °C, 1 h | 1.0 | 3.5 | 0.0 |
| 11 | propionoyl | pyridine, DCM | -78-18 °C then 18 °C, 2 h | 1.0 | 15.1 | 2.5 |
| 12 | benzoyl | triethylamine, DCM | -78-18 °C then 18 °C, 2 h | 1.0 | 6.5 | 0.0 |
| 13 | benzoyl | triethylamine, DCM | 18°C, 5 h | 24.7 | 178.3 | 1.0 |
| 14 | benzoyl | DIPEA, DCM | -78-18 °C then 18 °C, 2 h | 1.0 | 2.1 | 0.0 |
| 15 | benzoyl | DIPEA, DCM | rt, 24 h | 1.0 | 1.8 | 0.0 |
| 16 | benzoyl | K2CO3 | -78-18 °C then 18 °C, 2 h | 0.0 | 0.0 | 0.0 |
| 17 | benzoyl | pyridine, DCM | 18 °C, 2 h | 1.0 | 16.1 | 1.6 |
| 18 | benzoyl | pyridine, DCM | 40 °C, 80 min | 1.2 | 12.7 | 1.0 |
| 19 | benzoyl | pyridine, DCM | 0 °C, 3 h | 3.1 | 51.0 | 1.0 |
| 20 | benzoyl | pyridine, neat | 18 °C, 110 min | 0.0 | 2.0 | 1.0 |
| 21 | benzoyl | pyridine, THF | rt, 24 h | 10.3 | 33.4 | 1.0 |
| 22 | benzoyl | pyridine, dioxane | rt, 24 h | 3.5 | 11.1 | 1.0 |
| 23 | benzoyl | pyridine, DCM | 18 °C, 2 h | 1.0 | 17.5 | 1.6 |
| 24 | benzoyl | benZoic acid, DCC, DMAP, DCM | 18 °C, 2 h | 1.0 | 3.0 | 1.1 |

Advantageously, where the acylation was conducted with 1.1 to 1.2 equivalents of benzoyl chloride, in the presence of pyridine as a base and DCM as a solvent, none of the undesired C2-benzoylated product was observed (Expt No. 1 to 3).

Similarly, where the acylation was conducted with benzoyl chloride and in the presence of pyridine as both a solvent and base (Expt No. 20), none of the undesired C2-benzoylated product was observed.

The method step (i) of the present invention was able to be conducted over a range of temperatures, typically in the range of -78 and 40°C.

The method step (i) of the present invention also provides a range of acylating agents (Expt No. 4 to 11), typically optionally substituted benzoylating agents provide advantageous ratios of C2- to C3- protected products.

Advantageously, where any undesired O-2,3-bis-diacyl product is isolated, it may be recycled to recover Intermediate 3 by removal of the two acyl groups at C2- and C3-. The recovered Intermediate 3 may then be subjected to the selective acylation conditions to provide the desired O-3-acyl product.

### Example (7):

### Preparation of a compound of Formula Y; Diosgenyl-α-L-rhamnopyranosyl-(1→2)-β-D-glucopyranoside (Compound Y-1)

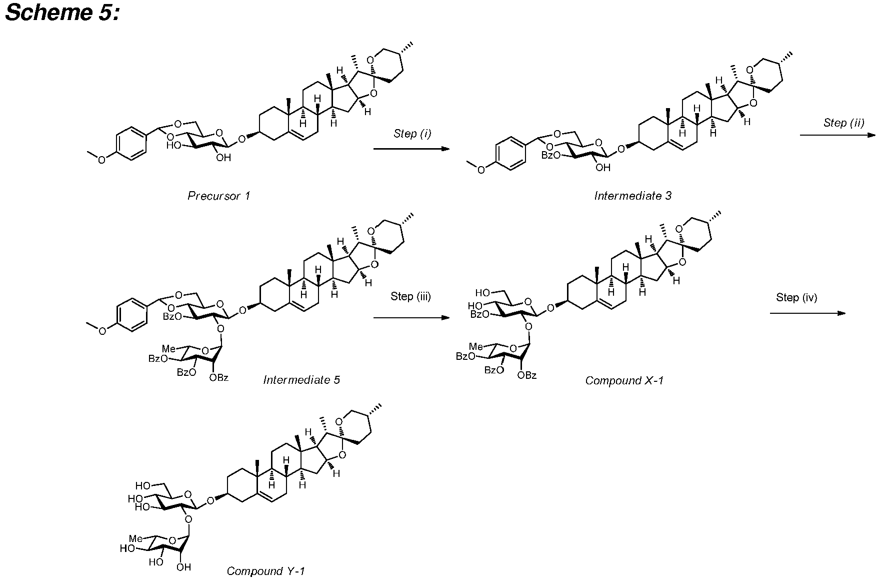

**Step (i):** Preparation of Diosgenyl-(4,6-O-(4-methoxybenzylidene)-3-benzoyl)-β-D-glucopyranoside (Intermediate 3) as described above.
**Step (ii):** Preparation of Diosgenyl-(2,3,4-tribenzoyl)-α-L-rhamnopyranosyl-(1→2)-(4,6-O-(4-methoxybenzylidene)-3-benzoyl)-β-D-glucopyranoside (Intermediate 5) as described above.
**Step (iii):** Preparation of Diosgenyl-(2,3,4-tribenzoyl)-α-L-rhamnopyranosyl-(1→2)-3-benzoyl)-β-D-glucopyranoside (Compound X-1) as described above.

### Compound Y-1: Step (iv)

### Deprotection of a compound of Formula X to provide a compound of Formula Y; Diosgenyl-α-L-rhamnopyranosyl-(1→2)-β-D-glucopyranoside (Compound Y-1)

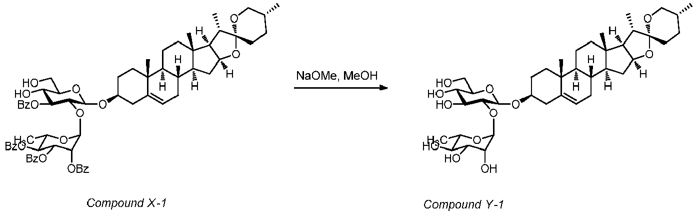

To a solution of Diosgenyl-(2,3,4-tribenzoyl)-α-L-rhamnopyranosyl-(1→2)-3-benzoyl)-β-D-glucopyranoside (Compound X-1) (16.542 g, 14.52 mmol) in MeOH (125 mL) was added 30 drops of NaOMe (5.4M in MeOH) and the pH was checked to be ∼10. Monitoring of the mixture by TLC indicated that the reaction was complete within 90 mins. The reaction was quenched with the addition of DOWEX 50W-X 400 until the pH ∼7 causing the DOWEX to change from a cream to light yellow colour. The resin was washed with MeOH and then 1:1 MeOH/CHCl₃.

The filtrate was evaporated to yield a dry white solid which was washed with EA (9.58g, 91%). The crude material was columned on silica eluting with 10% MeOH/DCM then 20% MeOH/DCM to give Compound Y-1 (8.67 g, 94% pure via HPLC).

Alternatively, Diosgenyl-(2,3,4-tribenzoyl)-α-L-rhamnopyranosyl-(1→2)-3-benzoyl)-β-D-glucopyranoside (Compound X-1) (9.3 g, 8.2 mmol) was dissolved in anhydrous methanol (74 mL). Sodium methoxide (0.1 mL, 30% solution in methanol) was added and the reaction mixture stirred for 22 hours at ambient temperature under argon. Tetrahydrofuran (74 mL) was added and the reaction mixture adjusted to pH 7 using Amberjet® 1200H resin. The resin was removed by filtration and washed with tetrahydrofuran (2 x 30 mL). The resulting solution was concentrated in vacuo and redissolved in methanol (74 mL). The product crystallized upon stirring at ambient temperature and the slurry was diluted with water (15 mL). The solid product was isolated by filtration, washed with 20% water in methanol (2 x 30 mL), water (30 mL) and ethyl acetate (3 x 30 mL). The solid product dried under vacuum at 35°C for 16 h to afford diosgenyl-α-L-rhamnopyranosyl-(1-2)-β-D-glucopyranoside (4.65 g, 79% yield) as a white solid.

¹H NMR (500 MHz, 3:1 CDCI3/CD3OD): δ5.35 (dd, J = 1.9, 3.2 Hz, 1 H), 5.19 (d, J = 1.5 Hz, 1 H), 4.46 (d, J = 7.6 Hz, 1 H), 4.41 (q, J = 7.6 Hz, 1 H), 4.08 (m, 1 H), 3.94 (dd, J = 1.5, 3.3 Hz, 1 H), 3.83 (dd, J = 3.0, 12.0 Hz, 1 H), 3.73 (dd, J = 4.7, 12.0 Hz, 1 H), 3.69 (dd, J = 3.5, 9.5 Hz, 1 H), 3.58 (m, 1 H), 3.49 (m, 2 H), 3.38 (m, 4 H), 3.25 (m, 1 H), 2.41 (ddd, J = 1.9, 4.7, 13.4 Hz, 1 H), 2.28 (m, 1 H), 2.00 (m, 2 H), 1.94-0.91 (m, 21 H), 1.27 (d, J = 6.2 Hz, 3 H), 1.02 (s, 3 H), 0.97 (d, J = 7.3 Hz, 3 H), 0.80 (d, J = 6.1 Hz, 3 H), 0.79 (s, 3 H); 13C NMR (126 MHz, 3:1 CDCI3/CD3OD): δ141.80, 123.05, 110.94, 101.93, 100.92, 82.35, 79.97, 79.09, 76.97, 74.19, 72.66, 71.93, 71.80, 69.69, 68.23, 63.38, 63.19, 57.86, 51.53, 42.99, 41.63, 41.09, 39.71, 38.60, 38.21, 33.40, 33.03, 32.78, 32.65, 31.54, 30.89, 30.01, 22.17, 20.47, 18.55, 18.28, 17.51, 15.62; HRMS (TOF ES+) m/z calcd for C₃₉H₆₂O₁₂Na 745.4139, found 745.4141.

### Example (8):

### Preparation of a compound of Formula Y

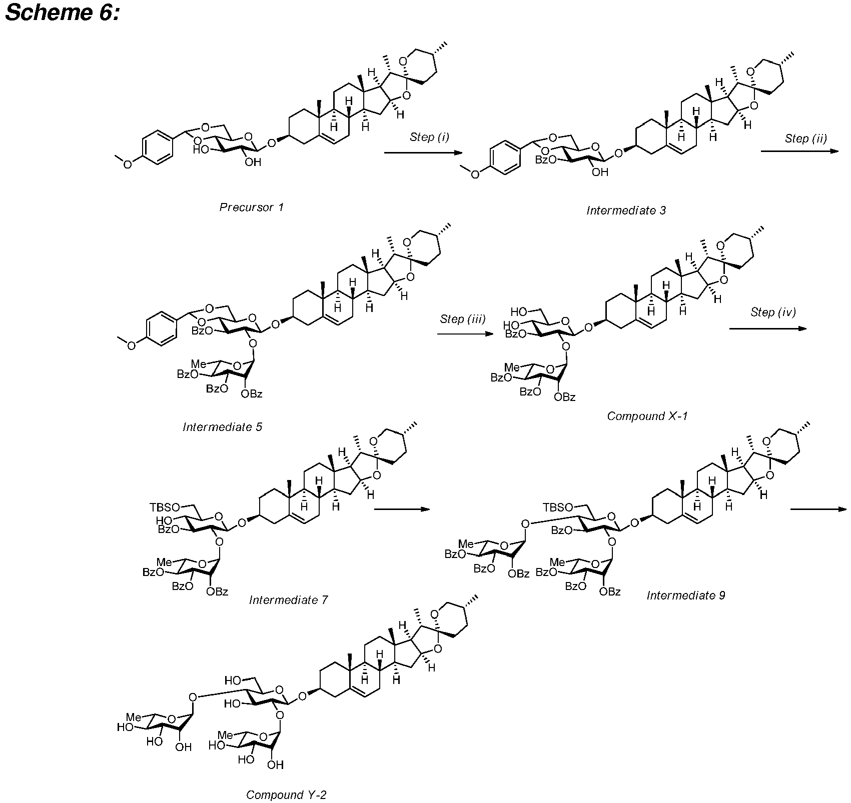

**Step (i):** Preparation of Diosgenyl-(4,6-O-(4-methoxybenzylidene)-3-benzoyl)-β-D-glucopyranoside (Intermediate 3) as previously described above.
**Step (ii):** Preparation of Diosgenyl-(2,3,4-tribenzoyl)-α-L-rhamnopyranosyl-(1→2)-(4,6-O-(4-methoxybenzylidene)-3-benzoyl)-β-D-glucopyranoside (Intermediate 5) as previously described above.
**Step (iii):** Preparation of Diosgenyl-(2,3,4-tribenzoyl)-α-L-rhamnopyranosyl-(1→2)-3-benzoyl)-β-D-glucopyranoside (Compound X-1) as previously described above.

### Intermediate 7: Step (iv)

### Selective protection at R⁷ to provide Intermediate 7

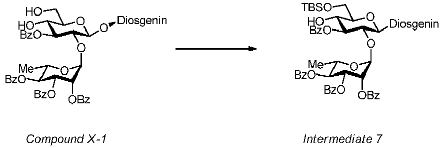

Diosgenyl-(2,3,4-tribenzoyl)-α-L-rhamnopyranosyl-(1→2)-3-benzoyl)-β-D-glucopyranoside (Compound X-1) (1.26 g, 1.1 mmol) was combined with imidazole (0.49 g, 7.2 mmol), DMAP (0.10 g, 0.83 mmol) and *tert*-butyldimethylsilyl chloride (0.29 g, 1.9 mmol) at room temperature in a dry flask under an Ar atmosphere. Dimethylformamide (5 mL) was added to effect dissolution and the reaction heated to 40°C under an Ar atmosphere for 1 h. The reaction was quenched by addition to of ethyl acetate (100 mL) and washed successively with saturated aq. NH₄Cl (2 x 100 mL), distilled water (2 x 100 mL), brine (100 mL), dried with MgSO₄ and evaporated. Columned chromatography upon silica with a gradient of ethyl acetate:toluene (5 to 10%) gave Intermediate 7 in near quantitative yield.

¹H NMR 500 MHz (CDCl₃) δ 8.04 (d, 2H), 7.91 (d, 2H), 7.78 (d, 2H), 7.74 (d, 2H), 7.53 (t, 1H), 7.49 (t, 1H), 7.39 (t, 2H), 7.35 (m, 1H), 7.31 (t, 2H), 7.28-7.15 (m, 7H), 5.75 (dd, J = 10.2, 3.5 Hz), 5.58 (t, J = 9.7 Hz), 5.47 (m, 2H), 5.44 (dd, J = 1.6, 3.4 Hz, 1H), 5.23 (d, J = 1.6 Hz, 1H), 4.75 (m, 2H), 4.43 (q, 1H), 3.92 (m, 3H), 3.76 (t, J = 9.2 Hz, 1H), 3.72 (m, 1H), 3.54 (dt, J = 5.4, 9.4 Hz, 1H), 3.48 (d of multiplets, J = 10.8 Hz, 1H), 3.39 (t, J = 10.8 Hz, 1H), 3.35 (s, 1H), 2.60 (d of multiplets, J = 13.1 Hz, 1H), 2.43 (t, 1H), 2.35 (s, 1H), 2.03 (m, 3H), 1.05-1.91 (m, 26H), 1.33 (d, J = 6.3 Hz, 3H), 0.98 (d, J = 6.8 Hz, 3H), 0.94 (s, 3H), 0.91 (s, 9H), 0.79 (d, J = 5.6 Hz, 3H), 0.79 (s, 3H), 0.11 (s, 3H), 0.10 (s, 3H); ¹³C NMR 126 MHz (CDCl₃) δ 167.1, 165.8, 165.4, 164.7, 140.3, 133.30, 133.25, 133.0, 130.1, 129.9, 129.9, 129.8, 129.5, 129.4, 129.1, 128.4, 128.4, 128.33, 128.26, 125.4, 122.2, 109.4, 99.9, 97.9, 80.9, 79.43, 79.41, 75.4, 75.2, 72.1, 72.0, 70.6, 69.7, 67.0, 66.9, 64.5, 62.3, 56.6, 50.2, 41.8, 40.4, 39.9, 38.9, 37.3, 37 0, 32.3, 32.0, 31.7, 31.6, 30.4, 30.3, 30.0, 29.8, 29.0, 26.0, 20.9, 19.4, 18.4, 17.5, 17.3, 16.4; ES-MS m/z C₇₃H₉₂O₁₆NaSi calcd: 1275.6052; found: 1275.6040

### Intermediate 9: Step (iv)

### Coupling of an additional saccharide at R⁶ to provide Intermediate 9

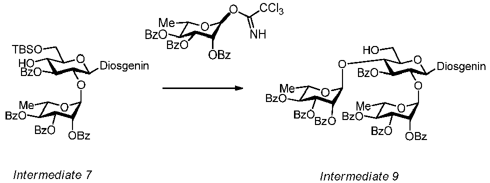

A solution of Intermediate 7 (116 mg, 0.09 mmol) and 4Å molecular sieve (0.49 g) in dry dichloromethane (5 mL) was stirred under Ar for 20 minutes at room temperature and then cooled to -78°C (internal) reaction temperature. Boron trifluoride diethyl etherate (60 uL, 0.47 mmol) was added via syringe followed but a solution of 2,3,4-tri-*O*-benzoyl-α/β-L-rhamnopyranoside trichloroacetimidate (177 mg, 0.29 mmol) in dry dichloromethane (2 mL) via a cannula at -78°C. The mixture was allowed to warm to room temperature and stirred for 3 h. The reaction was neutralised with triethylamine (0.2 mL), filtered to remove the sieves and concentrated under reduced pressure. TLC (30% ethyl acetate/petroleum ether) indicated significant desilylation of the reaction product. Complete desilylation was achieved with addition of tetrabutylammonium fluoride (1 mL, 1 mmol) with stirring at room temperature for 10 h. The reaction was partitioned into saturated aq. NH₄Cl (100 mL) and ethyl acetate (50 mL). The organic layer was washed with water (2 x 100 mL), brine (100 mL), dried with MgSO₄ and evaporated to dryness. Column chromatography was performed on silica eluting with a gradient of 0 to 15% ethyl acetate:toluene to give Intermediate 9; 76 mg, 69% yield.

¹H NMR 500 MHz (CDCl₃) δ 8.11 (d, 2H), 8.07 (m, 2H), 8.04 (d, 2H), 7.91 (d, 2H), 7.86 (d, 2H), 7.83 (d, 2H), 7.74 (d, 4H), 7.61 (t, 1H), 7.56 (t, 1H), 7.34-7.54 (m, 12H), 7.32 (t, 2H), 7.15-7.27 (m, 5H), 5.81 (t, J= 9.3 Hz, 1H), 5.76 (dd, J = 3.6, 10.2 Hz, 1H), 5.64 (dd, J = 3.4, 10.4 Hz, 1H), 5.58 (dd, J= 2.0, 3.7 Hz, 1H), 5.54 (t, J =10.4Hz, 1H), 5.51 (t, J =10.4 Hz, 1H), 5.48 (s, 1H), 5.42 (dd, J =1.5, 3.4 Hz, 1H), 5.23 (d, J = 1.5 Hz, 1H), 5.12 (d, J =1.3 Hz, 1H), 4.84 (d, J = 7.4 Hz, 1H), 4.74 (m, 1H), 4.44 (m, 1H), 4.26 (m, 1H), 4.20 (t, J = 9.8 Hz, 1H), 4.08 (m, 2H), 3.97 (m, 2H), 3.75 (m, 1H), 3.72 (m, 1H), 3.49 (d of multiplets, J = 10.7 Hz, 1H), 3.39 (t, J = 10.8 Hz, 1H), 2.63 (d of multiplets, J = 13.2 Hz, 1H), 2.44 (t, 1H), 2.17 (m, 1H), 2.04 (m, 4H), 1.05-1.97 (m, 21 H), 1.33 (d, J = 5.8 Hz, 3H), 0.99 (d, J = 7.1 Hz, 3H), 0.94 (s, 3H), 0.80 (d, J = 7.1 Hz, 3H), 0.79 (s, 3H), 0.73 (d, J = 6.2 Hz, 3H). ¹³C NMR 126 MHz (CDCl₃) δ TBC; ES-MS m/z C₉₄H₁₀₀O₂₃Na, calcd 1619.6548; found: ES-MS low res 1619.7.

¹³C NMR 500 MHz (CDCl₃) 165.9, 165.7, 165.6, 165.5, 165.4, 165.2, 164.5, 140.1, 133.5, 133.2, 133.2, 133.1, 133.0, 132.9, 130.0, 130.0, 129.8, 129.8, 129.7, 129.7, 129.4, 129.4, 129.3, 129.3, 129.0, 128.5, 128.3, 128.3, 128.2, 128.1, 122.3, 109.3, 99.8, 98.7, 98.0, 80.8, 79.4, 76.2, 76.1, 76.0, 75.4, 72.0, 71.3, 71.2, 70.4, 69.8, 69.6, 67.5, 66.9, 62.2, 61.3, 56.5, 50.1, 41.7, 40.3, 39.7, 38.7, 37.2, 36.9, 32.2, 31.9, 31.6, 31.5, 30.3, 29.9, 28.9, 20.8, 19.3, 17.4, 17.2, 17.1, 16.3, 14.5.

### Compound Y-2: Step (iv)

### Deprotection to provide a compound of Formula Y

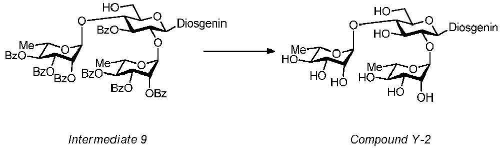

Intermediate 9 (82 mg, 0.051 mmol) was dissolved in 13 mL of methanol/tetrahydrofuran (8:5) at room temperature and 5.4 M sodium methoxide in methanol (0.011 mL, 0.059 mmol) was added to bring the reaction to ∼ pH 10. The reaction was stirred overnight at room temperature resulting in 50% conversion of starting material to desired product as monitored via LCMS. An additional charge of 5.4 M sodium methoxide in methanol (0.011 mL, 0.059 mmol) was added and the reaction was stirred overnight at room temperature resulting in the complete conversion to product as monitored via LCMS. The material was neutralised with Amberjet 1200H resin, filtered and evaporated to give a white powder. Column chromatography on silica eluting with 5 to 25% methanol in dichloromethane led to the isolation of 38 mg (85%) of Y2. NMR 500 MHz (3:1 CDCI3/CD3OD) δ 5.36 (dd, J = 1.9, 3.0 Hz, 1H), 5.23 (d, J = 1.4 Hz, 1H), 4.85 (d, J = 1.6 Hz, 1H), 4.46 (d, J = 7.8 Hz, 1H), 4.42 (q, J = 7.5 Hz, 1H), 4.09 (m, 1H), 3.96 (dd, J = 1.6, 3.4 Hz, 1H), 3.89-3.33 (m, 15H), 3.30 (dq, J = 2.2, 9.5 Hz, 1H), 2.41 (ddd, J = 1.9, 4.6, 13.5 Hz, 1H), 2.28 (t, J = 12.4 Hz 1H), 2.00 (m, 2 H), 1.94-0.91 (m, 21 H), 1.30 (d, *J = 6.2* Hz, 3 H), 1.27 (d, *J* = 6.2 Hz, 3 H), 1.02 (s, 3 H), 0.98 (d, *J* = 7.1 Hz, 3 H), 0.80 (d, *J* = 5.9 Hz, 3 H), 0.79 (s, 3 H), ¹³C NMR 500 MHz (CDCl₃) δ 141.8, 123.1, 111.0, 103.2, 102.0, 100.7, 82.4, 80.8, 80.0, 78.0, 76.1, 74.2, 73.8, 72.7, 72.3, 72.2, 72.0, 70.9, 69.7, 68.2, 63.4, 62.3, 57.9, 51.5, 43.0, 41.6, 41.1, 39.7, 38.6, 38.2, 33.4, 33.0, 32.8, 32.6, 31.5, 30.9, 30.0, 22.2, 20.5, 18.5, 18.5, 18.3, 17.5, 15.6; ES-MS m/z C₄₅H₇₂O₁₆Na, calcd 891.4718; found 891.4719.

### Example (9):

### Preparation of a compound of Formula Y

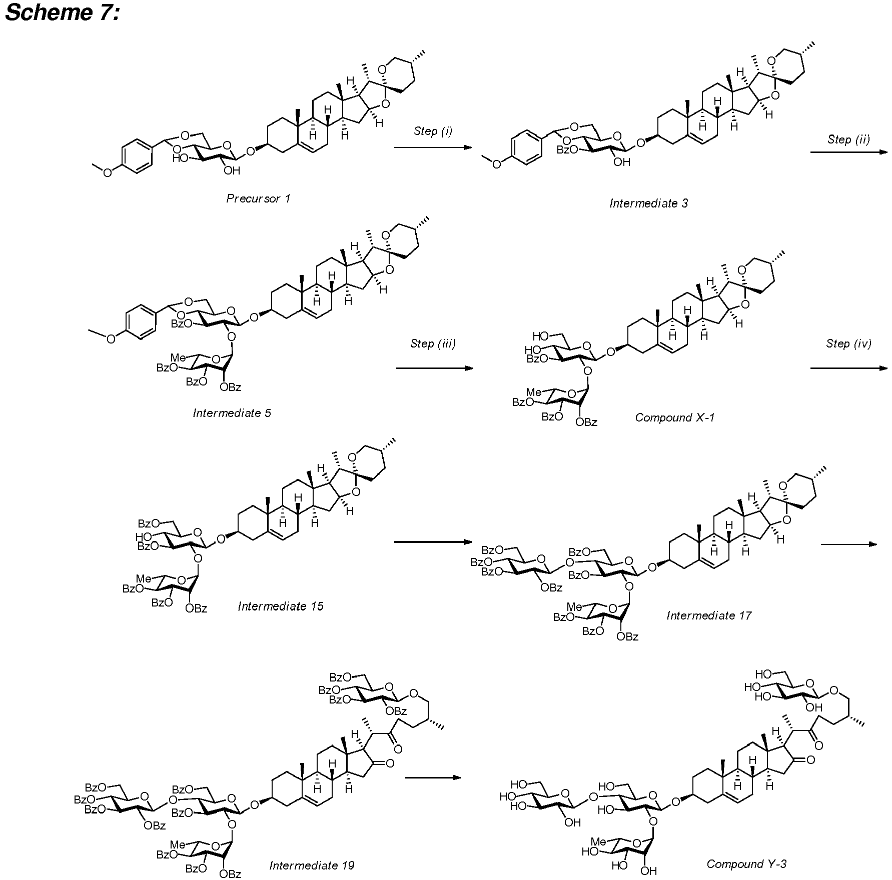

### Example (10):

### Preparation of a compound of Formula Y

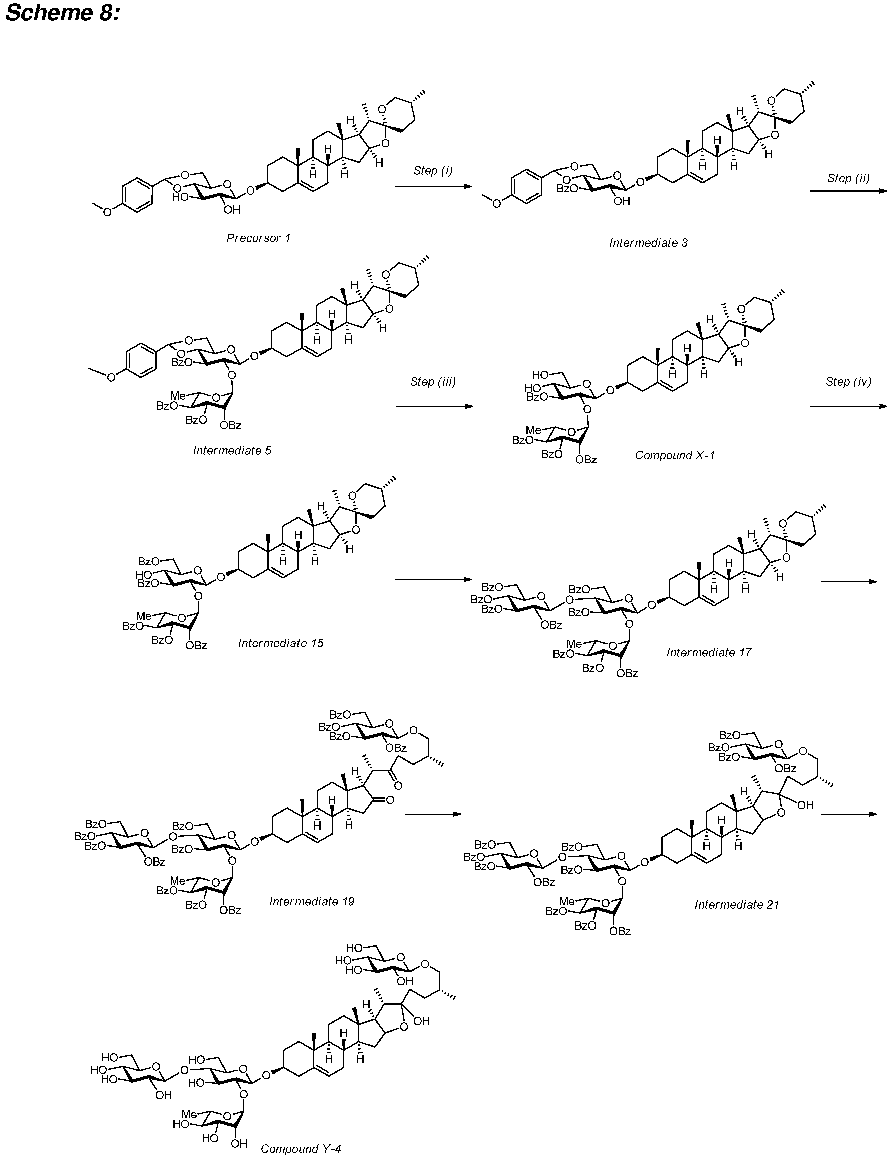

### Example (11):

### Preparation of a compound of Formula Y

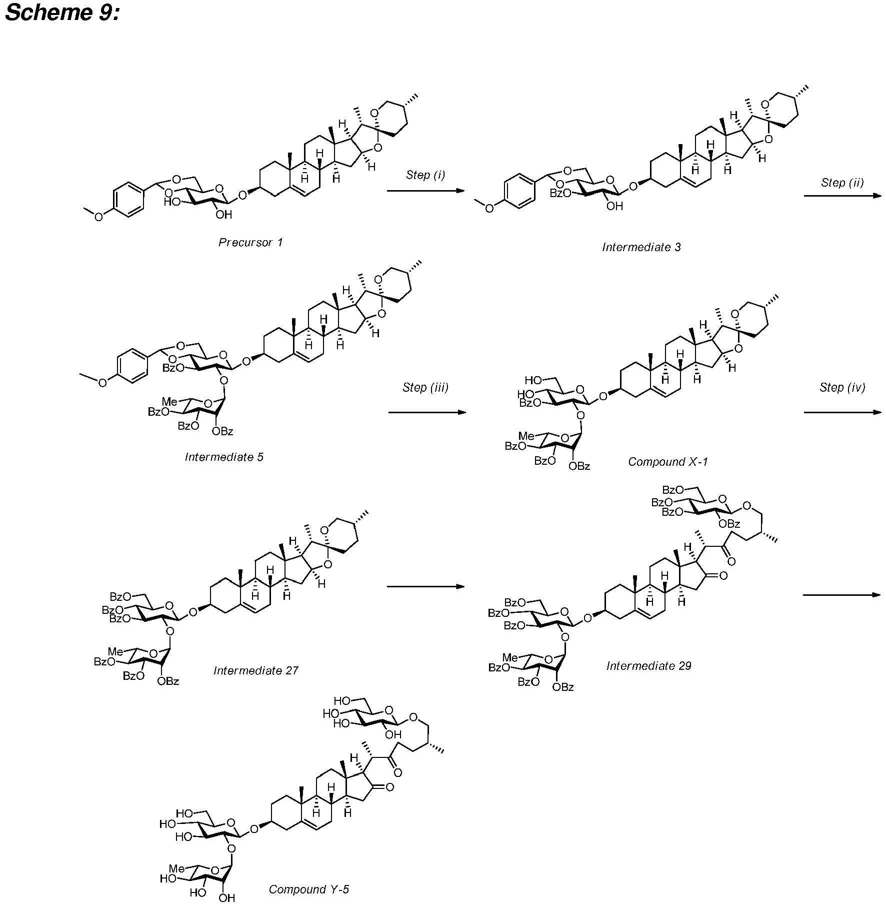

### Example (12):

### Preparation of a compound of Formula Y utilising Precursor 2

It will be appreciated that Compounds Y-1, Y-2, Y-3, Y-4, and Y-5 and other compounds of Formula Y can be prepared from Precursor 2 utilising the methods described for Example (3) to provide Compound X-1. Compound X-1 can then be elaborated by the methods described in Example 6 to Examples 11, to provide Compounds Y-1 to Y-5, respectively.

## Claims

1. A method for the preparation of a compound of Formula X 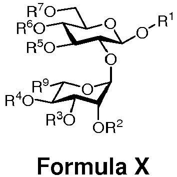 wherein
R¹ is a sapogenin of Formula E, F or G: 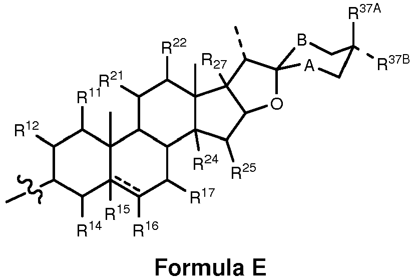 wherein
R¹¹, R¹², R¹⁴, R¹⁶, R¹⁷, R²¹, R²², R²⁴, R²⁵ and R²⁷ are independently H, OH, =O, pharmacologically acceptable ester groups or pharmacologically acceptable ether groups;
R¹⁵ is H when C-5,C-6 is a single bond, and nothing when C-5,C-6 is a double bond;
A is either O concurrently with B being CH₂, or B is O concurrently with A being CH₂;
R^{37A} is H concurrently with R^{37B} being CH₃, or R^{37A} is CH₃ concurrently with R^{37B} being H;
or a pharmaceutically acceptable salt thereof; 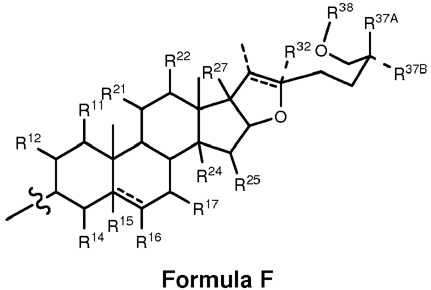 wherein
R¹¹, R¹², R¹⁴, R¹⁶, R¹⁷, R²¹, R²², R²⁴, R²⁵ and R²⁷ are independently H, OH, =O, pharmacologically acceptable ester groups or pharmacologically acceptable ether groups;
R¹⁵ is H when C-5, C-6 is a single bond, and nothing when C-5, C-6 is a double bond;
R³² is either a hydroxyl or an alkoxyl group when C-20, C-22 is a single bond, or nothing when C-20, C-22 is a double bond;
R^{37A} is H concurrently with R^{37B} being CH₃, or R^{37A} is CH₃ concurrently with R^{37B} being H;
R³⁸ is H or a saccharide; or a pharmaceutically acceptable salt;
or a pharmaceutically acceptable salt thereof; 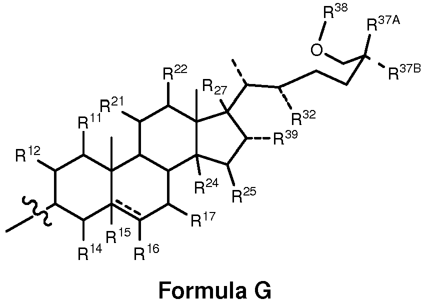 wherein
R¹¹, R¹², R¹⁴ , R¹⁶ , R¹⁷, R²¹, R²², R²⁴, R²⁵ and R²⁷ are each independently H, OH, =O, pharmacologically acceptable ester groups or pharmacologically acceptable ether groups;
R¹⁵ is H when C-5, C-6 is a single bond, and nothing when C-5, C-6 is a double bond;
R³² and R³⁹ are each independently H, OH, =O, pharmacologically acceptable ester groups or pharmacologically acceptable ether groups;
R^{37A} is H concurrently with R^{37B} being CH₃, or R^{37A} is CH₃ concurrently with R^{37B} being H;
R³⁸ is H or a saccharide; or a pharmaceutically acceptable salt thereof;
or a pharmaceutically acceptable salt thereof;
R², R³, and R⁴ are each independently an oxygen protecting group;
R⁵ is an acyl group selected from the group consisting of, benzoyl, 2-chlorobenzoyl, 4-chlorobenzoyl, 4-nitrobenzoyl and 4-methoxybenzoyl;
R⁶ and R⁷ are H;
R⁹ is selected from the group consisting of H, CH₃, and an oxygen protected by an oxygen protecting group;
the method comprising
(1) reacting a compound of Formula A with an acylating agent selected from the group consisting of benzoyl chloride, 2-chlorobenzoyl chloride, 4-chlorobenzoyl chloride, 4-nitrobenzoyl chloride and 4-methoxybenzoyl chloride in an acylation reaction in the presence of a base; 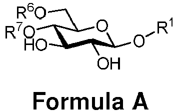 wherein
R¹ is a sapogenin as defined above;
R⁶ and R⁷ are each independently an oxygen protecting group; or when taken together form a cyclic di-oxygen protecting group;
to provide a compound of Formula B 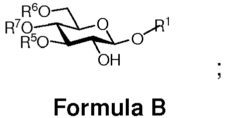
R¹ is sapogenin as defined above;
R⁵ is an acyl group selected from the group consisting of, benzoyl, 2-chlorobenzoyl, 4-chlorobenzoyl, 4-nitrobenzoyl and 4-methoxybenzoyl;
R⁶ and R⁷ are each independently an oxygen protecting group or when taken together form a cyclic di-oxygen protecting group;
(2) reacting a compound of Formula B with a compound of Formula C under coupling conditions 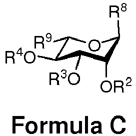 wherein
R², R³, and R⁴ are each independently an oxygen protecting group;
R⁸ is a leaving group; and
R⁹ is selected from the group consisting of H, CH₃, and an oxygen protected by an oxygen protecting group;
(3) selectively removing the oxygen protecting groups at R⁶ and R⁷ to provide a compound of Formula X.

2. A method according to claim 1 wherein the base in step (i) is selected from the group consisting of K₂CO₃, triethylamine, diisopropylethylamine, pyridine, 4-dimethylaminopyridine, and 1,8-diazabicycloundec-7-ene.

3. A method according to any one of the preceding claims wherein step (i) is carried out in the presence of a solvent selected from the group consisting of dichloromethane, tetrahydrafuran, 1,2-dioxane, dichloroethane, chloroform, carbon tetrachloride and pyridine.

4. A method according to any one of the preceding claims wherein step (i) is conducted at a temperature in the range of from -100 to 80°C, preferably wherein the temperature of step (i) is initially in the range of -10 to 20°C, and then subsequently increased over the course of the reaction to a temperature in the range of in the range of 10 to 25°C.

5. A method according to any one of the preceding claims wherein the ratio of acylating agent to a compound of Formula A is from 3:1 to 1:1.

6. A method according to any one of preceding claims wherein
R⁶ and R⁷ of Formula A are taken together form a cyclic group selected from the group consisting of 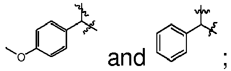 R², R³, R⁴ are each independently acetyl or benzoyl; and
R⁸ is selected from the group consisting of -SEt, -Br,

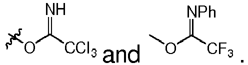

7. A method according to any one of the preceding claims wherein R¹ is selected from the group consisting of spirostanol aglycones and furostanol aglycones selected from the group consisting of diosgenin, yamogenin (neodiosgenin), yuccagenin, sarsasapogenin, tigogemn, smilagenin, hecogenin, gitogemn, convallamarogenin, neoruscogenin, solagenin, protodiosgenin, pseudoprotodiosgenin, methyl protodiosgenin, protoyamogenin, methyl protoyamogenin, and pharmaceutically acceptable salts, hydrates thererof.

8. A method according to any one of claims 1 to 7 further comprising the step of (4) converting a compound of Formula X into a compound of Formula Y, 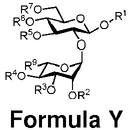 wherein
R¹ is a sapogenin as defined in claim 1;
R², R³, R⁴, R⁵ and R⁷are each H;
R⁶ is H or a saccharide;
R⁹ is selected from the group consisting of H, OH and CH₃;
pharmaceutically acceptable salts, hydrates or solvates thereof.

9. The method of claim 8 wherein step (iv) comprises removing any protecting groups to provide a compound of Formula Y.

10. The method of claim 8 wherein step (iv) comprises a process selected from the group consisting of:
(1)
(a) selectively introducing an oxygen protecting group at R⁷;
(b) coupling a suitable saccharide under coupling conditions such that R⁶ is a saccharide;
to provide a compound of Formula X' 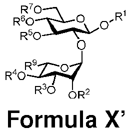 wherein
R¹ is a sapogenin as defined in claim 1;
R², R³, R⁴ and R⁷ are each independently an oxygen protecting group;
R⁵ is an acyl group selected from the group consisting of, benzoyl, 2-chlorobenzoyl, 4-chlorobenzoyl, 4-nitrobenzoyl and 4-methoxybenzoyl;
R⁶ is a saccharide;
R⁹ is selected from the group consisting of H, CH₃, and an oxygen protected by an oxygen protecting group; and
(c) converting a compound of Formula X' into a compound of Formula Y, a pharmaceutically acceptable salt, hydrate or solvate thereof;
(2)
(a) selectively introducing an oxygen protecting group at R⁷;
(b) coupling a suitable saccharide under coupling conditions such that R⁶ is a saccharide to provide a compound of Formula X' as defined above; and
(c) removing any protecting groups to provide a compound of Formula Y;
(3)
(a) selectively introducing an oxygen protecting group at R⁷;
(b) coupling a suitable saccharide under coupling conditions such that R⁶ is a saccharide to provide a compound of Formula X' as defined above;
(c) converting the sapogenin at R¹ into a sapogenin of Formula G as defined in claim 1; and
(d) removing any protecting groups to provide a compound of Formula Y; and
(4)
(a) selectively introducing an oxygen protecting group at R⁷;
(b) coupling a suitable saccharide under coupling conditions such that R⁶ is a saccharide to provide a compound of Formula X' as defined above;
(c) converting the sapogenin at R¹ into a sapogenin of Formula F as defined in claim 1; and
(d) removing any protecting groups to provide a compound of Formula Y.

11. The method of any one of claims 8 to 10 wherein the compound of Formula Y is selected from the group consisting of 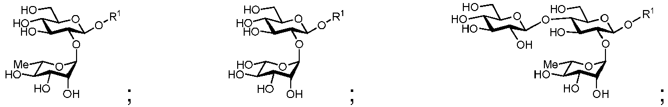 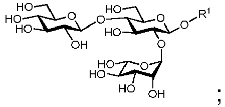 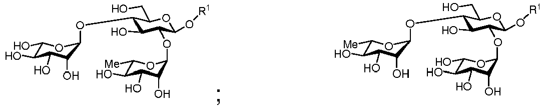 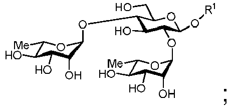 and 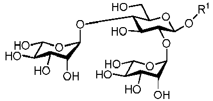 and/or wherein R¹ is selected from the group consisting of : 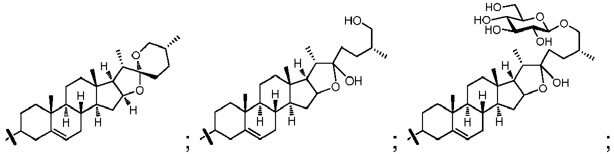 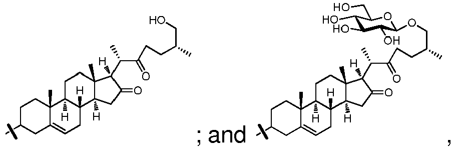 preferably wherein the compound of Formula Y is selected from the group consisting of: 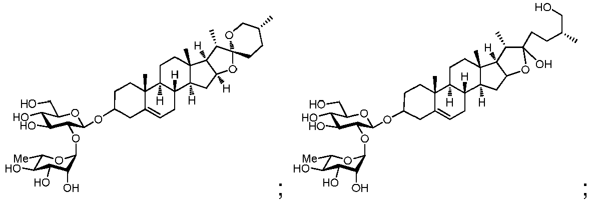 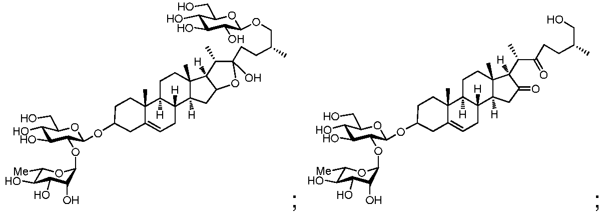 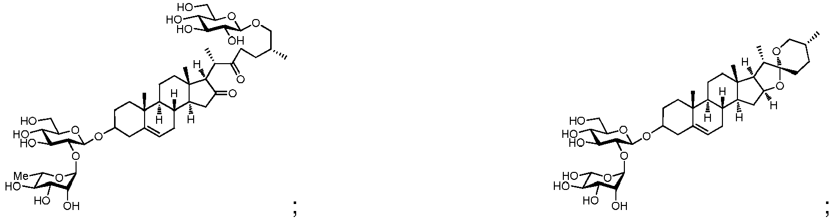 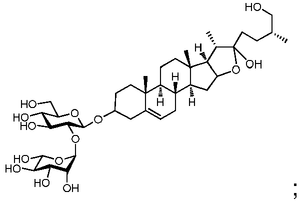 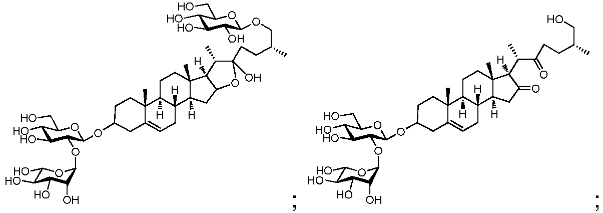 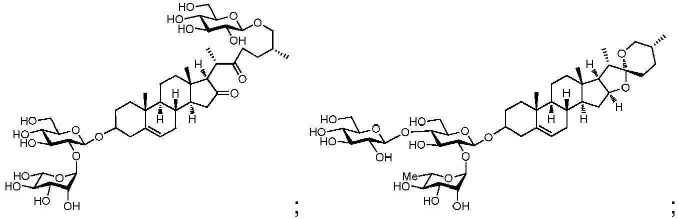 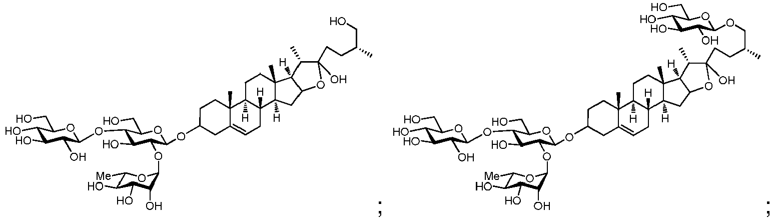 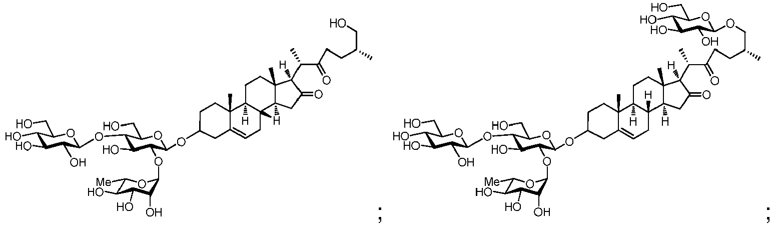 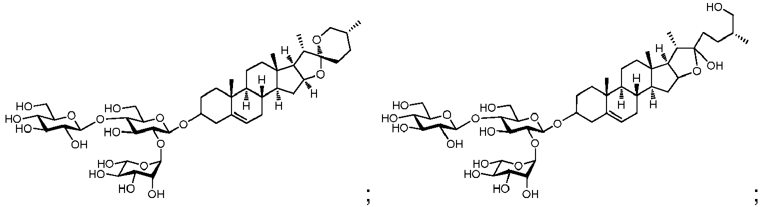 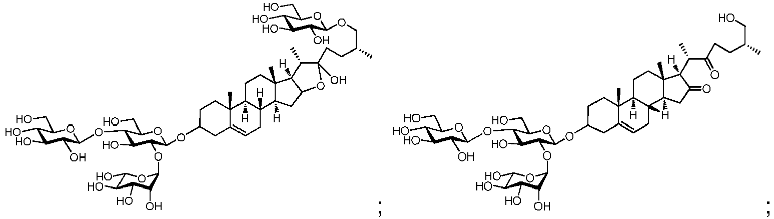 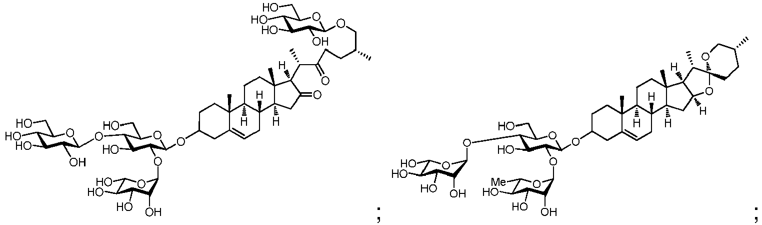 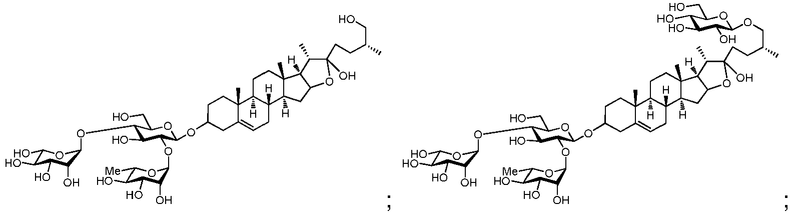 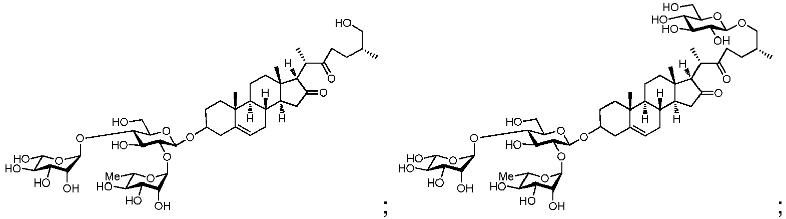 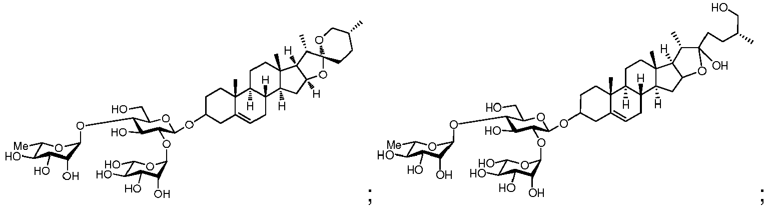 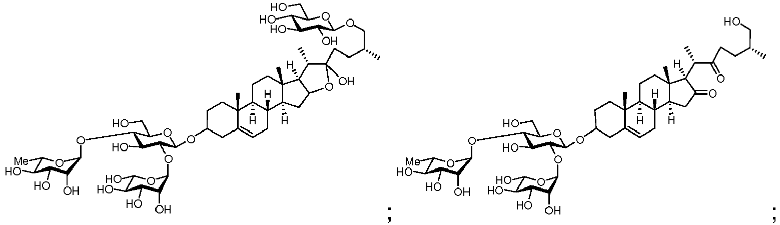 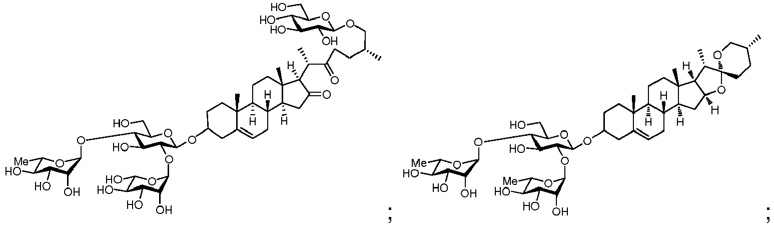 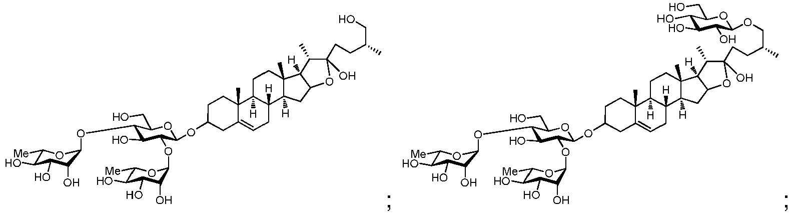 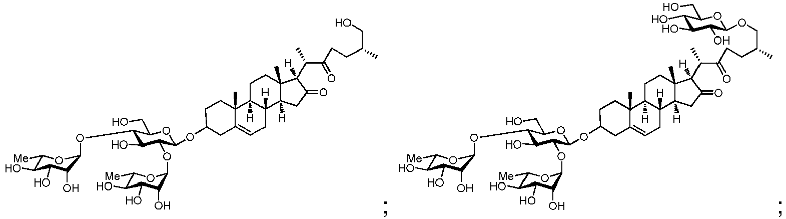 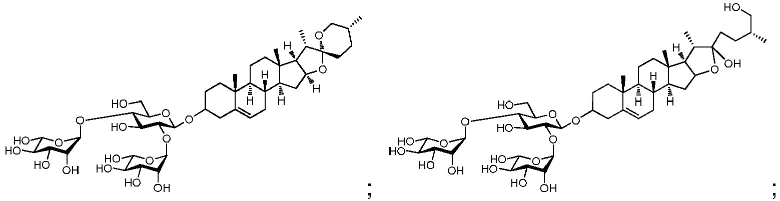 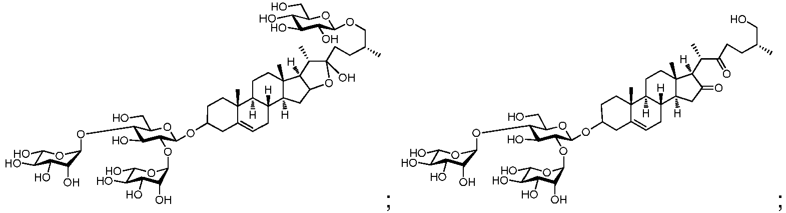 and 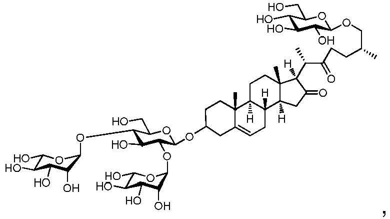 more preferably wherein the compound of Formula Y is selected from the group consisting of:

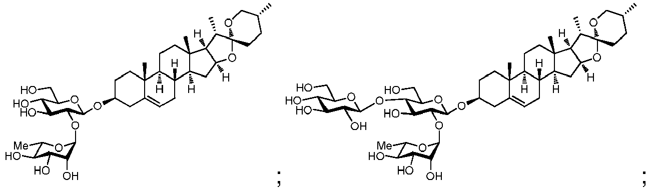

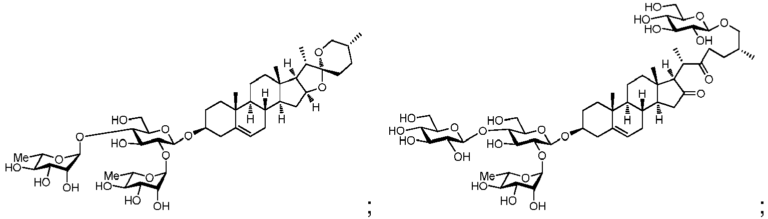

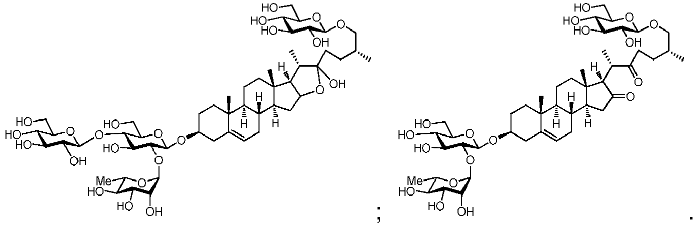

12. A method of any one of the preceding claims wherein the compound of formula X is

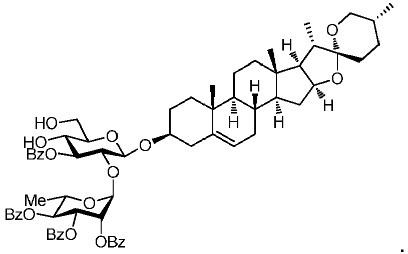

## Patentansprüche

1. Verfahren für die Herstellung einer Verbindung mit der Formel X 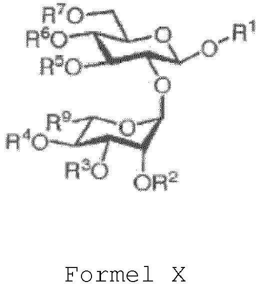 wobei
R¹ ein Sapogenin mit der Formel E, F oder G ist: 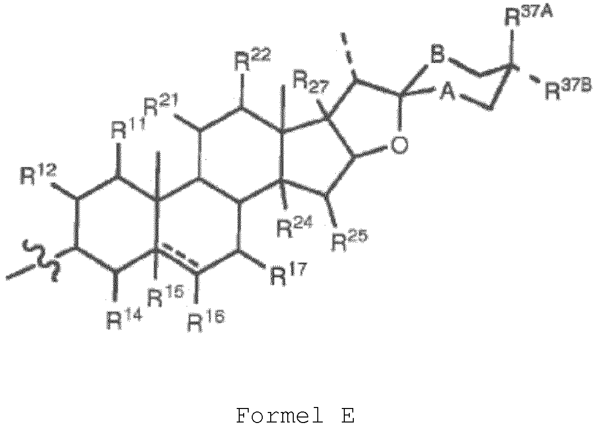 wobei
R¹¹, R¹², R¹⁴, R¹⁶, R¹⁷, R²¹, R²², R²⁴, R²⁵ und R²⁷ unabhängig H, OH, =O, pharmakologisch verträgliche Estergruppen oder pharmakologisch verträgliche Ethergruppen sind;
R¹⁵ H ist, wenn C-5, C-6 eine Einfachbindung ist, und nichts, wenn C-5, C-6 eine Doppelbindung ist;
A entweder O ist, wobei gleichzeitig B CH₂ ist, oder B O ist, wobei gleichzeitig A CH₂ ist;
R^{37A} H ist, wobei gleichzeitig R^{37B} CH₃ ist, oder R^{37A} CH₃ ist, wobei gleichzeitig R^{37B} H ist;
oder ein pharmazeutisch verträgliches Salz davon; 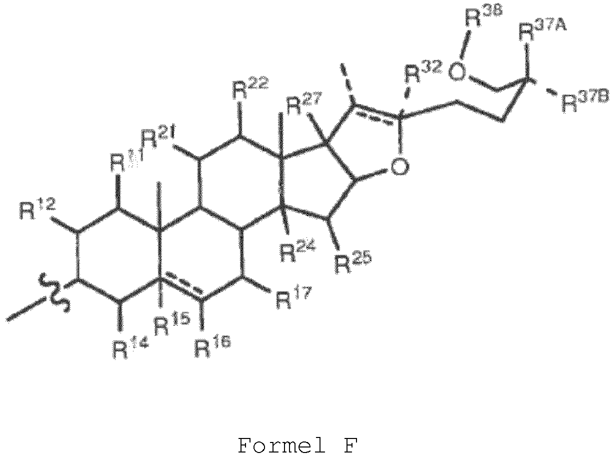 wobei
R¹¹, R¹², R¹⁴ , R¹⁶ , R¹⁷, R²¹, R²², R²⁴, R²⁵ und R²⁷ unabhängig H, OH, =O, pharmakologisch verträgliche Estergruppen oder pharmakologisch verträgliche Ethergruppen sind;
R¹⁵ H ist, wenn C-5, C-6 eine Einfachbindung ist, und nichts, wenn C-5, C-6 eine Doppelbindung ist;
R³² entweder eine Hydroxyl- oder eine Alkoxylgruppe ist, wenn C-20, C-22 eine Einfachbindung ist, oder nichts, wenn C-20, C-22 eine Doppelbindung ist;
R^{37A} H ist, wobei gleichzeitig R^{37B} CH₃ ist oder R^{37A} CH₃ ist, wobei gleichzeitig R^{37B} H ist;
R³⁸ H oder ein Saccharid; oder ein pharmazeutisch verträgliches Salz ist;
oder ein pharmazeutisch verträgliches Salz davon; 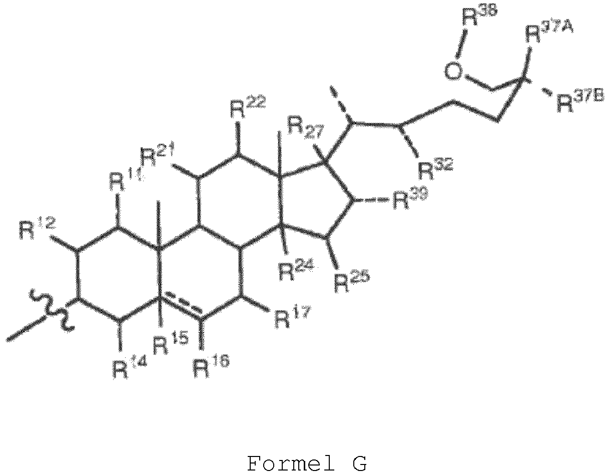 wobei
R¹¹, R¹², R¹⁴, R¹⁶, R¹⁷, R²¹, R²², R²⁴, R²⁵ und R²⁷ jeweils unabhängig H, OH, =O, pharmakologisch verträgliche Estergruppen oder pharmakologisch verträgliche Ethergruppen sind;
R¹⁵ H ist, wenn C-5, C-6 eine Einfachbindung ist, und nichts, wenn C-5, C-6 eine Doppelbindung ist;
R³² und R³⁹ jeweils unabhängig H, OH, =O, pharmakologisch verträgliche Estergruppen oder pharmakologisch verträgliche Ethergruppen sind;
R^{37A} H ist, wobei gleichzeitig R^{37B} CH₃ ist, oder R^{37A} CH₃ ist, wobei gleichzeitig R^{37B} H ist;
R³⁸ H oder ein Saccharid; oder ein pharmazeutisch verträgliches Salz davon ist;
oder ein pharmazeutisch verträgliches Salz davon;
R², R³ und R⁴ jeweils unabhängig eine Sauerstoffschutzgruppe sind;
R⁵ eine Acylgruppe ist, die aus der Gruppe ausgewählt ist, die aus Benzoyl, 2-Chlorbenzoyl, 4-Chlorbenzoyl, 4-Nitrobenzoyl und 4-Methoxybenzoyl besteht;
R⁶ und R⁷ H sind;
R⁹ aus der Gruppe ausgewählt ist, die aus H, CH₃ und einem durch eine Sauerstoffschutzgrupe geschützten Sauerstoff besteht;
wobei das Verfahren umfasst
(1) Reaktion einer Verbindung der Formel A mit einem Acylierungsmittel, das aus der Gruppe ausgewählt ist, die aus Benzoylchlorid, 2-Chlorbenzoylchlorid, 4-Chlorbenzoylchlorid, 4-Nitrobenzoylchlorid und 4-Methoxybenzoylchlorid besteht, in einer Acylierungsreaktion in Gegenwart einer Base; 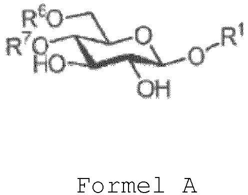 wobei
R¹ ein Sapogenin ist, wie vorstehend definiert;
R⁶ und R⁷ jeweils unabhängig eine Sauerstoffschutzgruppe sind;
oder zusammengenommen eine zyklische Disauerstoff-Schutzgruppe bilden;
um eine Verbindung der Formel B bereitzustellen 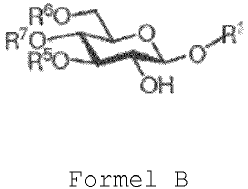
R¹ Sapogenin ist, wie vorstehend definiert;
R⁵ eine Acylgruppe ist, die aus der Gruppe ausgewählt ist, die aus Benzoyl, 2-Chlorbenzoyl, 4-Chlorbenzoyl, 4-Nitrobenzoyl und 4-Methoxybenzoyl besteht;
R⁶ und R⁷ jeweils unabhängig eine Sauerstoffschutzgruppe sind oder zusammengenommen eine zyklische Disauerstoff-Schutzgruppe bilden;
(2) Reaktion einer Verbindung der Formel B mit einer Verbindung der Formel C unter Kopplungsbedingungen 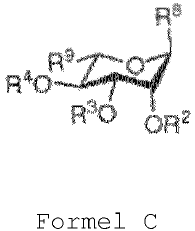 wobei
R², R³ und R⁴ jeweils unabhängig eine Sauerstoffschutzgruppe sind;
R⁸ eine Abgangsgruppe ist; und
R⁹ aus der Gruppe ausgewählt ist, die aus H, CH₃ und einem durch eine Sauerstoffschutzgruppe geschützten Sauerstoff besteht;
(3) selektives Entfernen der Sauerstoffschutzgruppen bei R⁶ und R⁷, um eine Verbindung der Formel X bereitzustellen.

2. Verfahren nach Anspruch 1, wobei die Base in Schritt (i) aus der Gruppe ausgewählt ist, die aus K₂CO₃, Triethylamin, Diisopropylethylamin, Pyridin, 4-Dimethylaminopyridin und 1,8-Diazabicycloundec-7-en besteht.

3. Verfahren nach einem der vorangehenden Ansprüche, wobei Schritt (i) in Gegenwart eines Lösungsmittels ausgeführt wird, das aus der Gruppe ausgewählt ist, die aus Dichlormethan, Tetrahydrofuran, 1,2-Dioxan, Dichlorethan, Chloroform, Kohlenstofftetrachlorid und Pyridin besteht.

4. Verfahren nach einem der vorangehenden Ansprüche, wobei Schritt (i) bei einer Temperatur im Bereich von -100 bis 80 °C durchgeführt wird, wobei die Temperatur von Schritt vorzugsweise (i) anfänglich im Bereich von -10 bis 20 °C liegt, und dann anschließend über den Verlauf der Reaktion auf eine Temperatur im Bereich von 10 bis 25 °C erhöht wird.

5. Verfahren nach einem der vorangehenden Ansprüche, wobei das Verhältnis von Acylierungsmittel zu einer Verbindung der Formel A 3:1 bis 1:1 ist.

6. Verfahren nach einem der vorangehenden Ansprüche, wobei
R⁶ und R⁷ von Formel A zusammengenommen eine zyklische Gruppe bilden, die aus der Gruppe ausgewählt ist, die aus 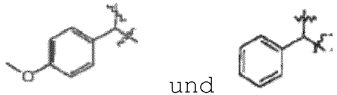 besteht;
R², R³, R⁴ jeweils unabhängig Acetyl oder Benzoyl sind; und
R⁸ aus der Gruppe ausgewählt ist, die aus -SEt, -Br, 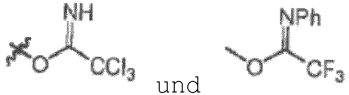 besteht.

7. Verfahren nach einem der vorangehenden Ansprüche, wobei R¹ aus der Gruppe ausgewählt ist, die aus Spirostanolaglyconen und Furostanolaglyconen besteht, die aus der Gruppe ausgewählt sind, die aus Diosgenin, Yamogenin (Neodiosgenin), Yuccagenin, Sarsasapogenin, Tigogemn, Smilagenin, Hecogenin, Gitogemn, Convallamarogenin, Neoruscogenin, Solagenin, Protodiosgenin, Pseudoprotodiosgenin, Methylprotodiosgenin, Protoyamogenin, Methylprotoyamogenin und pharmazeutisch verträglichen Salzen, Hydraten davon besteht.

8. Verfahren nach einem der Ansprüche 1 bis 7, das ferner den Schritt des (4) Umsetzens einer Verbindung der Formel X in eine Verbindung der Formel Y 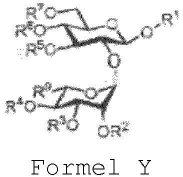 umfasst, wobei
R¹ ein Sapogenin ist, wie in Anspruch 1 definiert;
R², R³, R⁴, R⁵ und R⁷ jeweils H sind;
R⁶ H oder ein Saccharid ist;
R⁹ aus der Gruppe ausgewählt ist, die aus H, OH und CH₃ besteht;
pharmazeutisch verträgliche Salze, Hydrate oder Solvate davon.

9. Verfahren nach Anspruch 8, wobei Schritt (iv) das Entfernen von irgendwelchen Schutzgruppen umfasst, um eine Verbindung der Formel Y bereitzustellen.

10. Verfahren nach Anspruch 8, wobei Schritt (iv) einen Prozess umfasst, der aus der Gruppe ausgewählt ist, die besteht aus:
(1)
(a) selektivem Einführen einer Sauerstoffschutzgruppe bei R⁷;
(b) Koppeln eines geeigneten Saccharids unter Kopplungsbedingungen, so dass R⁶ ein Saccharid ist;
um eine Verbindung der Formel X' bereitzustellen 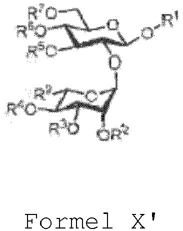 wobei
R¹ ein Sapogenin ist, wie in Anspruch 1 definiert;
R², R³, R⁴ und R⁷ jeweils unabhängig eine Sauerstoffschutzgruppe sind;
R⁵ eine Acylgruppe ist, die aus der Gruppe ausgewählt ist, die aus Benzoyl, 2-Chlorbenzoyl, 4-Chlorbenzoyl, 4-Nitrobenzoyl und 4-Methoxybenzoyl besteht;
R⁶ ein Saccharid ist;
R⁹ aus der Gruppe ausgewählt ist, die aus H, CH₃ und einem durch eine Sauerstoffschutzgruppe geschützten Sauerstoff besteht; und
(c) Umsetzen einer Verbindung der Formel X' in eine Verbindung der Formel Y, ein pharmazeutisch verträgliches Salz, Hydrat oder Solvat davon;
(2)
(a) selektives Einführen einer Sauerstoffschutzgruppe bei R⁷;
(b) Koppeln eines geeigneten Saccharids unter Kopplungsbedingungen, so dass R⁶ ein Saccharid ist, um eine Verbindung der Formel X' bereitzustellen, wie vorstehend definiert; und
(c) Entfernen von irgendwelchen Schutzgruppen, um eine Verbindung der Formel Y bereitzustellen;
(3)
(a) selektives Einführen einer Sauerstoffschutzgruppe bei R⁷;
(b) Koppeln eines geeigneten Saccharids unter Kopplungsbedingungen, so dass R⁶ ein Saccharid ist, um eine Verbindung der Formel X' bereitzustellen, wie vorstehend definiert;
(c) Umsetzen des Sapogenins bei R¹ in ein Sapogenin der Formel G, wie in Anspruch 1 definiert; und
(d) Entfernen von irgendwelchen Schutzgruppen, um eine Verbindung der Formel Y bereitzustellen; und
(4)
(a) selektives Einführen einer Sauerstoffschutzgruppe bei R⁷;
(b) Koppeln eines geeigneten Saccharids unter Kopplungsbedingungen, so dass R⁶ ein Saccharid ist, um eine Verbindung der Formel X' bereitzustellen, wie vorstehend definiert;
(c) Umsetzen des Sapogenins bei R¹ in ein Sapogenin der Formel F, wie in Anspruch 1 definiert; und
(d) Entfernen von irgendwelchen Schutzgruppen, um eine Verbindung der Formel Y bereitzustellen.

11. Verfahren nach einem der Ansprüche 8 bis 10, wobei die Verbindung der Formel Y aus der Gruppe ausgewählt ist, die besteht aus 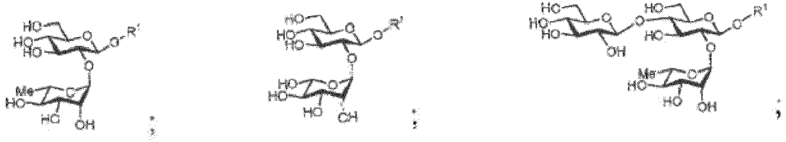 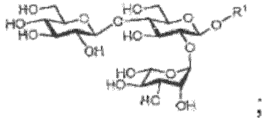 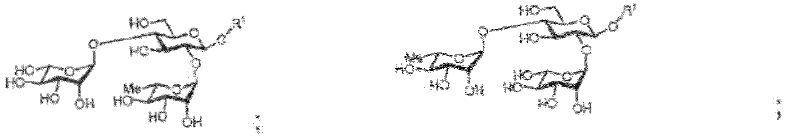 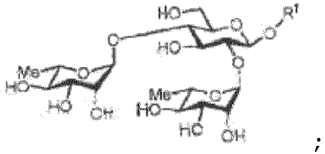 und 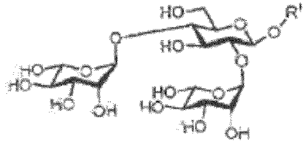 und/oder
wobei R¹ aus der Gruppe ausgewählt ist, die besteht aus: 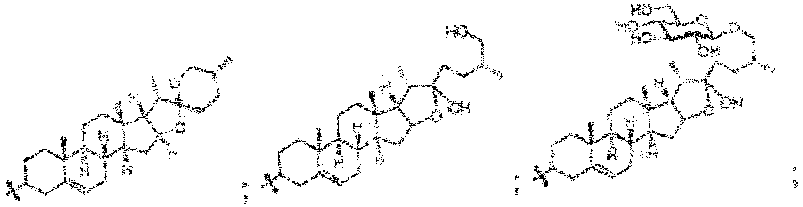 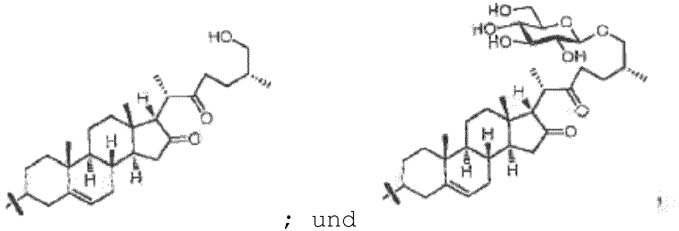 wobei vorzugsweise die Verbindung der Formel Y aus der Gruppe ausgewählt ist, die besteht aus: 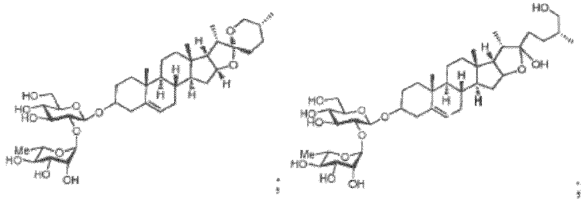 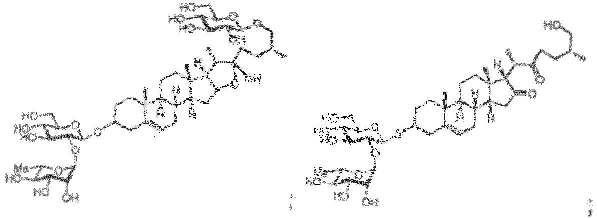 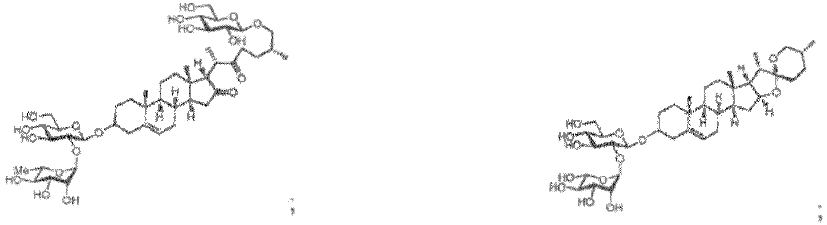 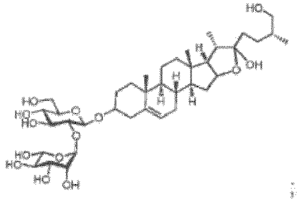 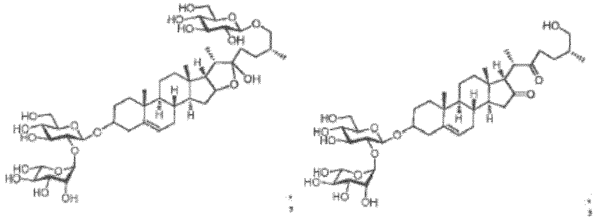 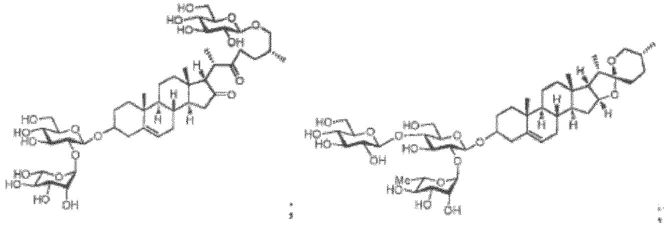 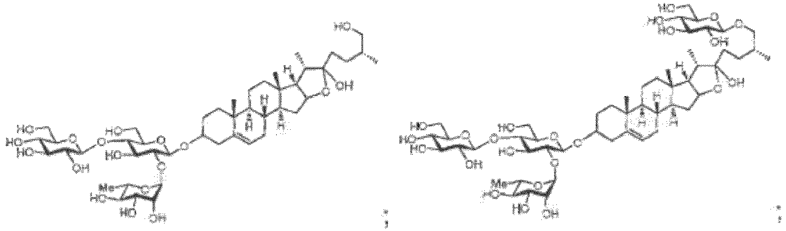 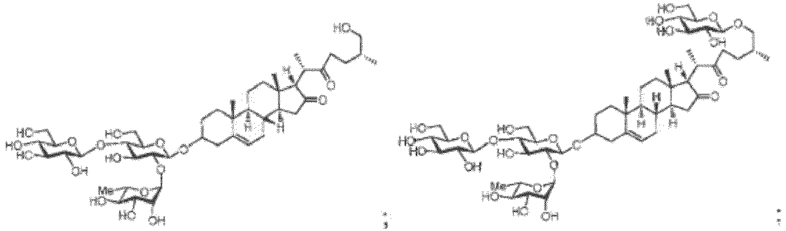 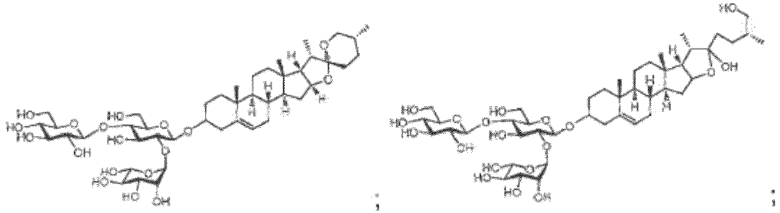 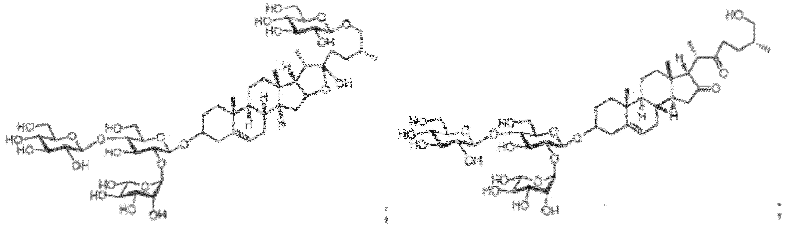 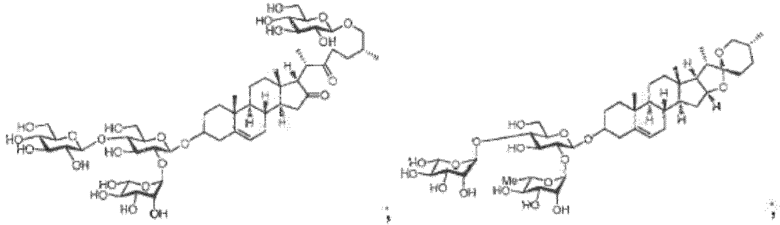 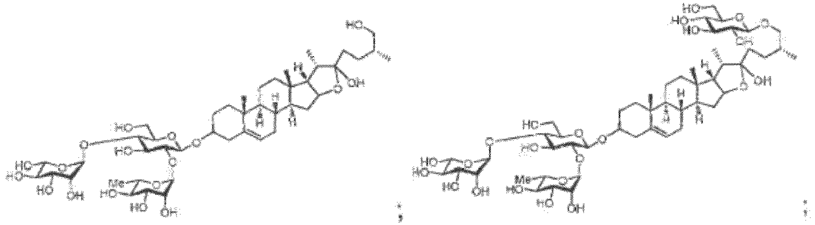 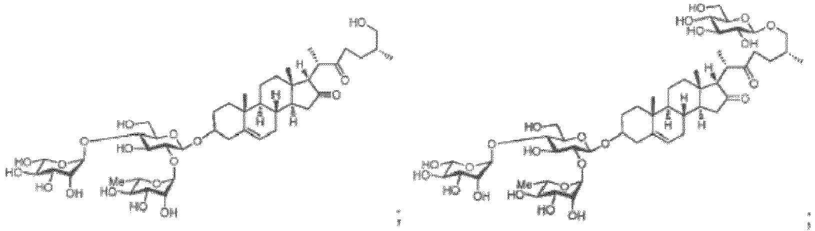 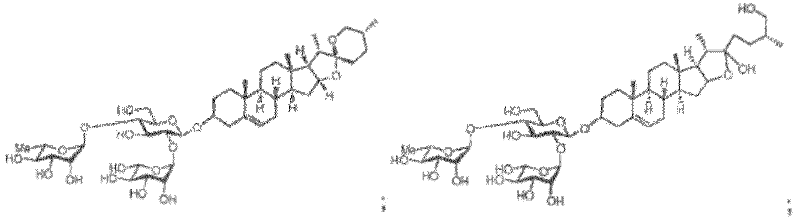 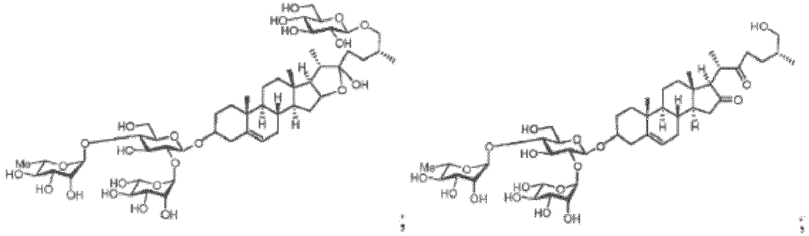 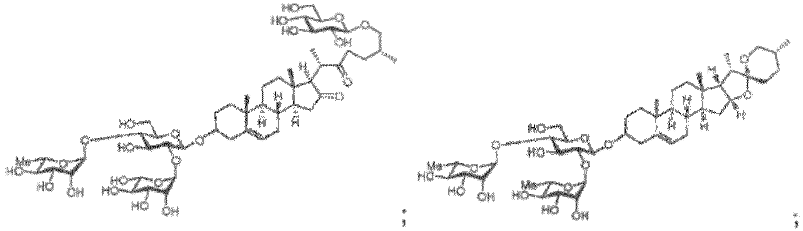 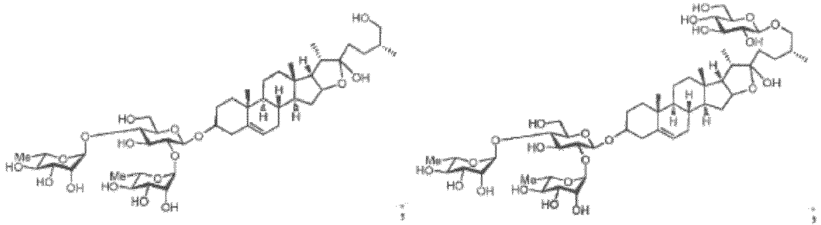 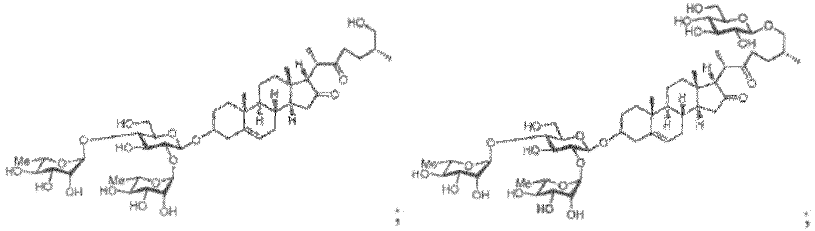 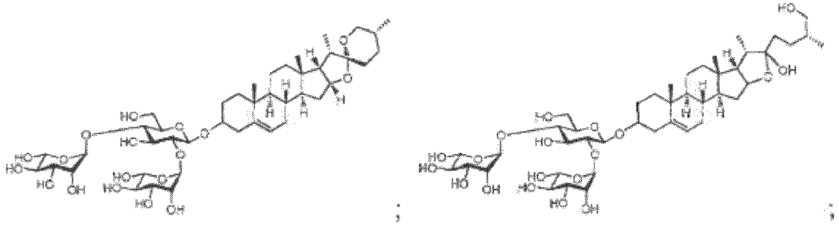 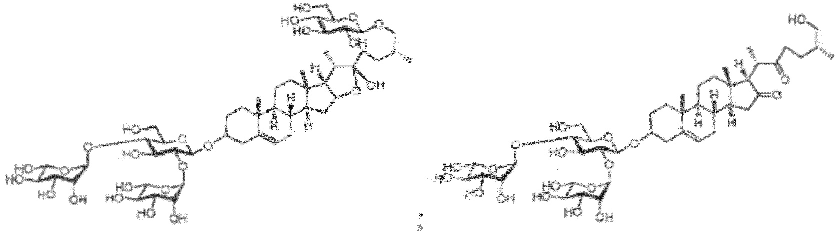 ; und 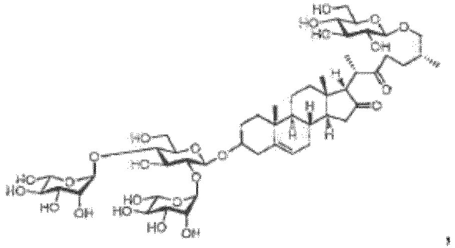 wobei bevorzugter die Verbindung der Formel Y aus der Gruppe ausgewählt ist, die besteht aus:

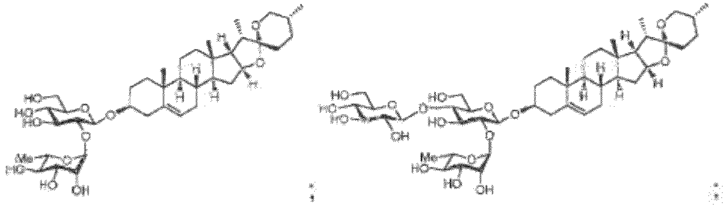

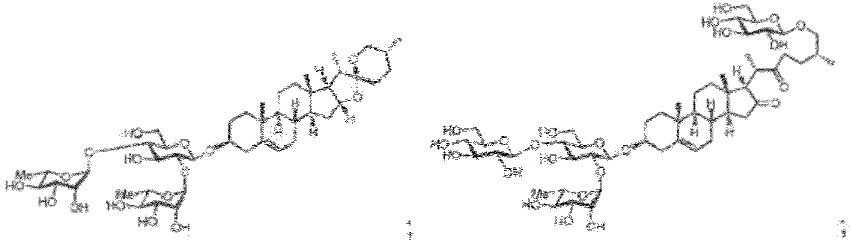

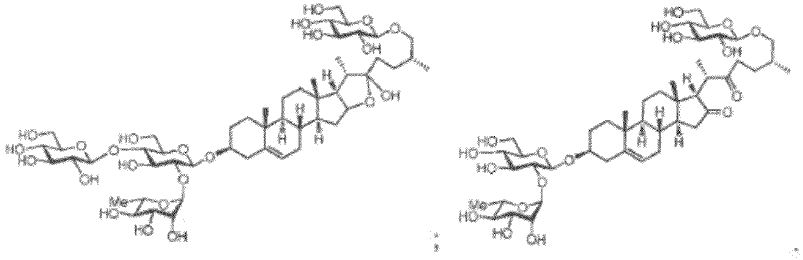

12. Verfahren nach einem der vorangehenden Ansprüche, wobei die Verbindung der Formel X 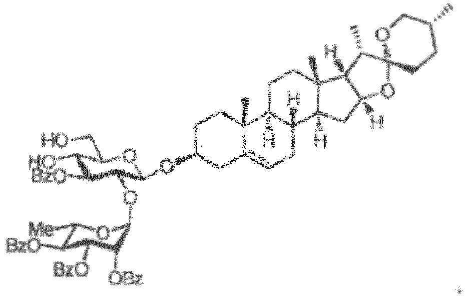 ist.

## Revendications

1. Procédé pour la préparation d'un composé de Formule X : 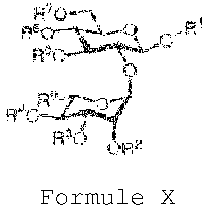 dans laquelle
R¹ représente une sapogénine de Formule E, F ou G : 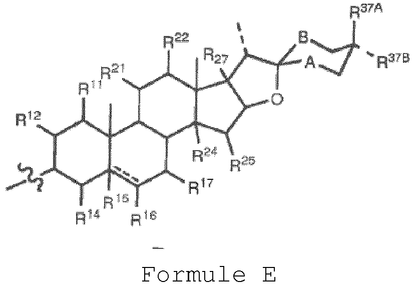 dans laquelle
R¹¹, R¹², R¹⁴, R¹⁶, R¹⁷, R²¹, R²², R²⁴, R²⁵ et R²⁷ représentent chacun, indépendamment les uns des autres, H, OH, =O, des groupes ester pharmacologiquement acceptables ou des groupes éther pharmacologiquement acceptables ;
R¹⁵ représente H lorsque C-5, C-6 est une liaison simple, et rien lorsque C-5, C-6 est une liaison double ;
A représente soit O avec B représentant simultanément CH₂, soit B représente O avec A représentant simultanément CH₂ ;
R^{37A} représente H avec R^{37B} représentant simultanément CH₃, ou R^{37A} représente CH₃ avec R^{37B} représentant simultanément H ;
ou un sel pharmaceutiquement acceptable de celui-ci ; 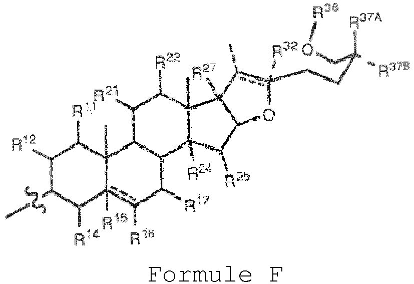 dans laquelle
R¹¹, R¹², R¹⁴, R¹⁶, R¹⁷, R²¹, R²², R²⁴, R²⁵ et R²⁷ représentent chacun, indépendamment les uns des autres, H, OH, =O, des groupes ester pharmacologiquement acceptables ou des groupes éther pharmacologiquement acceptables ;
R¹⁵ représente H lorsque C-5, C-6 est une liaison simple, et rien lorsque C-5, C-6 est une liaison double ;
R³² représente soit un groupe hydroxyle ou alcoxyle lorsque C-20, C-22 est une liaison simple, soit rien lorsque C-20, C-22 représente une liaison double ;
R^{37A} représente H avec R^{37B} représentant simultanément CH₃, ou R^{37A} représente CH₃ avec R^{37B} représentant simultanément H ;
R³⁸ représente H ou un saccharide ; ou un sel pharmaceutiquement acceptable ;
ou un sel pharmaceutiquement acceptable de celui-ci ; 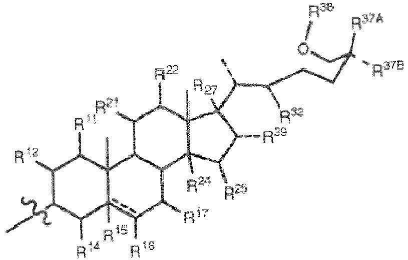 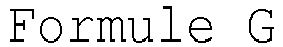 dans laquelle
R¹¹, R¹², R¹⁴, R¹⁶, R¹⁷, R²¹, R²², R²⁴, R²⁵ et R²⁷ représentent chacun, indépendamment les uns des autres, H, OH, =O, des groupes ester pharmacologiquement acceptables ou des groupes éther pharmacologiquement acceptables ;
R¹⁵ représente H lorsque C-5, C-6 est une liaison simple, et rien lorsque C-5, C-6 est une liaison double ;
R³² et R³⁹ représentent chacun, indépendamment les uns des autres, H, OH, =O, des groupes ester pharmacologiquement acceptables ou des groupes éther pharmacologiquement acceptables ;
R^{37A} représente H avec R^{37B} représentant simultanément CH₃, ou R^{37A} représente CH₃ avec R^{37B} représentant simultanément H ;
R³⁸ représente H ou un saccharide ; ou un sel pharmaceutiquement acceptable ;
ou un sel pharmaceutiquement acceptable de celui-ci ;
R², R³ et R⁴ représentent chacun, indépendamment les uns des autres, un groupe protecteur de l'oxygène ;
R⁵ représente un groupe acyle choisi parmi le groupe constitué de benzoyle, de 2-chlorobenzoyle, de 4-chlorobenzoyle, de 4-nitrobenzoyle et de 4-méthoxybenzoyle ;
R⁶ et R⁷ représentent H ;
R⁹ est choisi parmi le groupe constitué de H, CH₃ et d'un oxygène protégé par un groupe protecteur de l'oxygène ;
le procédé comportant les étapes consistant à :
(1) faire réagir un composé de Formule A avec un agent acylant choisi parmi le groupe constitué de chlorure de benzoyle, de chlorure de 2-chlorobenzoyle, de chlorure de 4-chlorobenzoyle, de chlorure de 4-nitrobennzoyle et de chlorure de 4-méthoxybenzoyle, dans une réaction d'acylation en présence d'une base ; 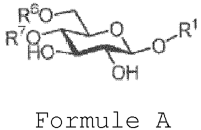 dans laquelle
R¹ représente une sapogénine telle que définie ci-dessus ; R⁶ et R⁷ représentent chacun, indépendamment l'un de l'autre, un groupe protecteur de l'oxygène ;
ou, lorsque pris ensemble, forment un groupe protecteur du dioxygène cyclique ;
pour fournir un composé de Formule B 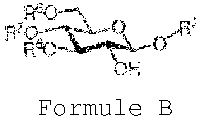 dans laquelle
R¹ représente une sapogénine telle que définie ci-dessus ; R⁵ représente un groupe acyle choisi parmi le groupe constitué de benzoyle, de 2-chlorobenzoyle, de 4-chlorobenzoyle, de 4-nitrobenzoyle et de 4-méthoxybenzoyle ;
R⁶ et R⁷ représentent chacun, indépendamment l'un de l'autre, un groupe protecteur de l'oxygène ou, lorsque pris ensemble, forment un groupe protecteur du di-oxygène cyclique ;
(2) faire réagir un composé de Formule B avec un composé de Formule C dans des conditions de couplage 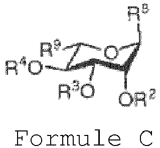 dans laquelle
R², R³ et R⁴ représentent chacun, indépendamment les uns des autres, un groupe protecteur de l'oxygène ;
R⁸ représente un groupe partant ; et
R⁹ est choisi parmi le groupe constitué de H, CH₃ et d'un oxygène protégé par un groupe protecteur de l'oxygène ;
(3) éliminer sélectivement les groupes protecteurs de l'oxygène en R⁶ et R⁷ pour fournir un composé de Formule X.

2. Procédé selon la revendication 1, dans lequel la base à l'étape (i) est choisie parmi le groupe constitué de K₂CO₃, triéthylamine, diisopropyléthylamine, pyridine, 4-diméthylaminopyridine et 1,8-diazabicycloundéc-7-ène.

3. Procédé selon l'une quelconque des revendications précédentes, dans lequel l'étape (i) est exécutée en présence d'un solvant choisi parmi le groupe constitué de dichlorométhane, tétrahydraofurane, 1,2-dioxane, dichloréthane, chloroforme, tétrachlorure de carbone et pyridine.

4. Procédé selon l'une quelconque des revendications précédentes, dans lequel l'étape (i) est réalisée à une température dans la plage de -100 à 80 °C, de préférence dans lequel la température de l'étape (i) est initialement dans la plage de -10 à 20 °C, et ensuite ultérieurement augmentée au cours de la réaction jusqu'à une température dans la plage de 10 à 25 °C.

5. Procédé selon l'une quelconque des revendications précédentes, dans lequel le rapport entre l'agent acylant et un composé de Formule A est compris entre 3:1 et 1:1.

6. Procédé selon l'une quelconque des revendications précédentes, dans lequel R⁶ et R⁷ de la Formule A sont pris ensemble pour former un groupe cyclique choisi parmi le groupe constitué de 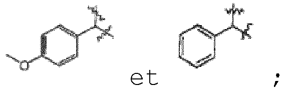
R², R³, R⁴ représentent chacun, indépendamment les uns des autres, un acétyle ou un benzoyle ; et
R⁸ est choisi parmi le groupe constitué de -SEt, -Br,

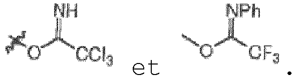

7. Procédé selon l'une quelconque des revendications précédentes, dans lequel R¹ est choisi parmi le groupe constitué de spirostanol aglycones et de furostanol aglycones choisis parmi le groupe constitué de diosgénine, yamogénine (néodiosgénine), yuccagénine, sarsasapogénine, tigogemn, smilagénine, hécogénine, gitogemn, convallamarogénine, néoruscogénine, solagénine, protodiosgénine, pseudoprotodiosgénine, méthyl protodiosgénine, protoyamogénine, méthyl protoyamogénine et de sels pharmaceutiquement acceptables, d'hydrates de ceux-ci.

8. Procédé selon l'une quelconque des revendications 1 à 7, comportant en outre l'étape consistant à (4) convertir un composé de Formule X en un composé de Formule Y : 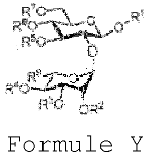 dans laquelle
R¹ représente une sapogénine telle que définie dans la revendication 1 ;
R², R³, R⁴, R⁵ et R⁷ représentent chacun H ;
R⁶ représente H ou un saccharide ;
R⁹ est choisi parmi le groupe constitué de H, OH et CH₃ ; des sels pharmaceutiquement acceptables, des hydrates ou des solvates de ceux-ci.

9. Procédé selon la revendication 8, dans lequel l'étape (iv) comporte l'élimination de tout groupe protecteur pour fournir un composé de Formule Y.

10. Procédé selon la revendication 8, dans lequel l'étape (iv) comporte un processus choisi parmi le groupe constitué de :
(1)
(a) introduire sélectivement un groupe protecteur de l'oxygène en R⁷ ;
(b) coupler un saccharide adapté dans des conditions de couplage telles que R⁶ est un saccharide ;
pour fournir un composé de Formule X' 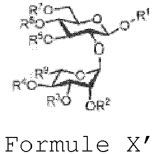 dans laquelle
R¹ représente une sapogénine telle que définie dans la revendication 1 ;
R², R³, R⁴ et R⁷ représentent chacun, indépendamment les uns des autres, un groupe protecteur de l'oxygène ;
R⁵ représente un groupe acyle choisi parmi le groupe constitué de benzoyle, de 2-chlorobenzoyle, de 4-chlorobenzoyle, de 4-nitrobenzoyle et de 4-méthoxybenzoyle ;
R⁶ représente un saccharide ;
R⁹ est choisi parmi le groupe constitué de H, CH₃ et d'un oxygène protégé par un groupe protecteur de l'oxygène ; et
(c) convertir un composé de Formule X' en un composé de Formule Y, un sel pharmaceutiquement acceptable, un hydrate ou un solvate de celui-ci ;
(2)
(a) introduire sélectivement un groupe protecteur de l'oxygène en R⁷ ;
(b) coupler un saccharide adapté dans des conditions de couplage telles que R⁶ est un saccharide pour fournir un composé de Formule X' telle que définie ci-dessus ; et
(c) éliminer tout groupe protecteur pour fournir un composé de Formule Y ;
(3)
(a) introduire sélectivement un groupe protecteur de l'oxygène en R⁷ ;
(b) coupler un saccharide adapté dans des conditions de couplage telles que R⁶ est un saccharide pour fournir un composé de Formule X' telle que définie ci-dessus ;
(c) convertir la sapogénine en R¹ en une sapogénine de Formule G telle que définie dans la revendication 1 ; et
(d) éliminer tout groupe protecteur pour fournir un composé de Formule Y ; et
(4)
(a) introduire sélectivement un groupe protecteur de l'oxygène en R⁷ ;
(b) coupler un saccharide adapté dans des conditions de couplage telles que R⁶ est un saccharide pour fournir un composé de Formule X' telle que définie ci-dessus ;
(c) convertir la sapogénine en R¹ en une sapogénine de Formule F telle que définie dans la revendication 1 ; et
(d) éliminer tout groupe protecteur pour fournir un composé de Formule Y.

11. Procédé selon l'une quelconque des revendications 8 à 10, dans lequel le composé de Formule Y est choisi parmi le groupe constitué de : 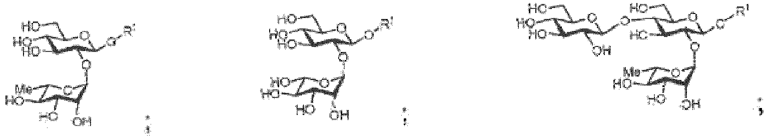 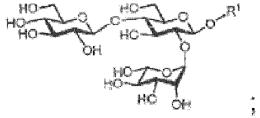 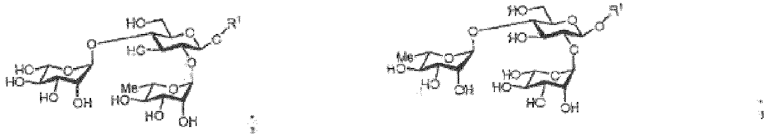 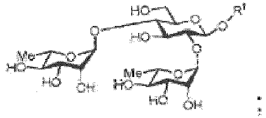 et 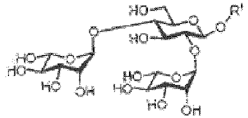 et/ou
dans lequel R¹ est choisi parmi le groupe constitué de : 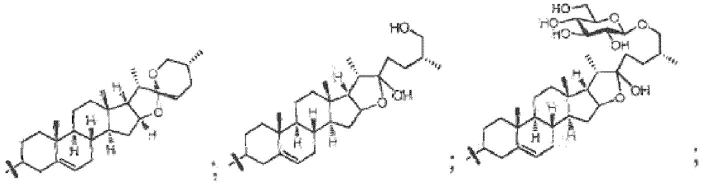 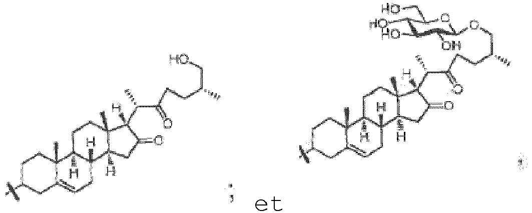 de préférence dans lequel le composé de Formule Y est choisi parmi le groupe constitué de : 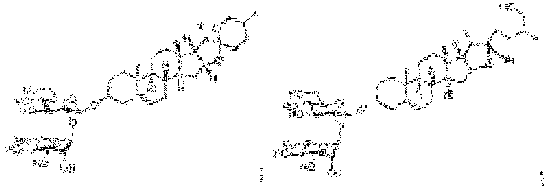 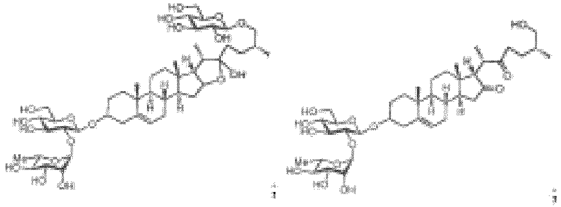 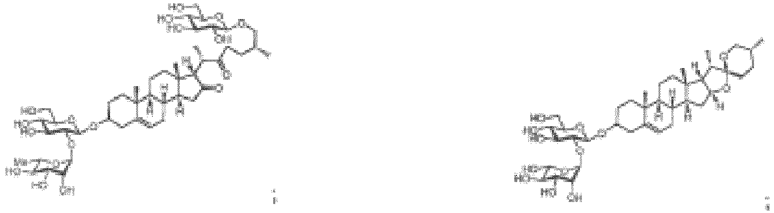 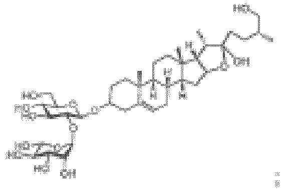 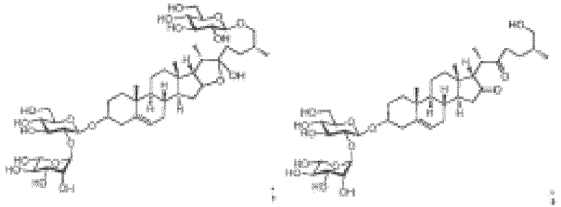 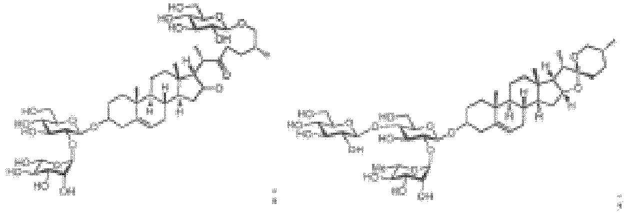 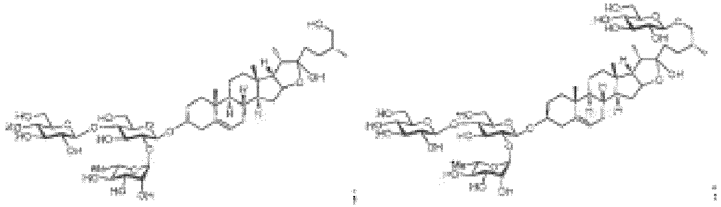 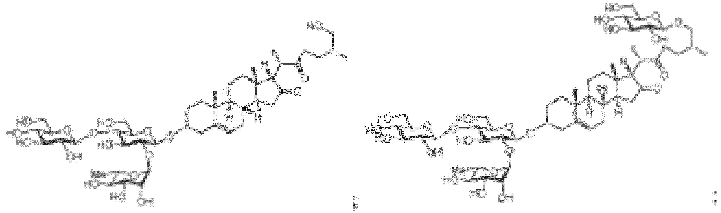 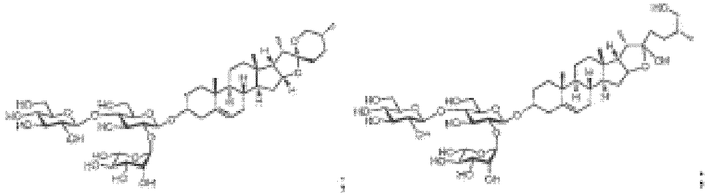 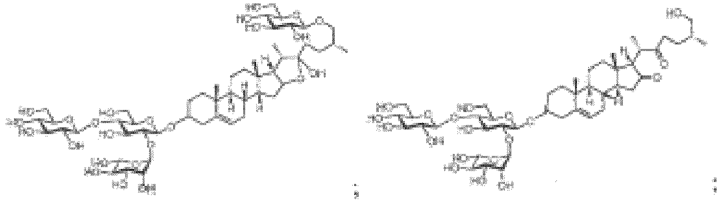 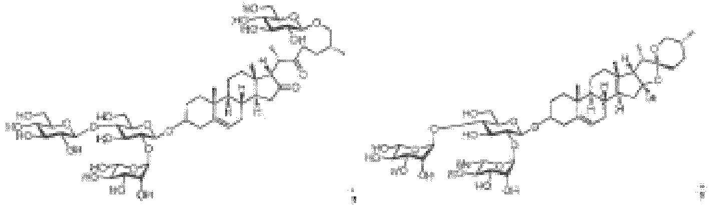 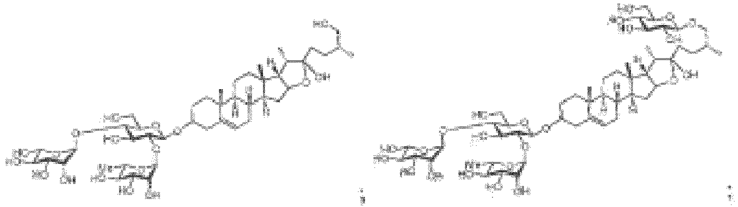 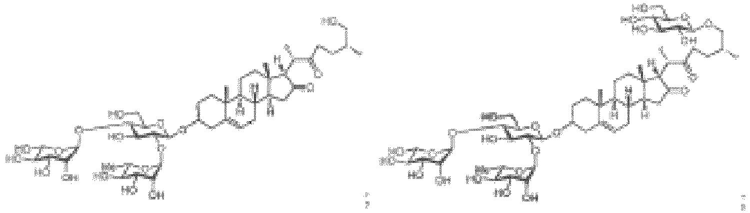 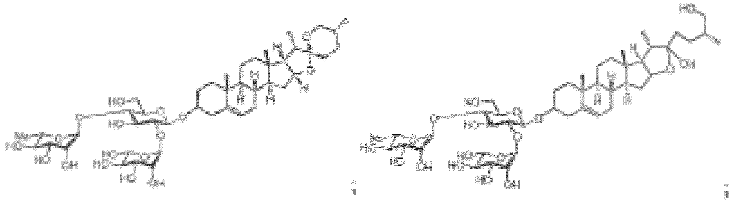 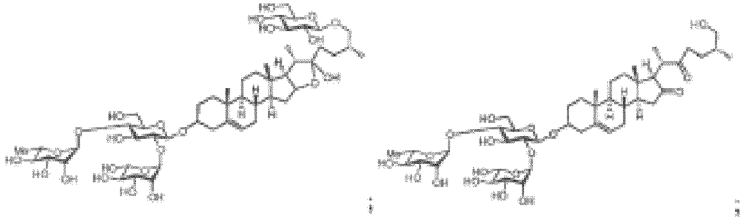 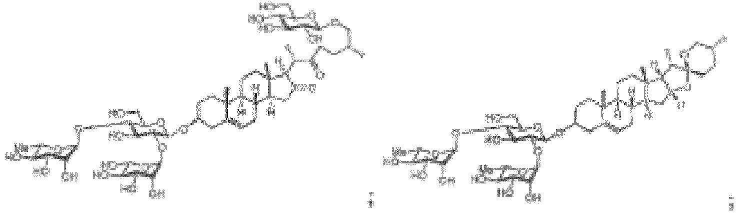 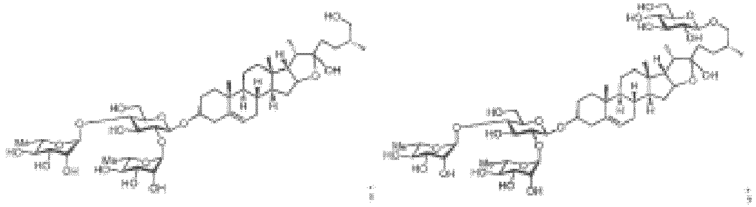 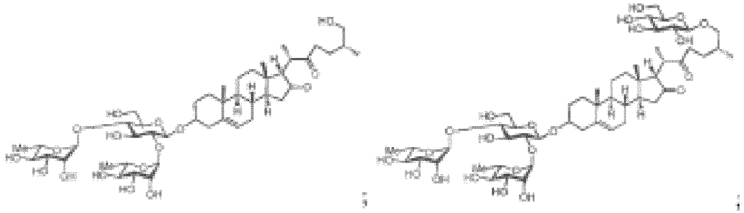 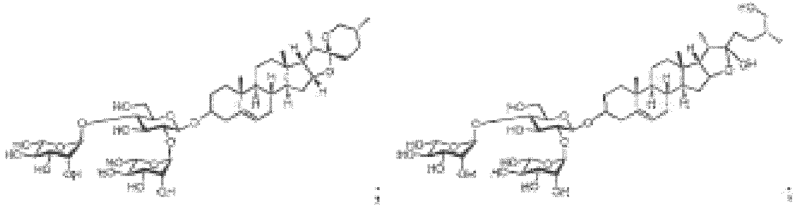 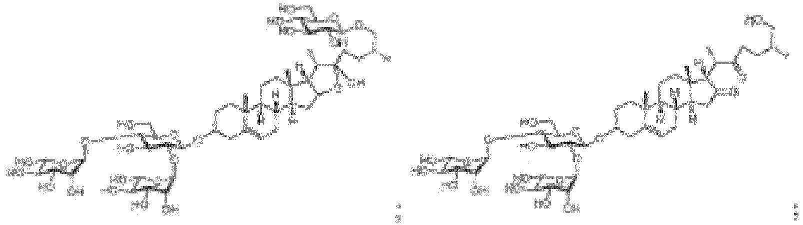 et 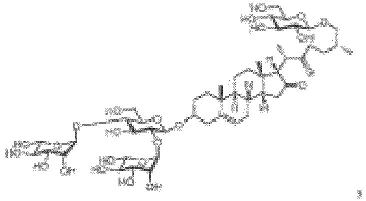 de manière plus préférée, dans lequel le composé de Formule Y est choisi parmi le groupe constitué de :

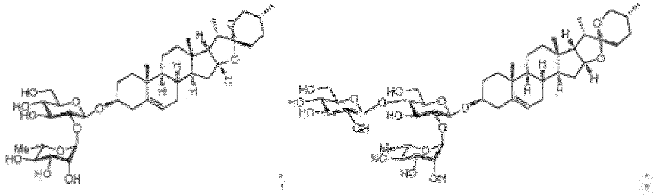

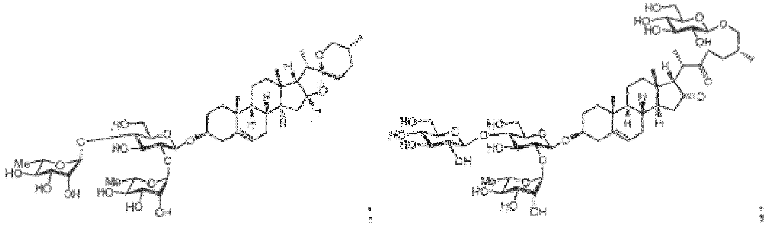

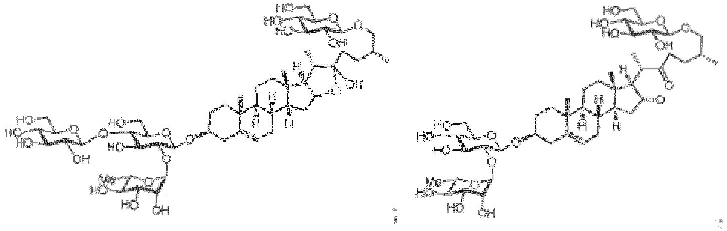

12. Procédé selon l'une quelconque des revendications précédentes, dans lequel le composé de Formule X est
